(19) Europäisches Patentamt
European Patent Office
Office européen des brevets

(11) EP 1 036 175 B1

(12) **EUROPEAN PATENT SPECIFICATION**

(45) Date of publication and mention of the grant of the patent:
**09.04.2008 Bulletin 2008/15**

(21) Application number: **98960004.4**

(22) Date of filing: **11.12.1998**

(51) Int Cl.:
***C12N 15/12*** (2006.01) ***C07K 14/705*** (2006.01)
***C07K 16/28*** (2006.01) ***C12N 5/10*** (2006.01)
***A61K 38/17*** (2006.01) ***G01N 33/566*** (2006.01)

(86) International application number:
**PCT/GB1998/003720**

(87) International publication number:
**WO 1999/031232 (24.06.1999 Gazette 1999/25)**

(54) **HUMAN BRAIN DERIVED KCNQ2 POTASSIUM CHANNEL**

AUS DEM MENSCHLICHEN GEHIRN STAMMENDER KCNQ2 KALIUMKANAL

CANAL POTASSIQUE KCNQ2 EXTRAIT DU CERVEAU HUMAIN

(84) Designated Contracting States:
**AT BE CH CY DE DK ES FI FR GB GR IE IT LI LU MC NL PT SE**

(30) Priority: **13.12.1997 GB 9726339**

(43) Date of publication of application:
**20.09.2000 Bulletin 2000/38**

(73) Proprietor: **AstraZeneca AB**
**151 85 Södertälje (SE)**

(72) Inventors:
- **Aiyar, Jayashree**
  **Wilmington, DE 19850-5437 (US)**
- **Iannotti, Claudia Ann**
  **Wilmington, DE 19850-5437 (US)**
- **Christian, Edward Philip**
  **Wilmington, DE 19850-5437 (US)**
- **Logsdon, Naomi Jean**
  **35222 Alabama (US)**

(74) Representative: **Phillips, Neil Godfrey Alasdair et al**
**Astra Zeneca PLC**
**Global Intellectual Property**
**Mereside**
**Alderley Park**
**Macclesfield Cheshire SK10 4TG (GB)**

(56) References cited:
**WO-A-97/23598**

- **PATENT ABSTRACTS OF JAPAN vol. 097, no. 011, 28 November 1997 & JP 09 191882 A (JAPAN TOBACCO INC), 29 July 1997 -& EMPATENT DATABASE Accession number E13516 Yokoyama M. ET AL. 23-JUN-1998 (Rel. 56, Created) Human mRNA for a K+ channel protein XP002095811**
- **YOKOYAMA M ET AL: "Identification and cloning of neuroblastoma-specific and nerve tissue-specific genes through compiled expression profiles." DNA RES, OCT 31 1996, 3 (5) P311-20, XP002095807 JAPAN cited in the application -& EMHUM1 DATABASE Accession number D82346 Yokoyama M. ET AL. 15-FEB-1997 (Rel. 50, Created) Homo sapiens mRNA for HNSPC XP002095812**
- **WEI A ET AL: "Eight potassium channel families revealed by the C. elegans genome project." NEUROPHARMACOLOGY, 1996, 35 (7) P805-29, XP002095808 ENGLAND**
- **BIERVERT C ET AL: "A potassium channel mutation in neonatal human epilepsy." SCIENCE, JAN 16 1998, 279 (5349) P403-6, XP002095809 UNITED STATES**
- **SINGH NA ET AL: "A novel potassium channel gene, KCNQ2, is mutated in an inherited epilepsy of newborns [see comments]" NAT GENET, JAN 1998, 18 (1) P25-9, XP002095810 UNITED STATES cited in the application**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

SEQUENCE LISTING

(1) GENERAL INFORMATION:

(i) APPLICANT:

(A) NAME: Zeneca Ltd
(B) STREET: 15 Stanhope Gate
(C) CITY: London
(D) STATE: Greater London
(E) COUNTRY: England
(F) POSTAL CODE (ZIP): W1Y 6LN
(G) TELEPHONE: 0171 304 5000
(H) TELEFAX: 0171 304 5151
(I) TELEX: 0171 834 2042

(ii) TITLE OF INVENTION: POLYNUCLEOTIDES
(iii) NUMBER OF SEQUENCES: 8
(iv) COMPUTER READABLE FORM:

(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(vi) PRIOR APPLICATION DATA:

(A) APPLICATION NUMBER: GB 9726339.6
(B) FILING DATE: 13-DEC-1997

(2) INFORMATION FOR SEQ ID NO: 1:

(i) SEQUENCE CHARACTERISTICS:

(A) LENGTH: 3029 base pairs

(B) TYPE: nucleic acid
(C) STRANDEDNESS: single
(D) TOPOLOGY: linear

(ii) MOLECULE TYPE: other nucleic acid
(xi) SEQUENCE DESCRIPTION: SEQ ID NO: 1:

GCCAGGCACC ATGGTGCAGA AGTCGCGCAA CGGCGGCGTA TACCCCGGCC CGAGCGGGGA 60

GAAGAAGCTG AAGGTGGGCT TCGTGGGGCT GGACCCCGGC GCGCCCGACT CCACCCGGGA 120

CGGGGCGCTG CTGATCGCCG GCTCCGAGGC CCCCAAGCGC GGCAGCATCC TCAGCAAACC 180

TCGCGCGGGC GGCGCGGGCG CCGGGAAGCC CCCCAAGCGC AACGCCTTCT ACCGCAAGCT 240

GCAGAATTTC CTCTACAACG TGCTGGAGCG GCCGCGCGGC TGGGCGTTCA TCTACCACGC 300

CTACGTGTTC CTCCTGGTTT TCTCCTGCCT CGTGCTGTCT GTGTTTTCCA CCATCAAGGA 360

GTATGAGAAG AGCTCGGAGG GGGCCCTCTA CATCCTGGAA ATCGTGACTA TCGTGGTGTT 420

TGGCGTGGAG TACTTCGTGC GGATCTGGGC CGCAGGCTGC TGCTGCCGGT ACCGTGGCTG 480

GAGGGGGCGG CTCAAGTTTG CCCGGAAACC GTTCTGTGTG ATTGACATCA TGGTGCTCAT 540

CGCCTCCATT GCGGTGCTGG CCGCCGGCTC CCAGGGCAAC GTCTTTGCCA CATCTGCGCT 600

CCGGAGCCTG CGCTTCCTGC AGATTCTGCG GATGATCCGC ATGGACCGGC GGGGAGGCAC 660

CTGGAAGCTG CTGGGCTCTG TGGTCTATGC CCACAGCAAG GAGCTGGTCA CTGCCTGGTA 720

CATCGGCTTC CTTTGTCTCA TCCTGGCCTC GTTCCTGGTG TACTTGGCAG AGAAGGGGGA 780

GAACGACCAC TTTGACACCT ACGCGGATGC ACTCTGGTGG GGCCTGATCA CGCTGACACC 840

ATTGGCTACG GGGACAAGTA CCCCCAGACC TGGAACGGCA GGCTCCTTGC GGCAACCTTC 900

ACCCTCATCG GTGTCTCCTT CTTCGCGCTG CCTGCAGGCA TCTTGGGGTC TGGGTTTGCC 960

CTGAAGGTTC AGGAGCAGCA CAGGCAGAAG CACTTTGAGA AGAGGCGGAA CCCGGCAGCA 1020

GGCCTGATCC AGTCGGCCTG GAGATTCTAC GCCACCAACC TCTCGCGCAC AGACCTGCAC 1080

TCCACGTGGC AGTACTACGA GCGAACGGTC ACCGTGCCCA TGTACAGTTC GCAAACTCAA 1140

ACCTACGGGG CCTCCAGACT TATCCCCCCG CTGAACCAGC TGGAGCTGCT GAGGAACCTC 1200

AAGAGTAAAT CTGGACTCGC TTTCAGGAAG GACCCCCCGC CGGAGCCGTC TCCAAGCCAG 1260

AAGGTCAGTT TGAAAGATCG TGTCTTCTCC AGCCCCCGAG GCGTGGCTGC CAAGGGGAAG 1320

GGGTCCCCGC AGGCCCAGAC TGTGAGGCGG TCACCCAGCG CCGACCAGAG CCTCGAGGAC 1380

AGCCCCAGCA AGGTGCCCAA GAGCTGGAGC TTCGGGGACC GCAGCCGGGC ACGCCAGGCT 1440

TTCCGCATCA AGGGTGCCGC GTCACGGCAG AACTCAGAAG AAGCAAGCCT CCCCGGAGAG 1500

GACATTGTGG ATGACAAGAG CTGCCCCTGC GAGTTTGTGA CCGAGGACCT GACCCCGGGC 1560

CTCAAAGTCA GCATCAGAGC CGTGTGTGTC ATGCGGTTCC TGGTGTCCAA GCGGAAGTTC 1620

AAGGAGAGCC TGCGGCCCTA CGACGTGATG GACGTCATCG AGCAGTACTC AGCCGGCCAC 1680

CTGGACATGC TGTCCCGAAT TAAGAGCCTG CAGTCCAGAG TGGACCAGAT CGTGGGGCGG 1740

GGCCCAGCGA TCACGGACAA GGACCGCACC AAGGGCCCGG CCGAGGCGGA GCTGCCCGAG 1800

GACCCCAGCA TGATGGGACG GCTCGGGAAG GTGGAGAAGC AGGTCTTGTC CATGGAGAAG 1860

AAGCTGGACT TCCTGGTGAA TATCTACATG CAGCGGATGG GCATCCCCCC GACAGAGACC 1920

GAGGCCTACT TTGGGGCCAA AGAGCCGGAG CCGGCGCCGC CGTACCACAG CCCGGAAGAC 1980

AGCCGGGAGC ATGTCGACAG GCACGGCTGC ATTGTCAAGA TCGTGCGCTC CAGCAGCTCC 2040

ACGGGCCAGA AGAACTTCTC GGCGCCCCCG GCCGCGCCCC CTGTCCAGTG TCCGCCCTCC 2100

ACCTCCTGGC AGCCACAGAG CCACCCGCGC CAGGGCCACG GCACCTCCCC CGTGGGGGAC 2160

CACGGCTCCC TGGTGCGCAT CCCGCCGCCG CCTGCCCACG AGCGGTCGCT GTCCGCCTAC 2220

GGCGGGGGCA ACCGCGCCAG CATGGAGTTC CTGCGGCAGG AGGACACCCC GGGCTGCAGG 2280

CCCCCCGAGG GGACCCTGCG GGACAGCGAC ACGTCCATCT CCATCCCGTC CGTGGACCAC 2340

GAGGAGCTGG AGCGTTCCTT CAGCGGCTTC AGCATCTCCC AGTCCAAGGA GAACCTGGAT 2400

GCTCTCAACA GCTGCTACGC GGCCGTGGCG CCTTGTGCCA AAGTCAGGCC CTACATTGCG 2460

GAGGGAGAGT CAGACACCGA CTCCGACCTC TGTACCCCGT GCGGGCCCCC GCCACGCTCG 2520

GCCACCGGCG AGGGTCCCTT TGGTGACGTG GGCTGGGCCG GGCCCAGGAA GTGAGGCGGC 2580

GCTGGGCCAG TGGACCCGCC CGCGGCCCTC CTCAGCACGG TGCCTCCGAG GTTTTGAGGC 2640

GGGAACCCTC TGGGGCCCTT TTCTTACAGT AACTGAGTGT GGCGGGAAGG GTGGGCCCTG 2700

GAGGGGCCCA TGTGGGCTGA AGGATGGGGG CTCCTGGCAG TGACCTTTTA CAAAAGTTAT 2760

TTTCCAACAG GGGCTGGAGG GCTGGGCAGG GCCCTGTGGC TCCAGGAGCA GCGTGCAGGA 2820

GCAAGGCTGC CCTGTCCACT CTGCTCAGGG CCGCGGCCGA CATCAGCCCG GTGTGAGGAG 2880

GGGCGGGAGT GATGACGGGG TGTTGCCAGC GTGGCAACAG GCGGGGGGTT GTCTCAGCCG 2940

AGCCCAGGGG AGGCACAAAG GGCAGGCCTG TTCCCTGAGG ACCTGCGCAA AGGGCGGGCC 3000

TGTTTGGTGA GGACCTGCGG CCTTGGGTC 3029

(2) INFORMATION FOR SEQ ID NO: 2:

(i) SEQUENCE CHARACTERISTICS:

(A) LENGTH: 2565 base pairs
(B) TYPE: nucleic acid
(C) STRANDEDNESS: single
(D) TOPOLOGY: linear

(ii) MOLECULE TYPE: other nucleic acid
(xi) SEQUENCE DESCRIPTION: SEQ ID NO: 2:

ATGGTGCAGA AGTCGCGCAA CGGCGGCGTA TACCCCGGCC CGAGCGGGGA GAAGAAGCTG 60

AAGGTGGGCT TCGTGGGGCT GGACCCCGGC GCGCCCGACT CCACCCGGGA CGGGGCGCTG 120

CTGATCGCCG GCTCCGAGGC CCCCAAGCGC GGCAGCATCC TCAGCAAACC TCGCGCGGGC 180

GGCGCGGGCG CCGGGAAGCC CCCCAAGCGC AACGCCTTCT ACCGCAAGCT GCAGAATTTC 240

CTCTACAACG TGCTGGAGCG GCCGCGCGGC TGGGCGTTCA TCTACCACGC CTACGTGTTC 300

CTCCTGGTTT TCTCCTGCCT CGTGCTGTCT GTGTTTTCCA CCATCAAGGA GTATGAGAAG 360

AGCTCGGAGG GGGCCCTCTA CATCCTGGAA ATCGTGACTA TCGTGGTGTT TGGCGTGGAG 420

TACTTCGTGC GGATCTGGGC CGCAGGCTGC TGCTGCCGGT ACCGTGGCTG GAGGGGGCGG 480

CTCAAGTTTG CCCGGAAACC GTTCTGTGTG ATTGACATCA TGGTGCTCAT CGCCTCCATT 540

GCGGTGCTGG CCGCCGGCTC CCAGGGCAAC GTCTTTGCCA CATCTGCGCT CCGGAGCCTG 600

CGCTTCCTGC AGATTCTGCG GATGATCCGC ATGGACCGGC GGGGAGGCAC CTGGAAGCTG 660

CTGGGCTCTG TGGTCTATGC CCACAGCAAG GAGCTGGTCA CTGCCTGGTA CATCGGCTTC 720

CTTTGTCTCA TCCTGGCCTC GTTCCTGGTG TACTTGGCAG AGAAGGGGGA GAACGACCAC 780

TTTGACACCT ACGCGGATGC ACTCTGGTGG GGCCTGATCA CGCTGACCAC CATTGGCTAC 840

GGGGACAAGT ACCCCCAGAC CTGGAACGGC AGGCTCCTTG CGGCAACCTT CACCCTCATC 900

GGTGTCTCCT TCTTCGCGCT GCCTGCAGGC ATCTTGGGGT CTGGGTTTGC CCTGAAGGTT 960

CAGGAGCAGC ACAGGCAGAA GCACTTTGAG AAGAGGCGGA ACCCGGCAGC AGGCCTGATC 1020

CAGTCGGCCT GGAGATTCTA CGCCACCAAC CTCTCGCGCA CAGACCTGCA CTCCACGTGG 1080

CAGTACTACG AGCGAACGGT CACCGTGCCC ATGTACAGTT CGCAAACTCA AACCTACGGG 1140

GCCTCCAGAC TTATCCCCCC GCTGAACCAG CTGGAGCTGC TGAGGAACCT CAAGAGTAAA 1200

TCTGGACTCG CTTTCAGGAA GGACCCCCCG CCGGAGCCGT CTCCAAGCCA GAAGGTCAGT 1260

TTGAAAGATC GTGTCTTCTC CAGCCCCCGA GGCGTGGCTG CCAAGGGGAA GGGGTCCCCG 1320

CAGGCCCAGA CTGTGAGGCG GTCACCCAGC GCCGACCAGA GCCTCGAGGA CAGCCCCAGC 1380

AAGGTGCCCA AGAGCTGGAG CTTCGGGGAC CGCAGCCGGG CACGCCAGGC TTTCCGCATC 1440

AAGGGTGCCG CGTCACGGCA GAACTCAGAA GAAGCAAGCC TCCCCGGAGA GGACATTGTG 1500

GATGACAAGA GCTGCCCCTG CGAGTTTGTG ACCGAGGACC TGACCCCGGG CCTCAAAGTC 1560

AGCATCAGAG CCGTGTGTGT CATGCGGTTC CTGGTGTCCA AGCGGAAGTT CAAGGAGAGC 1620

CTGCGGCCCT ACGACGTGAT GGACGTCATC GAGCAGTACT CAGCCGGCCA CCTGGACATG 1680

CTGTCCCGAA TTAAGAGCCT GCAGTCCAGA GTGGACCAGA TCGTGGGGCG GGGCCCAGCG 1740

ATCACGGACA AGGACCGCAC CAAGGGCCCG GCCGAGGCGG AGCTGCCCGA GGACCCCAGC 1800

ATGATGGGAC GGCTCGGGAA GGTGGAGAAG CAGGTCTTGT CCATGGAGAA GAAGCTGGAC 1860

TTCCTGGTGA ATATCTACAT GCAGCGGATG GGCATCCCCC CGACAGAGAC CGAGGCCTAC 1920

TTTGGGGCCA AAGAGCCGGA GCCGGCGCCG CCGTACCACA GCCCGGAAGA CAGCCGGGAG 1980

CATGTCGACA GGCACGGCTG CATTGTCAAG ATCGTGCGCT CCAGCAGCTC CACGGGCCAG 2040

AAGAACTTCT CGGCGCCCCC GGCCGCGCCC CCTGTCCAGT GTCCGCCCTC CACCTCCTGG 2100

CAGCCACAGA GCCACCCGCG CCAGGGCCAC GGCACCTCCC CCGTGGGGGA CCACGGCTCC 2160

```
CTGGTGCGCA TCCCGCCGCC GCCTGCCCAC GAGCGGTCGC TGTCCGCCTA CGGCGGGGGC    2220

AACCGCGCCA GCATGGAGTT CCTGCGGCAG GAGGACACCC CGGGCTGCAG GCCCCCCGAG    2280

GGGACCCTGC GGGACAGCGA CACGTCCATC TCCATCCCGT CCGTGGACCA CGAGGAGCTG    2340

GAGCGTTCCT TCAGCGGCTT CAGCATCTCC CAGTCCAAGG AGAACCTGGA TGCTCTCAAC    2400

AGCTGCTACG CGGCCGTGGC GCCTTGTGCC AAAGTCAGGC CCTACATTGC GGAGGGAGAG    2460

TCAGACACCG ACTCCGACCT CTGTACCCCG TGCGGGCCCC CGCCACGCTC GGCCACCGGC    2520

GAGGGTCCCT TTGGTGACGT GGGCTGGGCC GGGCCCAGGA AGTGA                    2565
```

(2) INFORMATION FOR SEQ ID NO: 3:

(i) SEQUENCE CHARACTERISTICS:

(A) LENGTH: 854 amino acids
(B) TYPE: amino acid
(C) STRANDEDNESS: single
(D) TOPOLOGY: linear

(ii) MOLECULE TYPE: peptide
(xi) SEQUENCE DESCRIPTION: SEQ ID NO: 3:

```
Met Val Gln Lys Ser Arg Asn Gly Gly Val Tyr Pro Gly Pro Ser Gly
1               5                   10                  15

Glu Lys Lys Leu Lys Val Gly Phe Val Gly Leu Asp Pro Gly Ala Pro
            20                  25                  30

Asp Ser Thr Arg Asp Gly Ala Leu Leu Ile Ala Gly Ser Glu Ala Pro
        35                  40                  45

Lys Arg Gly Ser Ile Leu Ser Lys Pro Arg Ala Gly Gly Ala Gly Ala
    50                  55                  60

Gly Lys Pro Pro Lys Arg Asn Ala Phe Tyr Arg Lys Leu Gln Asn Phe
65                  70                  75                  80

Leu Tyr Asn Val Leu Glu Arg Pro Arg Gly Trp Ala Phe Ile Tyr His
                85                  90                  95
```

```
Ala Tyr Val Phe Leu Leu Val Phe Ser Cys Leu Val Leu Ser Val Phe
            100                 105                 110

Ser Thr Ile Lys Glu Tyr Glu Lys Ser Ser Glu Gly Ala Leu Tyr Ile
        115                 120                 125

Leu Glu Ile Val Thr Ile Val Val Phe Gly Val Glu Tyr Phe Val Arg
    130                 135                 140

Ile Trp Ala Ala Gly Cys Cys Cys Arg Tyr Arg Gly Trp Arg Gly Arg
145                 150                 155                 160

Leu Lys Phe Ala Arg Lys Pro Phe Cys Val Ile Asp Ile Met Val Leu
                165                 170                 175

Ile Ala Ser Ile Ala Val Leu Ala Ala Gly Ser Gln Gly Asn Val Phe
            180                 185                 190

Ala Thr Ser Ala Leu Arg Ser Leu Arg Phe Leu Gln Ile Leu Arg Met
        195                 200                 205

Ile Arg Met Asp Arg Arg Gly Gly Thr Trp Lys Leu Leu Gly Ser Val
    210                 215                 220

Val Tyr Ala His Ser Lys Glu Leu Val Thr Ala Trp Tyr Ile Gly Phe
225                 230                 235                 240

Leu Cys Leu Ile Leu Ala Ser Phe Leu Val Tyr Leu Ala Glu Lys Gly
                245                 250                 255

Glu Asn Asp His Phe Asp Thr Tyr Ala Asp Ala Leu Trp Trp Gly Leu
            260                 265                 270

Ile Thr Leu Thr Thr Ile Gly Tyr Gly Asp Lys Tyr Pro Gln Thr Trp
        275                 280                 285

Asn Gly Arg Leu Leu Ala Ala Thr Phe Thr Leu Ile Gly Val Ser Phe
    290                 295                 300

Phe Ala Leu Pro Ala Gly Ile Leu Gly Ser Gly Phe Ala Leu Lys Val
305                 310                 315                 320

Gln Glu Gln His Arg Gln Lys His Phe Glu Lys Arg Arg Asn Pro Ala
                325                 330                 335

Ala Gly Leu Ile Gln Ser Ala Trp Arg Phe Tyr Ala Thr Asn Leu Ser
            340                 345                 350
```

Arg Thr Asp Leu His Ser Thr Trp Gln Tyr Tyr Glu Arg Thr Val Thr
355 360 365

Val Pro Met Tyr Ser Ser Gln Thr Gln Thr Tyr Gly Ala Ser Arg Leu
370 375 380

Ile Pro Pro Leu Asn Gln Leu Glu Leu Leu Arg Asn Leu Lys Ser Lys
385 390 395 400

Ser Gly Leu Ala Phe Arg Lys Asp Pro Pro Pro Glu Pro Ser Pro Ser
405 410 415

Gln Lys Val Ser Leu Lys Asp Arg Val Phe Ser Ser Pro Arg Gly Val
420 425 430

Ala Ala Lys Gly Lys Gly Ser Pro Gln Ala Gln Thr Val Arg Arg Ser
435 440 445

Pro Ser Ala Asp Gln Ser Leu Glu Asp Ser Pro Ser Lys Val Pro Lys
450 455 460

Ser Trp Ser Phe Gly Asp Arg Ser Arg Ala Arg Gln Ala Phe Arg Ile
465 470 475 480

Lys Gly Ala Ala Ser Arg Gln Asn Ser Glu Glu Ala Ser Leu Pro Gly
485 490 495

Glu Asp Ile Val Asp Asp Lys Ser Cys Pro Cys Glu Phe Val Thr Glu
500 505 510

Asp Leu Thr Pro Gly Leu Lys Val Ser Ile Arg Ala Val Cys Val Met
515 520 525

Arg Phe Leu Val Ser Lys Arg Lys Phe Lys Glu Ser Leu Arg Pro Tyr
530 535 540

Asp Val Met Asp Val Ile Glu Gln Tyr Ser Ala Gly His Leu Asp Met
545 550 555 560

Leu Ser Arg Ile Lys Ser Leu Gln Ser Arg Val Asp Gln Ile Val Gly
565 570 575

Arg Gly Pro Ala Ile Thr Asp Lys Asp Arg Thr Lys Gly Pro Ala Glu
580 585 590

Ala Glu Leu Pro Glu Asp Pro Ser Met Met Gly Arg Leu Gly Lys Val

595 600 605

Glu Lys Gln Val Leu Ser Met Glu Lys Lys Leu Asp Phe Leu Val Asn
610 615 620

Ile Tyr Met Gln Arg Met Gly Ile Pro Pro Thr Glu Thr Glu Ala Tyr
625 630 635 640

Phe Gly Ala Lys Glu Pro Glu Pro Ala Pro Pro Tyr His Ser Pro Glu
645 650 655

Asp Ser Arg Glu His Val Asp Arg His Gly Cys Ile Val Lys Ile Val
660 665 670

Arg Ser Ser Ser Ser Thr Gly Gln Lys Asn Phe Ser Ala Pro Pro Ala
675 680 685

Ala Pro Pro Val Gln Cys Pro Pro Ser Thr Ser Trp Gln Pro Gln Ser
690 695 700

His Pro Arg Gln Gly His Gly Thr Ser Pro Val Gly Asp His Gly Ser
705 710 715 720

Leu Val Arg Ile Pro Pro Pro Pro Ala His Glu Arg Ser Leu Ser Ala
725 730 735

Tyr Gly Gly Gly Asn Arg Ala Ser Met Glu Phe Leu Arg Gln Glu Asp
740 745 750

Thr Pro Gly Cys Arg Pro Pro Glu Gly Thr Leu Arg Asp Ser Asp Thr
755 760 765

Ser Ile Ser Ile Pro Ser Val Asp His Glu Glu Leu Glu Arg Ser Phe
770 775 780

Ser Gly Phe Ser Ile Ser Gln Ser Lys Glu Asn Leu Asp Ala Leu Asn
785 790 795 800

Ser Cys Tyr Ala Ala Val Ala Pro Cys Ala Lys Val Arg Pro Tyr Ile
805 810 815

Ala Glu Gly Glu Ser Asp Thr Asp Ser Asp Leu Cys Thr Pro Cys Gly
820 825 830

Pro Pro Pro Arg Ser Ala Thr Gly Glu Gly Pro Phe Gly Asp Val Gly
835 840 845

```
Trp Ala Gly Pro Arg Lys
    850
```

(2) INFORMATION FOR SEQ ID NO: 4:

(i) SEQUENCE CHARACTERISTICS:

(A) LENGTH: 1425 base pairs
(B) TYPE: nucleic acid
(C) STRANDEDNESS: single
(D) TOPOLOGY: linear

(ii) MOLECULE TYPE: other nucleic acid
(xi) SEQUENCE DESCRIPTION: SEQ ID NO: 4:

```
CGCGGAGCGA GGTGGCCGCA GCGTCTCCGC GCGCGGCCCA AGCCCGGCAG GAGTGCGGAA     60
CCGCCGCCTC GGCCATGCGG CTCCCGGCCG GGGGGCCTGG GCTGGGGCCC GCGCCGCCCC    120
CCGCGCTCCG CCCCCGCTGA GCCTGAGCCC GACCCGGGGC GCCTCCCGCC AGGCACCATG    180
GTGCAGAAGT CGCGCAACGG CGGCGTATAC CCCGGCCCGA GCGGGGAGAA GAAGCTGAAG    240
GTGGGCTTCG TGGGGCTGGA CCCCGGCGCG CCCGACTCCA CCCGGGACGG GGCGCTGCTG    300
ATCGCCGGCT CCGAGGCCCC CAAGCGCGGC AGCATCCTCA GCAAACCTCG CGCGGGCGGC    360
GCGGGCGCCG GGAAGCCCCC CAAGCGCAAC GCCTTCTACC GCAAGCTGCA GAATTTCCTC    420
TACAACGTGC TGGAGCGGCC GCGCGGCTGG GCGTTCATCT ACCACGCCTA CGTGTTCCTC    480
CTGGTTTTCT CCTGCCTCGT GCTGTCTGTG TTTTCCACCA TCAAGGAGTA TGAGAAGAGC    540
TCGGAGGGGG CCCTCTACAT CCTGGAAATC GTGACTATCG TGGTGTTTGG CGTGGAGTAC    600
TTCGTGCGGA TCTGGGCCGC AGGCTGCTGC TGCCGGTACC GTGGCTGGAG GGGGCGGCTC    660
AAGTTTGCCC GGAAACCGTT CTGTGTGATT GACATCATGG TGCTCATCGC CTCCATTGCG    720
GTGCTGGCCG CCGGCTCCCA GGGCAACGTC TTTGCCACAT CTGCGCTCCG GAGCCTGCGC    780
TTCCTGCAGA TTCTGCGGAT GATCCGCATG GACCGGCGGG GAGGCACCTG GAAGCTGCTG    840
GGCTCTGTGG TCTATGCCCA CAGCAAGGAG CTGGTCACTG CCTGGTACAT CGGCTTCCTT    900
```

```
TGTCTCATCC TGGCCTCGTT CCTGGTGTAC TTGGCAGAGA AGGGGGAGAA CGACCACTTT     960

GACACCTACG CGGATGCACT CTGGTGGGGC CTGATCACGC TGACCACCAT TGGCTACGGG    1020

GACAAGTACC CCCAGACCTG GAACGGCAGG CTCCTTGCGG CAACCTTCAC CCTCATCGGT    1080

GTCTCCTTCT TCGCGCTGCC TGCAGGCATC TTGGGGTCTG GGTTTGCCCT GAAGGTTCAG    1140

GAGCAGCACA GGCAGAAGCA CTTTGAGAAG AGGCGGAACC CGGCAGCAGG CCTGATCCAG    1200

TCGGCCTGGA GATTCTACGC CACCAACCTC TCGCGCACAG ACCTGCACTC CACGTGGCAG    1260

TACTACGAGC GAACGGTCAC CGTGCCCATG TACAGGTACC GCCGCCGGGC ACCTGCCACC    1320

AAGCAACTGT TTCATTTTTT ATTTTCCATT TGTTCTTAAA CCCCACTTTT TGTTGTTCAT    1380

TATTTTGATT GATTTTTTTT CTTTAAAATG TATTTTTCAC AAAGG                    1425
```

(2) INFORMATION FOR SEQ ID NO: 5:

(i) SEQUENCE CHARACTERISTICS:

(A) LENGTH: 393 amino acids
(B) TYPE: amino acid
(C) STRANDEDNESS: single
(D) TOPOLOGY: linear

(ii) MOLECULE TYPE: peptide
(xi) SEQUENCE DESCRIPTION: SEQ ID NO: 5:

```
Met Val Gln Lys Ser Arg Asn Gly Gly Val Tyr Pro Gly Pro Ser Gly
1               5                   10                  15

Glu Lys Lys Leu Lys Val Gly Phe Val Gly Leu Asp Pro Gly Ala Pro
            20                  25                  30

Asp Ser Thr Arg Asp Gly Ala Leu Leu Ile Ala Gly Ser Glu Ala Pro
        35                  40                  45

Lys Arg Gly Ser Ile Leu Ser Lys Pro Arg Ala Gly Gly Ala Gly Ala
    50                  55                  60

Gly Lys Pro Pro Lys Arg Asn Ala Phe Tyr Arg Lys Leu Gln Asn Phe
```

```
65                  70                  75                  80

Leu Tyr Asn Val Leu Glu Arg Pro Arg Gly Trp Ala Phe Ile Tyr His
                85                  90                  95

Ala Tyr Val Phe Leu Leu Val Phe Ser Cys Leu Val Leu Ser Val Phe
            100                 105                 110

Ser Thr Ile Lys Glu Tyr Glu Lys Ser Ser Glu Gly Ala Leu Tyr Ile
        115                 120                 125

Leu Glu Ile Val Thr Ile Val Val Phe Gly Val Glu Tyr Phe Val Arg
    130                 135                 140

Ile Trp Ala Ala Gly Cys Cys Cys Arg Tyr Arg Gly Trp Arg Gly Arg
145                 150                 155                 160

Leu Lys Phe Ala Arg Lys Pro Phe Cys Val Ile Asp Ile Met Val Leu
                165                 170                 175

Ile Ala Ser Ile Ala Val Leu Ala Ala Gly Ser Gln Gly Asn Val Phe
            180                 185                 190

Ala Thr Ser Ala Leu Arg Ser Leu Arg Phe Leu Gln Ile Leu Arg Met
        195                 200                 205

Ile Arg Met Asp Arg Arg Gly Gly Thr Trp Lys Leu Leu Gly Ser Val
    210                 215                 220

Val Tyr Ala His Ser Lys Glu Leu Val Thr Ala Trp Tyr Ile Gly Phe
225                 230                 235                 240

Leu Cys Leu Ile Leu Ala Ser Phe Leu Val Tyr Leu Ala Glu Lys Gly
                245                 250                 255

Glu Asn Asp His Phe Asp Thr Tyr Ala Asp Ala Leu Trp Trp Gly Leu
            260                 265                 270

Ile Thr Leu Thr Thr Ile Gly Tyr Gly Asp Lys Tyr Pro Gln Thr Trp
        275                 280                 285

Asn Gly Arg Leu Leu Ala Ala Thr Phe Thr Leu Ile Gly Val Ser Phe
    290                 295                 300

Phe Ala Leu Pro Ala Gly Ile Leu Gly Ser Gly Phe Ala Leu Lys Val
305                 310                 315                 320
```

```
Gln Glu Gln His Arg Gln Lys His Phe Glu Lys Arg Arg Asn Pro Ala
                    325                 330                 335

Ala Gly Leu Ile Gln Ser Ala Trp Arg Phe Tyr Ala Thr Asn Leu Ser
                340                 345                 350

Arg Thr Asp Leu His Ser Thr Trp Gln Tyr Tyr Glu Arg Thr Val Thr
            355                 360                 365

Val Pro Met Tyr Arg Tyr Arg Arg Arg Ala Pro Ala Thr Lys Gln Leu
    370                 375                 380

Phe His Phe Leu Phe Ser Ile Cys Ser
385                 390
```

(2) INFORMATION FOR SEQ ID NO: 6:

(i) SEQUENCE CHARACTERISTICS:

(A) LENGTH: 581 amino acids
(B) TYPE: amino acid
(C) STRANDEDNESS: single
(D) TOPOLOGY: linear

(ii) MOLECULE TYPE: peptide
(xi) SEQUENCE DESCRIPTION: SEQ ID NO: 6:

```
Met Glu Thr Arg Gly Ser Arg Leu Thr Gly Gly Gln Gly Arg Val Tyr
1               5                   10                  15

Asn Phe Leu Glu Arg Pro Thr Gly Trp Lys Cys Phe Val Tyr His Phe
            20                  25                  30

Ala Val Phe Leu Ile Val Leu Val Cys Leu Ile Phe Ser Val Leu Ser
        35                  40                  45

Thr Ile Glu Gln Tyr Ala Ala Leu Ala Thr Gly Thr Leu Phe Trp Met
    50                  55                  60

Glu Ile Val Leu Val Val Phe Phe Gly Thr Glu Tyr Val Val Arg Leu
65                  70                  75                  80

Trp Ser Ala Gly Cys Arg Ser Lys Tyr Val Gly Leu Trp Gly Arg Leu
                85                  90                  95
```

Arg Phe Ala Arg Lys Pro Ile Ser Ile Ile Asp Leu Ile Val Val Val
100 105 110

Ala Ser Met Val Val Leu Cys Val Gly Ser Lys Gly Gln Val Phe Ala
115 120 125

Thr Ser Ala Ile Arg Gly Ile Arg Phe Leu Gln Ile Leu Arg Met Leu
130 135 140

His Val Asp Arg Gln Gly Gly Thr Trp Arg Leu Leu Gly Ser Val Val
145 150 155 160

Phe Ile His Arg Gln Glu Leu Ile Thr Thr Leu Tyr Ile Gly Phe Leu
165 170 175

Gly Leu Ile Phe Ser Ser Tyr Phe Val Tyr Leu Ala Glu Lys Asp Ala
180 185 190

Val Asn Glu Ser Gly Arg Val Glu Phe Gly Ser Tyr Ala Asp Ala Leu
195 200 205

Trp Trp Gly Val Val Thr Val Thr Thr Ile Gly Tyr Gly Asp Lys Val
210 215 220

Pro Gln Thr Trp Val Gly Lys Thr Ile Ala Ser Cys Phe Ser Val Phe
225 230 235 240

Ala Ile Ser Phe Phe Ala Leu Pro Ala Gly Ile Leu Gly Ser Gly Phe
245 250 255

Ala Leu Lys Val Gln Gln Lys Gln Arg Gln Lys His Phe Asn Arg Gln
260 265 270

Ile Pro Ala Ala Ala Ser Leu Ile Gln Thr Ala Trp Arg Cys Tyr Ala
275 280 285

Ala Glu Asn Pro Asp Ser Ser Thr Trp Lys Ile Tyr Ile Arg Lys Ala
290 295 300

Pro Arg Ser His Thr Leu Leu Ser Pro Ser Pro Lys Pro Lys Lys Ser
305 310 315 320

Val Val Val Lys Lys Lys Lys Phe Lys Leu Asp Lys Asp Asn Gly Val
325 330 335

Thr Pro Gly Glu Lys Met Leu Thr Val Pro His Ile Thr Cys Asp Pro

340 345 350

Pro Glu Glu Arg Arg Leu Asp His Phe Ser Val Asp Gly Tyr Asp Ser
355 360 365

Ser Val Arg Lys Ser Pro Thr Leu Leu Glu Val Ser Met Pro His Phe
370 375 380

Met Arg Thr Asn Ser Phe Ala Glu Asp Leu Asp Leu Glu Gly Glu Thr
385 390 395 400

Leu Leu Thr Pro Ile Thr His Ile Ser Gln Leu Arg Glu His His Arg
405 410 415

Ala Thr Ile Lys Val Ile Arg Arg Met Gln Tyr Phe Val Ala Lys Lys
420 425 430

Lys Phe Gln Gln Ala Arg Lys Pro Tyr Asp Val Arg Asp Val Ile Glu
435 440 445

Gln Tyr Ser Gln Gly His Leu Asn Leu Met Val Arg Ile Lys Glu Leu
450 455 460

Gln Arg Arg Leu Asp Gln Ser Ile Gly Lys Pro Ser Leu Phe Ile Ser
465 470 475 480

Val Ser Glu Lys Ser Lys Asp Arg Gly Ser Asn Thr Ile Gly Ala Arg
485 490 495

Leu Asn Arg Val Glu Asp Lys Val Thr Gln Leu Asp Gln Arg Leu Ala
500 505 510

Leu Ile Thr Asp Met Leu His Gln Leu Leu Ser Leu His Gly Gly Ser
515 520 525

Thr Pro Gly Ser Gly Gly Pro Pro Arg Glu Gly Gly Ala His Ile Thr
530 535 540

Gln Pro Cys Gly Ser Gly Gly Ser Val Asp Pro Glu Leu Phe Leu Pro
545 550 555 560

Ser Asn Thr Leu Pro Thr Tyr Glu Gln Leu Thr Val Pro Arg Arg Gly
565 570 575

Pro Asp Glu Gly Ser
580

(2) INFORMATION FOR SEQ ID NO: 7:

(i) SEQUENCE CHARACTERISTICS:

(A) LENGTH: 872 amino acids
(B) TYPE: amino acid
(C) STRANDEDNESS: single
(D) TOPOLOGY: linear

(ii) MOLECULE TYPE: peptide
(xi) SEQUENCE DESCRIPTION: SEQ ID NO: 7:

```
Met Gly Leu Lys Ala Arg Arg Ala Ala Gly Ala Ala Gly Gly Gly Gly
1               5                   10                  15

Gly Glu Gly Gly Gly Gly Gly Gly Gly Ala Ala Asn Pro Ala Gly Gly
            20                  25                  30

Asp Ser Ala Val Ala Gly Asp Glu Glu Arg Lys Val Gly Leu Ala Pro
        35                  40                  45

Gly Asp Val Glu Gln Val Thr Leu Ala Leu Gly Thr Gly Ala Asp Lys
    50                  55                  60

Asp Gly Thr Leu Leu Leu Glu Gly Gly Gly Arg Glu Glu Gly Gln Arg
65                  70                  75                  80

Arg Thr Pro Gln Gly Ile Gly Leu Leu Ala Lys Thr Pro Leu Ser Arg
                85                  90                  95

Pro Val Lys Arg Asn Asn Ala Lys Tyr Arg Arg Ile Gln Thr Leu Ile
            100                 105                 110

Tyr Asp Ala Leu Glu Arg Pro Arg Gly Trp Ala Leu Leu Tyr His Ala
        115                 120                 125

Leu Val Phe Leu Ile Val Leu Gly Cys Leu Ile Leu Ala Val Leu Thr
    130                 135                 140

Thr Phe Lys Glu Tyr Glu Thr Val Ser Gly Asp Trp Leu Leu Val Pro
145                 150                 155                 160

Glu Thr Phe Ala Ile Phe Ile Phe Gly Ala Glu Phe Ala Leu Arg Ile
                165                 170                 175
```

Trp Ala Ala Gly Cys Cys Cys Arg Tyr Lys Gly Trp Arg Gly Arg Leu
180 185 190

Lys Phe Ala Arg Lys Pro Leu Cys Met Leu Asp Ile Phe Val Leu Ile
195 200 205

Ala Ser Val Pro Val Val Ala Val Gly Asn Gln Gly Asn Val Leu Ala
210 215 220

Thr Ser Leu Arg Ser Leu Arg Phe Leu Gln Ile Leu Arg Met Leu Arg
225 230 235 240

Met Asp Arg Arg Gly Gly Thr Trp Lys Leu Leu Gly Ser Ala Ile Cys
245 250 255

Ala His Ser Lys Glu Leu Ile Thr Ala Trp Tyr Ile Gly Phe Leu Thr
260 265 270

Leu Ile Leu Ser Ser Phe Leu Val Tyr Leu Val Glu Lys Asp Val Pro
275 280 285

Glu Met Asp Ala Gln Gly Glu Glu Met Lys Glu Glu Phe Glu Thr Tyr
290 295 300

Ala Asp Ala Leu Trp Trp Gly Leu Ile Thr Leu Ala Thr Ile Gly Tyr
305 310 315 320

Gly Asp Lys Thr Pro Lys Thr Trp Glu Gly Arg Leu Ile Ala Ala Thr
325 330 335

Phe Ser Leu Ile Gly Val Ser Phe Phe Ala Leu Pro Ala Gly Ile Leu
340 345 350

Gly Ser Gly Leu Ala Leu Lys Val Gln Glu Gln His Arg Gln Lys His
355 360 365

Phe Glu Lys Arg Arg Lys Pro Ala Ala Glu Leu Ile Gln Ala Ala Trp
370 375 380

Arg Tyr Tyr Ala Thr Asn Asn Arg Leu Asp Leu Val Ala Thr Trp Arg
385 390 395 400

Phe Tyr Glu Ser Val Val Ser Phe Pro Phe Phe Arg Lys Glu Gln Leu
405 410 415

Glu Ala Ala Ala Ser Gln Lys Leu Gly Leu Leu Asp Arg Val Arg Leu

420 425 430

Ser Asn Pro Arg Gly Ser Asn Thr Lys Gly Lys Leu Phe Thr Pro Leu
435 440 445

Asn Val Asp Ala Ile Glu Glu Ser Pro Ser Lys Glu Pro Lys Pro Val
450 455 460

Gly Leu Asn Asn Lys Glu Arg Phe Arg Thr Ala Phe Arg Met Lys Ala
465 470 475 480

Tyr Ala Phe Trp Gln Ser Ser Glu Asp Ala Gly Thr Gly Asp Pro Met
485 490 495

Thr Glu Asp Arg Gly Tyr Gly Asn Asp Phe Leu Ile Glu Asp Met Ile
500 505 510

Pro Thr Leu Lys Ala Ala Ile Arg Ala Val Arg Ile Leu Gln Phe Arg
515 520 525

Leu Tyr Lys Lys Lys Phe Lys Glu Thr Leu Arg Pro Tyr Asp Val Lys
530 535 540

Asp Val Ile Glu Gln Tyr Ser Ala Gly His Leu Asp Met Leu Ser Arg
545 550 555 560

Ile Lys Tyr Leu Gln Thr Arg Ile Asp Met Ile Phe Thr Pro Gly Pro
565 570 575

Pro Ser Thr Pro Lys His Lys Lys Ser Gln Lys Gly Ser Ala Phe Thr
580 585 590

Tyr Pro Ser Gln Gln Ser Pro Arg Asn Glu Pro Tyr Val Ala Arg Ala
595 600 605

Ala Thr Ser Glu Thr Glu Asp Gln Ser Met Met Gly Lys Phe Val Lys
610 615 620

Val Glu Arg Gln Val His Asp Met Gly Lys Lys Leu Asp Phe Leu Val
625 630 635 640

Asp Met His Met Gln His Met Glu Arg Leu Gln Val His Val Thr Glu
645 650 655

Tyr Tyr Pro Thr Lys Gly Ala Ser Ser Pro Ala Glu Gly Glu Lys Lys
660 665 670

```
Glu Asp Asn Arg Tyr Ser Asp Leu Lys Thr Ile Ile Cys Asn Tyr Ser
        675                 680                 685

Glu Ser Gly Pro Pro Asp Pro Pro Tyr Ser Phe His Gln Val Pro Ile
    690                 695                 700

Asp Arg Val Gly Pro Tyr Gly Phe Phe Ala His Asp Pro Val Lys Leu
705                 710                 715                 720

Thr Arg Gly Gly Pro Ser Ser Thr Lys Ala Gln Ala Asn Leu Pro Ser
                725                 730                 735

Ser Gly Ser Thr Tyr Ala Glu Arg Pro Thr Val Leu Pro Ile Leu Thr
            740                 745                 750

Leu Leu Asp Ser Cys Val Ser Tyr His Ser Gln Thr Glu Leu Gln Gly
        755                 760                 765

Pro Tyr Ser Asp His Ile Ser Pro Arg Gln Arg Arg Ser Ile Thr Arg
    770                 775                 780

Asp Ser Asp Thr Pro Leu Ser Leu Met Ser Val Asn His Glu Glu Leu
785                 790                 795                 800

Glu Arg Ser Pro Ser Gly Phe Ser Ile Ser Gln Asp Arg Asp Asp Tyr
                805                 810                 815

Val Phe Gly Pro Ser Gly Gly Ser Ser Trp Met Arg Glu Lys Arg Tyr
            820                 825                 830

Leu Ala Glu Gly Glu Thr Asp Thr Asp Thr Asp Pro Phe Thr Pro Ser
        835                 840                 845

Gly Ser Met Pro Met Ser Ser Thr Gly Asp Gly Ile Ser Asp Ser Ile
    850                 855                 860

Trp Thr Pro Ser Asn Lys Pro Thr
865                 870
```

(2) INFORMATION FOR SEQ ID NO: 8:

(i) SEQUENCE CHARACTERISTICS:

(A) LENGTH: 825 amino acids
(B) TYPE: amino acid
(C) STRANDEDNESS: single
(D) TOPOLOGY: linear

(ii) MOLECULE TYPE: peptide
(xi) SEQUENCE DESCRIPTION: SEQ ID NO: 8:

```
Gly Asp Val Glu Gln Val Thr Leu Ala Leu Gly Ala Gly Ala Asp Lys
1               5                   10                  15

Asp Gly Thr Leu Leu Leu Glu Gly Gly Gly Arg Asp Glu Gly Gln Arg
            20                  25                  30

Arg Thr Pro Gln Gly Ile Gly Leu Leu Ala Lys Thr Pro Leu Ser Arg
        35                  40                  45

Pro Val Lys Arg Asn Asn Ala Lys Tyr Arg Arg Ile Gln Thr Leu Ile
    50                  55                  60

Tyr Asp Ala Leu Glu Arg Pro Arg Gly Trp Ala Leu Leu Tyr His Ala
65                  70                  75                  80

Leu Val Phe Leu Ile Val Leu Gly Cys Leu Ile Leu Ala Val Leu Thr
                85                  90                  95

Thr Phe Lys Glu Tyr Glu Thr Val Ser Gly Asp Trp Leu Leu Leu Leu
            100                 105                 110

Glu Thr Phe Ala Ile Phe Ile Phe Gly Ala Glu Phe Ala Leu Arg Ile
        115                 120                 125

Trp Ala Ala Gly Cys Cys Cys Arg Tyr Lys Gly Trp Arg Gly Arg Leu
    130                 135                 140

Lys Phe Ala Arg Lys Pro Leu Cys Met Leu Asp Ile Phe Val Leu Ile
145                 150                 155                 160

Ala Ser Val Pro Val Val Ala Val Gly Asn Gln Gly Asn Val Leu Ala
                165                 170                 175

Thr Ser Leu Arg Ser Leu Arg Phe Leu Gln Ile Leu Arg Met Leu Arg
            180                 185                 190

Met Asp Arg Arg Gly Gly Thr Trp Lys Leu Leu Gly Ser Ala Ile Cys
        195                 200                 205

Ala His Ser Lys Glu Leu Ile Thr Ala Trp Tyr Ile Gly Phe Leu Thr
```

210 215 220

Leu Ile Leu Ser Ser Phe Leu Val Tyr Leu Val Glu Lys Asp Val Pro
225 230 235 240

Glu Val Asp Ala Gln Gly Glu Glu Met Lys Glu Glu Phe Glu Thr Tyr
245 250 255

Ala Asp Ala Leu Trp Trp Gly Leu Ile Thr Leu Ala Thr Ile Gly Tyr
260 265 270

Gly Asp Lys Thr Pro Lys Thr Trp Glu Gly Arg Leu Ile Ala Ala Thr
275 280 285

Phe Ser Leu Ile Gly Val Ser Phe Phe Ala Leu Pro Ala Gly Ile Leu
290 295 300

Gly Ser Gly Leu Ala Leu Lys Val Gln Glu Gln His Arg Gln Lys His
305 310 315 320

Phe Glu Lys Arg Arg Lys Pro Ala Ala Glu Leu Ile Gln Ala Ala Trp
325 330 335

Arg Tyr Tyr Ala Thr Asn Pro Asn Arg Ile Asp Leu Val Ala Thr Trp
340 345 350

Arg Phe Tyr Glu Ser Val Val Ser Phe Pro Phe Phe Arg Lys Glu Gln
355 360 365

Leu Glu Ala Ala Ser Ser Gln Lys Leu Gly Leu Leu Asp Arg Val Arg
370 375 380

Leu Ser Asn Pro Arg Gly Ser Asn Thr Lys Gly Lys Leu Phe Thr Pro
385 390 395 400

Leu Asn Val Asp Ala Ile Glu Glu Ser Pro Ser Lys Glu Pro Lys Pro
405 410 415

Val Gly Leu Asn Asn Lys Glu Arg Phe Arg Thr Ala Phe Arg Met Lys
420 425 430

Ala Tyr Ala Phe Trp Gln Ser Ser Glu Asp Ala Gly Thr Gly Asp Pro
435 440 445

Met Ala Glu Asp Arg Gly Tyr Gly Asn Asp Phe Pro Ile Glu Asp Met
450 455 460

Ile Pro Thr Leu Lys Ala Ala Ile Arg Ala Val Arg Ile Leu Gln Phe
465 470 475 480

Arg Leu Tyr Lys Lys Lys Phe Lys Glu Thr Leu Arg Pro Tyr Asp Val
485 490 495

Lys Asp Val Ile Glu Gln Tyr Ser Ala Gly His Leu Asp Met Leu Ser
500 505 510

Arg Ile Lys Tyr Leu Gln Thr Arg Ile Asp Met Ile Phe Thr Pro Gly
515 520 525

Pro Pro Ser Thr Pro Lys His Lys Lys Ser Gln Lys Gly Ser Ala Phe
530 535 540

Thr Phe Pro Ser Gln Gln Ser Pro Arg Asn Glu Pro Tyr Val Ala Arg
545 550 555 560

Pro Ser Thr Ser Glu Ile Glu Asp Gln Ser Met Met Gly Lys Phe Val
565 570 575

Lys Val Glu Arg Gln Val Gln Asp Met Gly Lys Lys Leu Asp Phe Leu
580 585 590

Val Asp Met His Met Gln His Met Glu Arg Leu Gln Val Gln Val Thr
595 600 605

Glu Tyr Tyr Pro Thr Lys Gly Thr Ser Ser Pro Ala Glu Ala Glu Lys
610 615 620

Lys Glu Asp Asn Arg Tyr Ser Asp Leu Lys Thr Ile Ile Cys Asn Tyr
625 630 635 640

Ser Glu Thr Gly Pro Pro Glu Pro Pro Tyr Ser Phe His Gln Val Thr
645 650 655

Ile Asp Lys Val Ser Pro Tyr Gly Phe Phe Ala His Asp Pro Val Asn
660 665 670

Leu Pro Arg Gly Gly Pro Ser Ser Gly Lys Val Gln Ala Thr Pro Pro
675 680 685

Ser Ser Ala Thr Thr Tyr Val Glu Arg Pro Thr Val Leu Pro Ile Leu
690 695 700

Thr Leu Leu Asp Ser Arg Val Ser Cys His Ser Gln Ala Asp Leu Gln
705 710 715 720

Gly Pro Tyr Ser Asp Arg Ile Ser Pro Arg Gln Arg Arg Ser Ile Thr
725 730 735

Arg Asp Ser Asp Thr Pro Leu Ser Leu Met Ser Val Asn His Glu Glu
740 745 750

Leu Glu Arg Ser Pro Ser Gly Phe Ser Ile Ser Gln Asp Arg Asp Asp
755 760 765

Tyr Val Phe Gly Pro Asn Gly Gly Ser Ser Trp Met Arg Glu Lys Arg
770 775 780

Tyr Leu Ala Glu Gly Glu Thr Asp Thr Asp Thr Asp Pro Phe Thr Pro
785 790 795 800

Ser Gly Ser Met Pro Leu Ser Ser Thr Gly Asp Gly Ile Ser Asp Ser
805 810 815

Val Trp Thr Pro Ser Asn Lys Pro Ile
820 825

## Description

### Field of the Invention

The present invention relates to nucleic acid and amino acid sequences of a novel human brain-derived potassium channel polypeptide and to the use of these sequences to identify compounds that modulate the biological and/or pharmacological activity of the native biomolecule. The invention is also related to the diagnosis, study, prevention, and treatment of pathophysiological disorders related to or mediated by the biological molecule.

### Background of the Invention

Potassium channels are integral membrane proteins of great molecular and functional diversity and are present in virtually all mammalian cells. Neuronal potassium channels in the brain are primarily responsible for maintaining a negative resting membrane potential, as well as controlling membrane repolarization following an action potential. Depending on the sub-family to which a given potassium channel belongs, it can be activated by a change in the membrane potential, an increase in the intracellular concentration of $Ca^{2+}$, or binding of ligands to their receptors including acetycholine, adrenaline, dopamine, galanin, calcitonin gene-related peptide, somatostatin, and ATP. The pharmacological identity of various voltage-gated potassium-channels is established by their sensitivity to standard compounds capable of blocking one or more types of potassium channels. These compounds, known as potassium-channel blockers, include tetraethylammonium chloride (TEA), 4-aminopyridine (4-AP, as well as 2-AP and 3-AP), 3,4- and 2,3-diaminopyridine, $BaCl_2$, CsCl, strychnine, phencyclidine, pyridostigmine, 9-aminoacridine, DuP-996 (3,3-bis (4-pyridinylmethyl)-1-phenylindolin-2-one; linopiridine), clofilium, quinidine, aminoquinolines and quinine. Extensive molecular and functional diversity renders potassium channels a potential target of drug discovery for a variety of pathophysiological indications, including memory degeneration, stroke, epilepsy, multiple sclerosis, Huntington's chorea, anxiety, depression, psychosis, urinary incontinence, diabetes, asthma, premature labour, hypertension, cardiac ischaemia, migraine headaches, autoimmune diseases, cancer, graft rejections, and arrhythmias. Toral, J., et al., Use of Cultured Human Neuroblastoma Cells in Rapid Discovery of the Voltage-gated Potassium-channel Blockers, J. Pharm. Pharmacol., 46:731 (1994).

Mammalian brain potassium channel-mediated neuronal depolarization prolongs neuronal survival *in vitro* and has become a major model of examining neuronal apoptosis. *See, e.g.,* Shan Ping Yu, et al., Mediation of Neuronal Apoptosis by Enhancement of Outward Potassium Current, Science, 278:114 (1997). Apoptotic neuronal death is a key mechanism that regulates the elimination of neuronal precursor cells during mammalian brain development and is integral to normal neurophysiology. Apoptosis induced by potassium deprivation has been demonstrated to trigger a lethal cascade of events that includes specific RNA and protein synthesis, induction of interleukin 1-converting enzyme-like protease activity, and generation of free radicals. Neuronal apoptosis also mediates certain pathophysiological disease states. Current investigators focus on the significance of the premature death of adult neurons in human neurodegenerative diseases. Weller, M., et al., Developmental and Genetic Regulation of Programmed Neuronal Death, J. Neural Transm. Suppl., 50:115 (1997).

The importance of the bioactive integrity of normal neurophysiology is readily perceived in clinical medicine today a result of the striking demonstration of the consequences of neuronal dysfunction. Neurodevelopmental and neurophysiological disorders such as *shizophrenia,* anxiety, and depression are significant physiological events involving neuronal dysfunction. *Shizophrenia* as well as neurodegenerative conditions including *Huntington's disease, Alzheimers, Parkinson's. Lou Gehrig's,* have been recently proposed to be the result mismanaged neuronal apoptosis. Aberrant neurodevelopmental processes have been attributed to both premature neuronal death as well as the failure to eliminate excess neurons. Furthermore, catastrophic neurodegeneration in neurophysiological diseases such as *stroke and epilepsy* are proposed to be a molecular collaboration between necrosis and apoptosis. Stahl, S.M., J. Clin. Psychiatry, Apoptosis: Neuronal Death by Design, (5):183 (1997).

Advances in cloning of KQT potassium channels have greatly expanded the understanding of the structure of these macromolecules. Wang, Q., et al., Nature Genet., 12:17 (1996); Wei, A., et al., Neuropharmacology, Vol. 35(7): 805 (1996); Curran, M.E., et al., Cell, 80:795 (1995); Barhanin, J., et al., Nature, 384. 78-80 (1996); Sanguinetti, M. C., et al., Nature, 384:80 (1996); Attali, B.,Nature, 384:25 (1996).

Recently a full-length 1.5 kb mRNA transcript has been described which is purported to encode a 393 amino acid polypeptide of a potassium channel from a human neuroblastoma line. Yokoyama, M., et. al., Identification and Cloning of Neuroblastoma-Specifcc and Nerve Tissue-Specific Genes through Compiled Express Profiles, DNA Research, 3:311 (1996).

The central role of potassium channels in regulating numerous neurophysiological functions makes them particularly important targets for therapeutic development. Unfortunately, no compound has yet been found to block *potassium channels* exclusively in human neurons. Accordingly, there remains a need to identify a potassium channel of differentiated neuron-origin as a pharmacological target for screening candidate compounds for the modulation of neurophysiological activity. Such modulators are potentially useful in treating disorders manifested by dysfunctional neurons. Selective channel blockers are moreover needed to address the neuropharmacology of potassium channels with regard to neurodevelopmental and neurophysiological disorders as well as to brain neuron apoptosis.

The identification and characterization of biomolecules responsible for the activation and manifestation of neuronal apoptosis is of paramount importance toward the development of therapeutic compounds for the control and/or interruption of neurophysiological disorders. Stahl, S.M., J. Clin. Psychiatry, Apoptosis: Neuronal Death by Design, (5): 183 (1997). The availability of a functional assay with a considerable through-put is an indispensable part of any drug discovery program focusing on potassium channel modulators. Such an assay should be able to screen hundreds of compounds on a routine basis. Toral, J., et al., Use of Cultured Human Neuroblastoma Cells in Rapid Discovery of the Voltage-gated Potassium-channel Blockers, J. Pharm. Pharmacol., 46:731(1994).

## Summary of the Invention

The present invention is directed to a purified polynucleotide comprising a nucleic acid sequence encoding the polypeptide of SEQ ID NO:3, said polypeptide possessing the ability to allow transmembrane potassium ion flow and/or transport. In some embodiments, the polynucleotide comprises the sequence of SEQ ID NO:2. The present invention is also directed to expression vectors comprising the polynucleotides of the invention, as well as host cells transformed with said expression vectors.

The present invention is also directed to a purified polypeptide of SEQ ID NO:3, said polypeptide possessing the ability to allow transmembrane potassium ion flow and/or transport.

The present invention is further directed to a method for producing cells which express a polypeptide of SEQ ID NO:3, said polypeptide possessing the ability to allow transmembrane potassium ion flow and/or transport, said method comprising:

a) transforming suitable host cells with a polynucleotide comprising a nucleic acid that encodes a polypeptide of SEQ ID NO:3, said polypeptide possessing the ability to allow transmembrane potassium ion flow and/or transport; and

b) selecting cells expressing the polypeptide encoded by the introduced nucleic acid.

The present invention is further directed to a method for producing a polypeptide of SEQ ID NO:3, said polypeptide possessing the ability to allow transmembrane potassium ion flow and/or transport, said method comprising:

a) culturing a host cell transformed with an expression vector of the present invention under conditions suitable for the expression of said polypeptide; and

b) recovering said polypeptide from the host cell culture.

The present invention is further directed to a method of identifying compounds that modulate the biological activity of a potassium channel, comprising:

a) combining a candidate compound modulator of biological activity with a polypeptide of SEQ ID NO:3, said polypeptide possessing the ability to allow transmembrane potassium ion flow and/or transport; and

b) measuring an effect of the candidate compound modulator on the transmembrane potassium ion flow and/or transport ability of the polypeptide.

The present invention is further directed to a method of identifying compounds that modulate the biological activity of a potassium channel comprising:

a) combining a candidate compound modulator of a potassium channel biological activity with a host-cell expressing a polypeptide of SEQ ID NO:3, said polypeptide possessing the ability to allow transmembrane potassium ion flow and/or transport; and

b) measuring an effect of the candidate compound modulator on the transmembrane potassium ion flow and/or transport ability of the polypeptide referred to in step (a). In some embodiments, compounds are identified as those that modulate the biological activity of a potassium channel, are modulators of neurophysiology.

The present invention is further directed to the use of a purified polypeptide of SEQ ID NO:3, or host cells expressing said polypeptide, or a preparation made from said cells in a screen for compounds that modulate the transmembrane potassium ion flow and/or transport ability of said polypeptide.

## Brief Description of the Figures

Figure 1 displays SEQ ID NO: 1 which is a 3029 base cDNA nucleic acid sequence which encodes the novel human brain-derived potassium channel polypeptide which is tissue-specific to differentiated neurons as described herein.

Figure 2 displays SEQ ID NO:2 which is the 2565 base translated structural region, ATG to TGA, of the cDNA nucleic acid sequence which encodes the human brain-derived potassium channel polypeptide tissue-specific to differentiated neurons as described herein.

Figure 3 displays SEQ ID NO:3 which is the 854 amino acid residue sequence of the human brain-derived potassium channel polypeptide tissue-specific to differentiated neurons as described herein.

Figure 4 shows SEQ ID NO:4 which is the 1425 base cDNA sequence pertaining to the HNSPC potassium channel. Genbank accession No. D82346.

Figure 5 shows SEQ ID NO:5 which is the 393 amino acid residue sequence of the recently described HNSPC potassium channel. Genbank accession No. D82346; Yokoyama, M., et. al., Identification and Cloning of Neuroblastoma-Specific and Nerve Tissue-Specific Genes through Compiled Express Profiles, DNA Research, 3:311 (1996).

Figure 6 shows SEQ ID NO:6 which is the 581 amino acid residue sequence of the recently described human KvLQT1 potassium channel. Genbank accession Nos. U40990, U71077; Barhanin, J., et al., Nature, 384, 78-80 (1996); Sanguinetti, M. C., et al., Nature, 384:80 (1996).

Figure 7 displays a comparison alignment between the amino acid residue sequence of the novel human brain-derived potassium channel polypeptide described herein (SEQ ID NO:3), and the amino acid residue sequences of potassium channel polypeptides HNSPC (SEQ ID NO:5) and hKvLQT1 (SEQ ID NO:6). Conserved amino acid residues are boxed. Dashes represent gaps introduced to optimize the alignment. Sequences shown in this figure were produced using the multisequence alignment program of DNASTAR software (DNASTAR Inc, Madison WI).

Figure 8 displays a hydropathy plot of the amino acid residue sequence of the novel human brain-derived potassium channel polypeptide described herein (SEQ ID NO:3), and the amino acid residue sequences of potassium channel polypeptides HNSPC (SEQ ID NO:5) and hKvLQT1 (SEQ ID NO:6) showing predicted transmembrane segments. Kyte, J., Doolittle, R.F., J. Mol. Biol., 157:105 (1982); DNASTAR Inc, Madison WI.

Figure 9 displays Northern blot analyses of multiple tissues, (Panel A) using nucleic acid probe no. 1 which is specific to the human brain-derived potassium channel polypeptide coding region described herein (SEQ ID NO:2); (Panel B) and (Panel C) using nucleic acid probe no.2 which is common to HNSPC cDNA (SEQ ID NO:4) and the human brain-derived potassium channel polypeptide coding region described herein (SEQ ID NO:2).

Figure 10 displays Northern blot analyses of distinct regions of human brain, (Panel A) using nucleic acid probe no. which is specific to the human brain-derived potassium channel polypeptide coding region described herein (SEQ ID NO:2); (Panel B) using nucleic acid probe no.2 which is common to HNSPC cDNA (SEQ ID NO:4) and the human brain-derived potassium channel polypeptide coding region described herein (SEQ ID NO:2).

Figure 11 displays Northern blot analyses of human adult brain and human fetal brain, uninduced and neuronal induced neuroblastoma cell line imr-32 and astrocytes, (Panel A) using nucleic acid probe no. 1 which is specific to the human brain-derived potassium channel polypeptide coding region described herein (SEQ ID NO:2); (Panel B) using nucleic acid probe no.2 which is common to HNSPC cDNA (SEQ ID NO:4) and the human brain-derived potassium channel polypeptide coding region described herein (SEQ ID NO:2).

Figure 12 illustrates the expression and activity of SEQ ID NO:3 functional $K^+$ currents in mammalian COS-7 and HEK 293 cell lines:

**A.** Slowly activating, non-inactivating human brain-derived potassium channel subunit current in a transfected COS-7 cell vs. lack of current in parental cell. Solid line over current is a monoexponential fit ($\tau$ = 216 ms).

**B.** Normalized mean ($\pm$ SEM; n=3-12) steady-state activation voltage-dependency of SEQ ID N0:3 subunit current expressed in COS-7 and HEK 293 cells. Solid lines are Boltzman fits to data (COS-7: $V_{1/2}$: -6 mV, slope factor: 13; HEK293: $V_{1/2}$: 12 mV, slope factor:18.

**C.** Dependence of SEQ ID NO:3 subunit (HEK 293cells) reversal potential on $[K^+]_0$. Reversal potential was measured from peak tail currents, and liquid junction potentials were corrected. Linear regression (solid line) on mean ($\pm$SEM; n=3-6) data points reveals a slope of 59 mV per 10-fold increase $[K^+]_0$, indicative of a highly $K^+$-selective current, as predicted by the Nernst equation.

**D.** Concentration-dependent inhibition of SEQ ID NO:3 subunit by tetraethylammonium (TEA). Red line is a Hill fit to mean ($\pm$SEM, n in parentheses) data points($IC_{50}$: 0.13 mM, slope factor: 0.59).

Figure 13 illustrates the effects on K+ current during co-transfection of COS-7 and HEK 293 cells with SEQ ID NO:

2 (long) and SEQ ID NO:4 (short).

**A.** Typical superimposed family of currents elicited by a series of increasing voltage steps in a COS-7 cell transfected with SEQ ID NO:2.
**B.** Superimposed family of currents obtained with the same protocol as in (A) from a cell co-transfected with SEQ ID NO:4 and SEQ ID N0:2 at a ratio of 5:1.
**C**. Summary data (mean ± SEM, n's in parentheses under x-axis) of current densities from all COS-7 and HEK 293 cells co-transfected with combinations of SEQ ID NO:4 and SEQ ID NO:2 at the indicated ratios (* denotes difference from parental cell current density at $p<0.05$, * * denotes $p<0.001$ levels by one-way ANOVA).

Figure 14 illustrates a comparison of activation voltage dependencies and kinetics for currents in COS-7 cells transfected with hKvQT2L (SEQ ID NO:2) (+ empty vector) vs. cells co-transfected with hKvQT2S (SEQ ID NO:4) + hKvQT2L(SEQ ID NO:2) at a 5:1 ratio. Neither activation voltage dependencies (A) nor activation kinetics (B) differed significantly for currents in cells transfected with KvQT2L vs. cells co-transfected with KvQT2L + KvQT2S (one-way ANOVA's; $p > 0.05$).

**A.** Normalized mean (± SEM; n=5-6) steady-state activation voltage-dependency of currents. Solid lines are Boltzman fits to data (Vector + KvQT2L: $V_{½}$: -6 mV, slope factor: 13; KvQT2S +KvQT2L 5:1: $V_{½}$: -2 mV, slope factor: 12).
**B.** Mean (± SEM; n=5-6) τ's obtained from monoexponential fits to activation of currents elicited by incremented range of voltage steps.

Figure 15 shows Northern blot analysis of a human brain tumor. An approximately 2Kb band, corresponding to the short splice variant of hKvQT2 (SEQ ID NO:4), was detected in brain tumor lanes, but not in adjacent non-tumor cells from the same brain, or from tumors of non-brain origin that metastasized to the brain.
Figure 16 shows Northern blot analysis of undifferentiated, differentiated and beta-amyloid treated SN56 and SN48 septal cell lines. SEQ ID NO:1/SEQ ID NO:2 specific bands were detected in the cholinergic SN 56 cells but not in SN48 cells (which lack the potassium current).
Figure 17 shows SEQ ID N0:7 and SEQ ID N0:8 which are the respective amino acid residue sequences of rat and human KvQT3.

## Detailed Description of the Invention

Unless defined otherwise, all technical and scientific terms used herein have the same meaning as is commonly understood by one of skill in the art to which this invention belongs.
Biological activity as used herein refers to the ability to allow transmembrane potassium ion flow and/or transport or regulate transmembrane potassium ion flow and/or transport or the ability of a subunit to bind another subunit, ligand, or cofactor and/or otherwise modulate the pharmacological activity of a potassium channel.
Nucleic acid sequence as used herein refers to an oligonucleotide, nucleotide or polynucleotide sequence, and fragments or portions thereof, and to DNA or RNA of genomic or synthetic origin which may be double-stranded or single-stranded whether representing the sense or antisense strand. Similarly, amino acid and/or residue sequence as used herein refers to peptide or protein sequences or portions thereof.
Purified as used herein refers to molecules, either nucleic acid or amino acid sequences, that are removed from their natural environment and isolated or separated from at least one other component with which they are naturally associated.
As used herein, a functional derivative of a potassium channel molecular structure or polypeptide disclosed herein is a compound or entity that possesses a biological activity (either functional or structural) that is substantially similar to SEQ ID N0:3. The term "functional derivatives" is intended to include the "fragments," "variants," "degenerate variants," "analogs" and "homologues", and to "chemical derivatives". The term "variant" is meant to refer to a molecule substantially similar in structure and function to either an entire potassium channel molecule or to a fragment thereof. A molecule is "substantially similar" to a potassium channel polypeptide if both molecules have substantially similar structures or if both molecules possess similar biological activity. The term "analog" refers to a molecule substantially similar in function to either an entire native polypeptide, or to a C- terminal fragment thereof.
The term "modulation" is used herein to refer to the capacity to either enhance or inhibit a functional property of a potassium channel. The term "modulation" is also used herein to refer to the capacity to affect the biophysical activity of a neuron.
Modulation or regulation of biological activity as used herein refers to binding, blocking, antagonization, repression, neutralization, or sequestration, of a potassium channel biomolecular structure including but not limited to the novel potassium channel polypeptide described herein; as well as *up regulation* or agonization or activation of a potassium channel by a compound identified by means described herein.

Modulate neurophysiology as used herein refers to the biophysiological regulation of neurons and the treatment of pathophysiological disorders related to or mediated by neurons. "Substantially as depicted" as used herein refers to functional derivative proteins, peptides and DNA sequences that may have changes but perform substantially the same biological function in substantially the same way.

Biologically active fragment as used herein includes peptides which have been truncated with respect to the N- or C-termini, or both; or the corresponding 5' or 3' end, or both, of the corresponding polynucleotide coding region, which fragments perform substantially the same biological function or encode peptides which perform substantially the same function in substantially the same way. The term "biologically active" also refers to the activity of a homolog or analog entity having structural, regulatory or biochemical functions substantially the same as the naturally occurring entity.

Expression vector as used herein refers to nucleic acid vector constructions which have components to direct the expression of heterologous protein coding regions including coding regions of the present invention through accurate transcription and translation in host cells. Expression vectors usually contain a promoter to direct polymerases to transcribe the heterologous coding region, a cloning site at which to introduce the heterologous coding region, and usually polyadenylation signals. Expression vectors include but are not limited to plasmids, retroviral vectors, viral and synthetic vectors.

Transformed host cells as used herein refer to cells which have coding regions of the present invention stably integrated into their genome, or episomally present as replicating or nonreplicating entities in the form of linear nucleic acid or transcript or circular plasmid or vector.

Direct administration as used herein refers to the direct administration of nucleic acid constructs which encode embodiments (e.g., SEQ ID NO:3, modulator compound molecule, antisense molecule, antibody molecule) of the present invention or fragments thereof; and the direct administration of embodiments of the present invention or fragments thereof, and the *in vivo* introduction of molecules of the present invention preferably via an effective eukaryotic expression vector in a suitable pharmaceutical carrier. Polynucleotides and therapeutic molecules of the present invention may also be delivered in the form of nucleic acid transcripts.

## Potassium Channels

Potassium channels are the most evolutionarily ancient, ubiquitous and diverse of ion channels. Present in all eukaryotes, potassium channels set the resting membrane potential and control electrical excitability in diverse cell types. Potassium channels perform functions in cells as specific as defining the interspike intervals of endogenously beating cells. In the nervous system, potassium channels are key regulators of signaling by virtue of their role in governing action potential shape and pattern which are ultimately critical to perception, learning and behavior. Wei, A., et al., Neuropharmacology, Vol. 35(7):805 (1996). The importance of the bioactive integrity of normal neurophysiology is readily perceived in clinical medicine today a result of the striking demonstration of the consequences of neuronal dysfunction.

Apoptosis of mouse neocortical neurons, for example, induced by serum deprivation or by staurosporine has been associated with an early enhancement of delayed rectifier ($I_K$) current and loss of total intracellular K+. Attenuating outward K+ current with tetraethylammonium (TEA) or elevated extracellular K+, but not blockers of $Ca^{2+}$, $Cl^-$, or other K+ channels, reduced apoptosis, even if associated increases in intracellular $Ca^{2+}$ concentration were prevented. Furthermore, exposure to the K+ ionophore valinomycin or the K+-channel opener cromakalim induced apoptosis. Enhanced K+ efflux may mediate certain forms of neuronal apoptosis. Shan Ping Yu, et al., Mediation of Neuronal Apoptosis by Enhancement of Outward Potassium Current, Science, 278:114 (1997).

In support of the hypothesis that increased K+ efflux might be a primary step leading to apoptosis, application of the selective K+ ionophore valinomycin triggers apoptosis in, for instance, thymocytes, lymphocytes, and tumor cells. Exposure to 20 nM valinomycin for 24 to 48 hours induced typical neuronal apoptosis in cortical cultures, characterized by chromatin condensation, cell body shrinkage, internucleosomal DNA fragmentation, and sensitivity to cyclohemimide. Furthermore, 24 to 48 hours of exposure to the K+ channel opener cromakalim, which activates $K_{ATP}$ channels as well as $I_K$-like currents in mammalian central neurons also induced typical neuronal apoptosis. Evidence suggests that a long-lasting enhancement of outward K+ current is a key mediator of the forms of cortical neuronal apoptosis. Shan Ping Yu*, et al.* suggest that interventions directed at blocking excessive K+ efflux, in particular by the noninactivating delayed rectifier K+ channel, be explored as a strategy for attenuating neuronal apoptosis in disease states. Shan Ping Yu, et al., Mediation of Neuronal Apoptosis by Enhancement of Outward Potassium Current, Science, 278:114 (1997).

Recent molecular cloning and functional expression experiments have revealed genes that underlie functional diversity similar to this can be grouped into several conserved classes of multigene families. In particular, KQT channels have core regions which distinguish them from voltage-gated channels. Wei, A., et al., Neuropharmacology, Vol. 35(7): 805 (1996).

The KQT class of voltage gated potassium channels are evolutionarily distinct from the other voltage gated potassium (Kv) channels. Barhanin, J., et al., Nature, 384, 78-80 (1996). A most notable difference is the absence of a tetramerization domain in the N-terminus that mediates sub-family specific association. Wei, A., et al., Neuropharmacology, Vol. 35(7):805 (1996). In the pore and sixth transmembrane regions, a unique pattern of conserved residues distinguishes KQT channels from other Kv channels, for example, the ADAL motif preceding the pore, the KxPQTW motif in the pore and SFFALPAGILGSG motif in S6.

The first member of the KQT family of genes is KvLQT1. Mutations of this gene have been implicated in one form of long QT syndrome, a hereditary disease which predisposes carriers to cardiac arrhythmias. Wang, Q., et al., Nature Genet., 12:17 (1996). Recently, it was shown by two independent groups that KvLQT1 and and another single-transmembrane protein called ISK, associate in the heart to form the slowly activating potassium current, $I_{KS}$ that is responsible for repolarization of action potentials. Barhanin, J., et al., Nature, 384, 78-80 (1996); Sanguinetti, M. C., et al., Nature, 384:80 (1996). Barhanin *et al.* and Sanguinetti *et al.* recently identified the molecular components of the slow delayed-rectifer K+ channels, a crucial step in the search for new anti-arrhythmic drugs. Nature, 384, 78-80 (1996) ; Nature, 384:80 (1996); Attali, B.,Nature, 384:25 (1996). In COS cells, $K_v$LQT1 expression induced a voltage-dependent, rapidly activating and slowly deactivating outward K+ current. $K_v$LQT1 messenger RNA is abundant in mouse heart, kidney and salivary glands, but are almost undetectable in brain, skeletal muscle and liver. The molecular identification of the $I_{KS}$ channel should help with the design of new anti-arrhythmic drugs. Barhanin, J., et al., Nature, 384:25 (1996). Northern blot analysis of KvLQT1 shows expression in human heart, placenta, lung, kidney and pancreas; no signal is detected in brain. Sanguinetti, M. C., et al., Nature, 384:80 (1996).

Recently, Yokoyoma, M., *et al* reported a transcript, which encodes a 393 amino acid poypeptide they called HNSPC (human neuron specific potassium channel), from a neuroblastoma cell-line (CHP 134). DNA Research, 3:311 (1996). Human neuroblastoma is a malignant neoplasm that arises from neuroblasts or undifferentiated nerve cells of sympathetic nerve origin in the adrenal medulla or sympathetic ganglion. The authors teach the identification of a gene that is purportedly expressed specifically in the nervous system. A full-length 1425 base cDNA sequence corresponding to the Yokoyama transcript is listed herein (SEQ ID NO:4) and shown to contain an open reading frame of 393 amino acids spanning positions 178-1356 of SEQ ID NO:4. HNSPC has been described as having all the structural characteristics of a voltage-gated potassium channel expressed specifically in the nervous system. Three transcripts have been demonstrated in Northern blot analysis using a full-length probe. A 1.5-kb transcript is abundant in neuroblastoma cell lines. In human brain tissue, a 9.5-kb transcript appeared as a major component and 1.5-kb and 3.8-kb transcripts appeared as minor components. In fetal brain, both 1.5-kb and 9.5-kb transcripts, but not a 3.8-kb transcript, are abundant. Yokoyama, M., et. al., Identification and Cloning of Neuroblastoma-Specific and Nerve Tissue-Specific Genes through Compiled Express Profiles, DNA Research, 3:311 (1996).

**<u>The novel human brain-derived potassium channel biomolecule is developmentally regulated and tissue-specific to differentiated neurons</u>**

Herein disclosed is SEQ ID NO: 1 which is a 3029 base cDNA nucleic acid sequence which encodes a novel human brain-derived potassium channel polypeptide which is tissue-specific to differentiated neurons as further described *vide infra.* SEQ ID NO:2 is the 2565 base translated structural region, ATG to TGA, of the cDNA nucleic acid sequence which encodes the novel human brain-derived tissue-specific potassium channel polypeptide. SEQ ID NO:3 which is the 854 amino acid residue sequence of the human brain-derived potassium channel polypeptide tissue-specific to differentiated neurons as described herein.

For reference and comparison: SEQ ID NO:4 is the 1425 base cDNA sequence pertaining to the HNSPC potassium channel. Genbank accession No. D82346. SEQ ID NO:5 is the 393 amino acid residue sequence of the recently described HNSPC potassium channel. Genbank accession No. D82346; Yokoyama, M., et. al., Identification and Cloning of Neuroblastoma-Specific and Nerve Tissue-Specific Genes through Compiled Express Profiles, DNA Research, 3:311 (1996). SEQ ID NO:6 which is the 581 amino acid residue sequence of the recently described human KvLQT1 potassium channel. Genbank accession Nos. U40990, U71077; Barhanin, J., et al., Nature, 384, 78-80 (1996) ; Sanguinetti, M. C., et al., Nature, 384:80 (1996).

The 854 residue developmentally regulated and tissue-specific human brain-derived potassium channel polypeptide described herein (SEQ ID NO:3) shares 37% total homology at the amino acid level with the 581 aa KvLQT1 (SEQ ID NO:6) and 44% total homology at the amino acid level to the393 aa HNSPC (SEQ ID NO:5) (95% homology in its alignable sequence) (comparison shown in FIG.7). The novel brain-derived potassium channel (SEQ ID NO:3) poypeptide has an additional 461 amino acids in its C-terminal portion when compared to HNSPC (SEQ ID NO:5).

HNSPC has previously been referred to as "human neuron specific potassium channel", based on recognition in Northern blots of a predominant 1.5 Kb transcript in several undifferentiated neuroblastoma cell-lines as well as a 9.5-10 Kb band in brain tissue. However, these bands were detected by Yokoyama *et al* using a HNSPC-coding region probe which shares 95% regional similarity to the novel cDNA described herein (SEQ ID NO:1). In fact, the 9.5-10 Kb band observed by Yokoyama *et al* in human brain corresponds to the novel cDNA (SEQ ID NO:1) described herein and not HNSPC as further described *infra.*

**The novel brain-derived posassium channel is expressed at a high level upon neuron differentiation**. The differentiation of nervous cells is a crucial process in the normal development of nervous tissue as well as for the regulation of cell plasticity during growth and regeneration in pathological conditions. The neuroblastoma line, IMR-32, is used extensively as a model system for studying neuronal differentiation. Sher, et al., J. Neurochemistry, 50:1708 (1988).

A nucleic acid probe (herein referred to as probe no. 1, further described *infra*) corresponding to positions 1257 to 1461 of SEQ ID NO:2 spans the C-terminal tail structural region of the novel brain-derived potassium channel cDNA. The nucleic acid probe referred to as probe no.2 herein corresponds to base positions 989 to 1109 of SEQ ID NO:2; which spans the relative coding region for a 40 amino acid region that is common to both a central region of the brain-derived potassium channel described herein, and a region of HNSPC pertaining to the C-terminus of the peptide.

Nucleic acid probe 1 is demonstrated herein to identify a very faint 9.5-10 Kb band in *uninduced* IMR-32 cells; while demonstrating a dramatically upregulated (~$20^{+}$fold) expression in differentiated IMR-32 cells (FIG.11). Thus, there is strong association between expression of the novel brain-derived potassium channel and induction of neuronal differentiation, which highlights the importance of the novel biomolecule in neurological function.

In multiple tissue blots using nucleic acid probe 1, a predominant 9.5-10 Kb transcript is seen exclusively in human brain (FIG.9). This transcript is seen in both adult and fetal brain (FIG. 11) and is present with similar intensity in the cerebellum, cerebral cortex, medulla, occipital lobe, frontal lobe, temporal lobe and putamen (FIG.10). FIG.9 displays Northern blot analyses of multiple tissues, (Panel A) using nucleic acid probe no. 1 which is specific to the human brain-derived potassium channel polypeptide coding region described herein (SEQ ID NO:2); (Panel B) and (Panel C) using nucleic acid probe no.2 which is common to HNSPC cDNA (SEQ ID NO:4) and the human brain-derived potassium channel polypeptide coding region described herein (SEQ ID NO:2). FIG.10 displays Northern blot analyses of distinct regions of human brain, (Panel A) using nucleic acid probe no. which is specific to the human brain-derived potassium channel polypeptide coding region described herein (SEQ ID NO:2); (Panel B) using nucleic acid probe no. 2 which is common to HNSPC cDNA (SEQ ID NO:4) and the human brain-derived potassium channel polypeptide coding region described herein (SEQ ID NO:2). FIG.11 displays Northern blot analyses of human adult brain and human fetal brain, imr-32 and astrocytes, (Panel A) using nucleic acid probe no. 1 which is specific to the human brain-derived potassium channel polypeptide coding region described herein (SEQ ID NO:2); (Panel B) using nucleic acid probe no. 2 which is common to HNSPC cDNA (SEQ ID NO:4) and the human brain-derived potassium channel polypeptide coding region described herein (SEQ ID NO:2).

The brain-derived potassium channel poypeptide described herein (SEQ ID NO:3) is developmentally regulated and expressed tissue-specifically in human brain and in neuroblastoma cell-lines that are induced to neuronal differentiation. This is a significant fact that functionally distinguishes and contrasts the novel biomolecule from HNSPC which is expressed at a very low level in adult brain and is not regulated upon neuronal differentiation of neuroblastoma cell-lines.

**The brain-derived posassium channel is expressed in neuronal cells and not in glial cells of human brain.** Human brain is made up of excitable neurons and non-excitable glial cells. The glial cells are 10 times more abundant than neuronal cells. To evaluate whether the novel potassium channel transcript (SEQ ID NO:1) in human brain is expressed in neuronal or non-neuronal cells, a Northern blot analysis of the non-neuronal glial cells cultured from primary human fetal brain was performed. The transcript pertaining to the brain-derived potassium channel described herein was not detected in mRNA from glial cells, confirming its specific expression in neuronal cells of the brain (FIG. 11). The transcript (SEQ ID NO:1) is present in both adult and fetal brain (FIG.11).

**HNSPC is predominantly expressed in neuroblastoma cell-lines and not in the human brain.** In contrast, probe 2 is herein demonstrated to detect a predominant 1.5 Kb band in both *native and differentiated* IMR-32 cells (representing HNSPC) and an additional 9.5-10Kb band in differentiated IMR-32 cells (representing the transcript pertaining to the novel brain-derived potassium channel described herein; FIG. 11). Thus, HNSPC is predominantly expressed in neuroblastoma cell-lines but is not the predominant transcript in the human brain.

Probe 2 picks up a predominant 9.5-10 Kb band in several regions of the brain (FIG.9 and FIG. 10). These results are very similar to those found with probe 1 (see above), suggesting very high expression the potassium channel polypeptide described herein (SEQ ID NO:3) in human brain. A 1.5 Kb transcript is seen in testes, using probe 2 (FIG. 9), representing HNSPC as described by Yokoyama *et al.*

The HNSPC polypeptide (SEQ ID NO:5) previously described by Yokoyama *et al* is recognized herein to fall under the KQT family. The cDNA described herein represents a novel potassium channel member of the KQT family. In brain tumors, gene regulation is altered. Brain tumors are hypothesized to produce more HNSPC (SEQ ID NO:5) rather than the longer tissue specific potassium channel polypeptide described herein (SEQ ID NO:3), leading to increased cellular proliferation. HNSPC (SEQ ID NO:5) is abundant in neuronal cell lines and in fetal brain, but adult brain has little to no HNSPC, while an abundance of the longer tissue specific potassium channel polypeptide described herein (SEQ ID NO:3). Accordingly, one would predict that the novel SEQ ID NO: 1 cDNA pertaining to the novel potassium channel will be down-regulated in brain tumors, with concomittant increase in the SEQ ID NO:4 HNSPC cDNA.

**Functional expression of the brain-derived potassium channel subunit described herein yields currents which are suppressed by HNSPC**

The human brain-derived potassium channel cDNA coding region (SEQ ID N0:2) when transfected into mammalian cells result in robust voltage-gated outward currents (FIG.12A, B) that are potassium selective (FIG.12C) and can be blocked potently by externally applied TEA (tetraethyl ammonium) (FIG.12D). Transfection of mammalian cells with HNSPC cDNA, SEQ ID NO: 4 (a short splice-variant of SEQ ID NO: 1), which yields the SEQ ID NO:5 potassium channel subunit, produces no functional currents. An EGFP (Enhanced Green Fluorescent Protein)-fusion construct of SEQ ID NO:2 is described herein to detect green cells as a marker for expression of the channel subunit. This construct produced functional channels with biophysical and pharmacological properties very similar to the "un-fused" channel subunit (SEQ ID NO:3) *(see,* FIG. 13A). Furthermore, co-expression of SEQ ID NO:3 along with EGFP-HNSPC results in suppression of potassium currents produced by the resulting long splice variant subunit: SEQ ID NO:3 *(see,* Fig. 13 A-D); however, no significant alteration is demonstrated in the kinetics of channel gating (FIG.14). See Example XIV.

In the context of neuronal development, it is demonstrated that immature developing brain tissue expresses both the short and the long splice variants of SEQ ID NO:1, e.g., SEQ ID NO:3 and SEQ ID NO:5, while the mature adult brain predominantly expresses the long splice variant SEQ ID NO:3 (*see,* FIG.11). The fact that the short version subunit, HNSPC (SEQ ID NO:5), suppresses the function of the long variant SEQ ID NO:3 implies altered action potential firing frequencies is critical for the developing brain.

**Expression of SEQ ID NO:4 in human brain tumors: use in diagnosis**

A Northern blot analysis of human brain tumor mRNA has revealed that the short transcript HNSPC (SEQ ID NO:4), which is very weakly expressed in normal adult brain, is upregulated in adult brain tumors (FIG.15). This result, in view of the observed association of the short splice variant (SEQ ID NO:4) of the brain-derived potassium channel subunit with proliferating brain cells such as fetal brain and undifferentiated neuroblastoma cell lines (FIG.11), leads to the expectation that detectable levels of the transcript are indicative of brain tumor tissue. Two forms of adult brain tumor RNA, an astracytoma and a glioma, are positive for the short transcript, SEQ ID N0:4. Control RNA from adjacent non-tumorgenic cells from the same brain were negative for SEQ ID NO:4 transcripts. Tumors of non-brain origin that had metastatized to the brain were also negative for SEQ ID NO:4 transcripts. Accordingly, detection of the short transcript of SEQ ID NO:4 by PCR or by antibodies, in cerebrospinal fluid of patients with brain tumors may be used as a diagnostic tool for monitoring the progress of the disease during treatment or otherwise for diagnostic purposes. For example, generation of a detectable 107bp PCR band that is specific for the short transcript is accomplished using the forward Primer: 5' AAC CTC TCG CGC ACA GAC CTG CA 3' (SEQ ID NO:9) corresponding to positions 1225-1247 of SEQ ID NO:4 as well as a reverse Primer: 5' AAC AGT TGC TTG GTG GCA GGA GCC 3' (SEQ ID NO: 1 0) corresponding to positions 1308-1331 of SEQ ID NO:4. See Example XV.

**Role of SEQ ID NO:3 potassium channel subunits in β-amyloid-induced neuronal death (molecular identification of genes encoding the $K^+$ channel in SN56 cells)**

Basal forebrain cholinergic neurons (Colom et al., J Neurochem., 70: 1925 (1998)) as well as cortical neurons (Yu, S. et al., Neurobiol Dis., 5(2):81 (1998) which are severely depleted early in the course of Alzheimer's Disease (AD) express a particular profile of $K^+$ currents that are voltage-gated and sensitive to TEA. The $K^+$ currents regulate vital cell functions; as a corollary, their alteration is expected to lead to cell injury and subsequent death. Beta-amyloid peptide (Aβ) has been shown to be a major player in neuronal toxicity in patients with AD. A cholinergic septal cell line, SN56, expresses a TEA-sensitive potassium current. Aβ has been demonstrated to enhance the potassium currents in these cells. Attenuation of this current via TEA is demonstrated to block Aβ-mediated toxicity neurons. Colom et al., J Neurochem. 70:1925 (1998). A non-cholinergic septal line (SN 48), does not appear to have significant potassium currents and is resistant to Aβ-induced cell death. The physiological characteristics and pharmacological profiles of the $K^+$ currents in SN56 are reminiscent of the properties of two sub-families of cloned and characterized gene-products: the *shaw* subfamily, *Kv3.1* and *Kv3.2* (Chandy and Gutman, Handbook of Receptors and Channels, (1995)) of voltage-gated channels and the more recently described *KvQT* sub-family, *KvQT2* and *KvQT3* (Singh, et al., Nature Genetics (1998); Charlier, et al., Nature Genetics (1998)).

To determine if one or all of these channels encoded the $K^+$ channel in SN56 cells, Northern blot analyses were performed. Total RNA from undifferentiated and differentiated SN48 and SN56 cells was isolated according to standard protocols. Twenty micrograms of each RNA sample, along with 20 μg of rat and mouse brain RNA (as positive controls), was run on a 1.0% agarose gel, transferred to nitrocellulose, and hybridized under high stringency conditions to a random primed $^{32}P$ labeled probe designed to pick up the shaw-subfamily of genes (*Kv3.1-Kv3.4*) or SEQ ID NO: 2 or SEQ ID NO:4 (the long and short alternate splice variants of the *KvQT2* gene). The autoradiogram was exposed for a sufficient amount of time so that even less-abundant transcripts1, if present, would be clearly visible. Strong 3.8 Kb and 1.5 Kb bands are detected which correspond to the long and short splice variants (SEQ ID NO:2 ans SEQ ID NO:4), respectively, in both undifferentiated and differentiated SN56 cells. This signal is very weak (or absent) in SN48 cells. *See,* FIG.16.

## Mutations in the KvQT family of genes cause benign famililal neonatal epilepsy

Epileptic disorders affect about 20-40 million people worldwide, and 40% of these are idiopathic generalized epilepsies of no known etiology (In Idiopathic Generalized Epilepsies. Eds Malafosase A. et. al., John Libbey, London, 1994). Most of the idopathic epliepsies are inherited in an autosomal dominant fashion. Two loci on chromosome 20q13.3 and chromosome 8q24 respectively, have been mapped by linkage analysis to benign, familial, neonatal convulsions. By positional cloning of these loci, two groups have identified hKvQT2 (SEQ ID NO:3 described herein) and KvQT3 (e.g., SEQ ID NO:7 (FIG.17)) coding regions which are mutated in epilepsy patients. Singh N. A. et al., Nature Genetics, 18, 25 (1998); Biervert C., et al., Science, 279: 403 (1998); Charlier C., et al., Nature Genetics, 18: 53 (1998). Several point mutations or missense/frameshift mutations in the pore region in post-S6 and C-terminii are found in epilepsy families. These mutations lead to loss of function of the corresponding K+ channel. One such truncation in the C-tail, which mimics the biological and/or pharmacological activity of **HNSPC** (SEQ ID NO:5), has been shown to be non-functional and to cause suppression of wild-type hKvQT2 (SEQ ID N0:3) currents, when co-expressed in Xenopus oocytes. Biervert C., et al., Science, 279: 403 (1998). At least eleven murine splice variants of mKQT2 (murine homolog of SEQ ID NO:3) have been described, all of which are in the C-tail. None of these C-terminal splice variants produced functional channels in Xenopus oocytes. Nakamura, M., et al., Receptors and Channels 5:255 (1998).

Since potassium channels are functional tetramers, a mutation in one allele is sufficient to cause a dominant negative phenotype in one subunit that could be fatal to the function of the channel tetramer. Thus, pathological loss of function of the channel is presumed to cause neuronal hyperexcitability arising from impaired repolarization in the epilepsy syndrome. Human KvQT2-agonists, agonists of the biological and/or pharmacological activity of SEQ ID NO: 3, for example, which function as channel openers, are expected to be useful in the treatment of seizures and epilepsies. Moreover, delivery of a normal subunit *via* gene therapy to a subject in need of a normal subunit, for example to a subject who has inherited an aberrant version, is expected to be of significant therapeutic value toward treatment of disorders mediated by abnormal levels or versions of SEQ ID NO:3.

## Molecular correlate of M-currents in the brain

M-current is a non-inactivating voltage-gated potassium current found in many neuronal cell types. Reviewed by Marrion N. V., Control of M-Current, Ann. Rev. Physiol., 59: 483 (1997). In each brain cell, the M-current is dominant in controlling membrane excitability by being the only sustained current in the range of action potential initiation. The M-current can be modulated by a large array of receptor types, resulting in either enhancement or suppression of the current. In fact, historically, the term M-current was coined based on the observed suppression of a time- and voltage-dependent potassium current by muscaranic receptor stimulation. Brown D. A. and Adams, P. R., Nature, 283: 673 (1980). Suppression of the M-current results in membrane depolarization, making the neurons more likely to fire action potentials. The current is also synaptically regulated, with synaptic suppression of M-current underlying the slow excitatory postsynaptic potential (EPSP) in sympathetic and hippocampal CA3 neurons. Therefore it is apparent that the presence of the M-current exerts a powerful effect in regulating neuronal excitability. Modulators of M-currents have been shown to enhance neurotranmitter release and are therefore attractive as cognition enhancers.

The biophysical and pharmacological properties of the hKvQT2 (SEQ ID NO:3) channel expressed in mammalian cells closely matches the properties of the M-current in neurons, including activation and inactivation kinetics, tail currents, and blocking by barium. *See* FIG.12. In fact a heteromultimer of the hKvQT2 potassium channel subunit (as described herein, e.g., SEQ ID NO:3) and another homolog of this family, KvQT3 (e.g., SEQ ID NO:7), has been implicated as a channel which carries the M-current. Neuroscience Meeting, Wang H.S., et al. Society for Neuroscience, 24: Abs # 792.1 (1998). *See* FIG.17 and FIG.18. Upon the co-expression of hKQT2 and hKQT3 in oocytes, resulting current mimicked all the properties of the native M-currents, including block by TEA. Furthermore, KvQT3 (SEQ ID NO: 7) channels when expressed alone were shown to produce very low potassium currents in *Xenopus* oocytes. Yang, W.P., et al., J. Biol.Chem., 31:19419 (1998). However, when coinjected with hKvQT2 mRNA (SEQ ID NO: 1), very robust currents with novel properties were produced, indicative of heteromultimerization of the two channel types.

The observed involvement of the SEQ ID NO:3 channel subunit integral to the formation of the channel that codes for the M-current has substantial implication in the screening for the identification of as well as design of small molecules that modulate M-current activity and thereby modulate neuronal function. For use in drug discovery screens, cell-lines are contemplated that stably express hKvQT2 (SEQ ID NO:3) alone or co-express SEQ ID NO:3 and any of its related homologs including, but not limited to, KvQT1 and/or KvQT3 (SEQ ID NO:7). Neuronal functions include, for example, cognition enhancement, and attention deficit disorders, and the like.

**Immunohistochemistry of brain sections using antibodies to hKvQT2 (SEQ ID NO:3) and KvQT3 (SEQ ID NO:7)**

Antibodies were raised to hKvQT2 (SEQ ID NO: 3) and KvQT3 (SEQ ID NO:7) by using peptides corresponding to amino acid residue positions 644-658 of SEQ ID NO: 3 as well as residues 639-653 of SEQ ID NO: 7. These peptides were synthesized, conjugated to KLH and used for immunizing rabbits. Antisera obtained 7-8 weeks following immunization gave very high titers of antibodies to the peptides in an ELISA (> 1: 100, 000 dilution). The antisera were used to perform immunohistochemistry on sample rat brain tissue as well as human brain tissue. Paraffin embedded sections were incubated with 1:3000 dilution of the primary hKvQT2 or hKvQT3 antiserum, followed by 1:200 dilution of HRP-conjugated goat anti-rabbit antiserum Staining was visualized using DAB substrate (Paragon Biotech Inc., Baltimore, MD). Positive staining for both hKvQT2 and hKvQT3 antibodies were seen in purkinjee cells in the cerebellar cortex, ependymal cells of the choroid plexus, large neurons in basal forebrain. Weaker staining was seen in neurons from several other regions of the brain including cortex and hippocampus. Pre-immune sera shows no detectable staining. The overlapping profile of expression of both hKvQT2 and hKvQT3 was striking and suggestive of co-localization.

Other names recently used for the brain-derived potassium channel subunit KvQT2 (SEQ ID NO:3) described herein include KQT2 (Wei et al., 1996, Neuropharmacology, 35:805-829) and KCNQ2 (Biervert et al., 1998, Science, 279:403-406).

The present invention relates to nucleic acid (SEQ ID NO: 1 and SEQ ID NO:2) and amino acid sequences (SEQ ID NO:3) of the novel human brain-derived potassium channel and to the use of these sequences to identify compounds that modulate the activity of potassium channels and human neurophysiology.

The invention further relates to the use of the nucleic acid sequences described herein in expression systems as assays for agonists or antagonists of the potassium channel biomolecule. The invention also relates to the diagnosis, study, prevention, and treatment of disease related to the human brain-derived potassium channel and/or diseases mediated by dysfunctional neurons.

Polynucleotide sequences which encode the human neuron potassium channel (SEQ ID NO:3) may be used in accordance with the present invention which comprise deletions, insertions and/or substitutions of the SEQ ID NO:2 nucleic acid sequence. A purified polynucleotide comprising a nucleic acid sequence encoding the polypeptide having the sequence of SEQ ID NO:3 is a particularly preferred embodiment of the present invention.

Nucleic acid sequences which encode the amino acid sequence of the novel potassium channel neuron-specific molecule described herein are of an exponential sum due to the potential substitution of degenerate codons (different codons which encode the same amino acid). The oligonucleotide sequence selected for heterologous expression is therefore preferably tailored to meet the most common characteristic tRNA codon recognition of the particular host expression system used as well known by those skilled in the art.

The nucleotide sequences of the present invention may also be engineered in order to alter a coding sequence for a variety of reasons, including but not limited to, alterations which modify the cloning, processing and/or expression of the gene product. For example, mutations may be introduced using techniques which are well known in the art, eg, site-directed mutagenesis to insert new restriction sites, to alter glycosylation patterns, to change codon preference, etc.

The present invention relates, in part, to the inclusion of the polynucleotide encoding the novel potassium channel molecule in an expression vector which can be used to transform host cells or non human organisms. Such transgenic hosts are useful for the production of the novel neurophysiological molecule and variations thereof described herein.

The nucleic acid sequence also provides for the design of antisense molecules useful in downregulating, diminishing, or eliminating expression of the genomic nucleotide sequence in cells including but not limited to neurons, and tumor or cancer cells.

The human potassium channel biomolecule of the present invention can also be used in screening assays to identify blockers, antagonists or inhibitors which bind, emulate substrate, or otherwise inactivate or compete with the biomolecule. The novel potassium channel can also be used in screening assays to identify agonists which activate the potassium channel or otherwise induce the production of or prolong the lifespan of the biomolecule *in vivo* or *in vitro.*

The invention also relates to pharmaceutical compounds and compositions comprising the human potassium channel polypeptide molecule substantially as depicted in SEQ ID NO:3, or fragments thereof, antisense molecules capable of disrupting expression of the naturally occurring gene, and agonists, antibodies, antagonists or inhibitors of the native biomolecule. These compositions are useful for the prevention and/or treatment of conditions associated with dysfunctional neurons and neurophysiological disorders.

Particularly preferred embodiments of the invention are directed to methods for screening for compounds which modulate neurophysiology, which interfere with or inhibit the biological activity of a potassium channel.

**Pharmacological Significance**

As discussed *supra,* Northern blot analysis reveals that the novel biomolecule is almost exclusively restricted to neuronal cells (and not glial cells) of human brain (FIG. 9-11). The novel transcript is in very low abundance in the neuroblastoma cell line IMR-32, however upon differentiation of these cells to neurons, there is significant up-regulation of the transcript (FIG. 11). The novel brain-derived developmentally regulated potassium channel described herein could play crucial roles in modulating synaptic transmission and electrical excitability in the brain. Modulation of function of the novel biomolecules described herein, e.g., SEQ ID NO:2 and SEQ ID NO:3 and variations thereof, could have profound implications in cognitive (e.g., learning and memory) behavioral (anxiety, depression), psychiatric (e.g. bipolar disorders, schizophrenia) neurodegenerative (e.g., Alzheimer's disease, Parkinson's disease) and developmental disorders (e.g., mental retardation) as well as in asthma, migraine, epilepsy and stroke and brain tumors. Of special interest would be its role in apoptotic mechanisms of neuronal cell death in Alzheimer's disease.

The neuropharmacology of a developmentally regulated human brain-derived potassium channel that is expressed specifically in neurons has significant potential for the diagnosis, study, prevention, and treatment of pathophysiological disorders mediated by dysfunctional neurons. The novel nucleic acid as well as peptide biomolecules described herein have particular value especially in the identification and development of comounds that modulate the biological activity of the potassium channel *in vivo.*

The present invention relates to nucleic acid (SEQ ID NO: 1 and SEQ ID NO:2) and amino acid sequences (SEQ ID NO:3) of a novel human brain-derived potassium channel and variations thereof and to the use of these sequences to identify compounds that modulate the activity of specialized cells, especially human neurons, as described *infra.*

Compounds (e.g., small-molecules, peptides, analogs, mimetics) that modulate the biological activity of the brain-derived potassium channel described herein are contemplated for use in the treatment of a variey of disease conditions including shizophrenia, anxiety, depression, brain tumors, Huntington's disease, Alzheimers, Parkinson's, Lou Gehrig's, stroke, epilepsy, memory degeneration, neurodegeneration, multiple sclerosis, psychosis, urinary incontinence, diabetes, asthma, premature labour, hypertension, cardiac ischaemia and arrhythmias, migraine headaches, autoimmune diseases, cancer, and graft rejections.

The novel human brain-derived potassium channel can be used to raise diagnostic antibodies as discussed *infra* to detect abnormal levels of the biomoleule *in vivo.* Therefore, in accordance with yet a further aspect of the present invention, there are provided antibodies against the potassium channel poypeptide which may used as part of various diagnostic assays for detecting physiological, especially neurophysiological, disorders.

The present invention relates to a screening assay for identifying molecules which have a modulating effect, e.g. compounds including but not limited to agonists and antagonists, on the biological activity of the potassium channel which comprises the brain-derived potassium channel polypeptide of the present invention. Such an assay comprises the steps of providing an expression system that produces a functional K+ channel expression product encoded by a nucleic acid sequence of the present invention, contacting the expression system or the product of the expression system with one or more molecules to determine its modulating effect on the bioactivity of the product and selecting from the molecules a candidate capable of modulating K+ channel expression.

Particularly preferred embodiments of the present invention are host cells transformed with a purified polynucleotide comprising a nucleic acid sequence encoding the polypeptide having the sequence substantially as depicted in SEQ ID NO:3 or a biologically active fragment thereof. Cells of this type or preparations made from them may be used to screen for pharmacologically active modulators of the novel potassium channel activity using methods which are well known in the art. *See, e.g.,* U.S. Patent No. 5,397,702, *Assay For and Treatment of Autoimmune Diseases,* issued March 14, 1995; U.S. Patent No. 5,637,470, *Screening array using cells expressing recombinant alpha and beta subunits of the mammalian large-conductance (maxi-K) potassium channel,* issued June 10, 1997; U.S. Patent No. 5,607,843, *Nucleic Acid Sequence Encoding Apamin Binding Protein,* issued March 4, 1997; Published PCT international application WO9603415A1, *HumanPotassium Channel;* and U.S. Patent No. 5,602,169, *3-substituted oxindole derivatives as potassium channel modulators,* issued Feb. 11, 1997.

Such modulators are potentially useful in treating disease which is manifested by dysfunctional specialized cells, neurons in particular, including but not limited to shizophrenia, anxiety, depression, brain tumors, Huntington's disease, Alzheimers, Parkinson's, Lou Gehrig's, stroke, epilepsy, memory degeneration, neurodegeneration, multiple sclerosis, psychosis, amyotrophic lateral sclerosis, retinitis pigmentosa, cerebellar degeneration, urinary incontinence, diabetes, asthma, premature labour, hypertension, cardiac ischaemia and arrhythmias, migraine headaches, autoimmune diseases, cancer, graft rejections, acute and chronic inflammation, allergies, proliferative disorders, anemias, neurodegenerative diseases with immunological components, as well as autoimmune diseases including rheumatoid arthritis, type-1 diabetes mellitus, myasthenia gravis, systemic lupus erythematosus, Sjogren's syndrome, mixed connective tissue disease, and experimental allergic encephalomyelitis (EAE).

Potential diagnostic and therapeutic applications are readily apparent for modulators of the human potassium channel biomolecule described herein. Pathophysiological disorder areas common to disease particularly in need of therapeutic intervention include but are not limited to neurophysiological disorders manifested by dysfunctional neurons.

**Generally Acceptable Vectors**

In accordance with the present invention, polynucleotide sequences which encode the novel neuron potassium channel polypeptide, fragments of the polypeptide, fusion proteins, or functional equivalents thereof may be used in recombinant DNA molecules that direct the expression of the neuron biomolecule in appropriate host cells. Due to the inherent degeneracy of the genetic code, other DNA sequences which encode substantially the same or a functionally equivalent amino acid sequence, may be used to clone and express the novel neuron biomolecule. As will be understood by those of skill in the art, it may be advantageous to produce novel potassium channel encoding nucleotide sequences possessing non-naturally occurring codons.

Specific initiation signals may also be required for efficient translation of a potassium channel nucleic acid sequence. These signals include the ATG initiation codon and adjacent sequences. In cases where the novel neuronal nucleic acid sequence, e.g., SEQ ID NO:2, its initiation codon and upstream sequences are inserted into the appropriate expression vector, no additional translational control signals may be needed. However, in cases where only coding sequence, or a portion thereof, is inserted, exogenous transcriptional control signals including the ATG initiation codon must be provided. Furthermore, the initiation codon must be in the correct reading frame to ensure transcription of the entire insert. Exogenous transcriptional elements and initiation codons can be of various origins, both natural and synthetic.

Nucleic acid sequences, e.g., SEQ ID NO:2, may be recombinantly expressed to produce a biologically active neuronal potassium channel biomolecule by molecular cloning into an expression vector containing a suitable promoter and other appropriate transcription regulatory elements, and transferred into prokaryotic or eukaryotic host cells to produce the novel polypeptide. Techniques for such manipulations are, for instance, fully described in Sambrook, J., et al., Molecular Cloning Second Edition, Cold Spring Harbor Press (1990), and are well known in the art.

Expression vectors are described herein as DNA sequences for the transcription of cloned copies of genes and the translation of their mRNAs in an appropriate host cell. Such vectors can be used to express nucleic acid sequences in a variety of hosts such as bacteria, bluegreen algae, plant cells, insect cells, fungal cells, human, and animal cells. Specifically designed vectors allow the shuttling of DNA between hosts such as bacteria-yeast, or bacteria-animal cells, or bacteria-fungal cells, or bacteria-invertebrate cells.

A variety of mammalian expression vectors may be used to express the recombinant neuron-specific molecule and variations thereof disclosed herein in mammalian cells. Commercially available mammalian expression vectors which are suitable for recombinant expression, include but are not limited to, pcDNA3 (Invitrogen), pMC1neo (Stratagene), pXT1 (Stratagene), pSG5 (Stratagene), EBO-pSV2-neo (ATCC 37593) pBPV-1(8-2) (ATCC 37110), pdBPV-MMTneo (342-12) (ATCC 37224), pRSVgpt (ATCC 37199), pRSVneo (ATCC 37198), pSV2-dhfr (ATCC 37146), pUCTag (ATCC 37460), and IZD35 (ATCC 37565), pLXIN and pSIR (CLONTECH), pIRES-EGFP (CLONTECH). INVITROGEN corporation provides a wide variety of commercially available mammalian expression vector/systems which can be effectively used with the present invention. INVITROGEN, Carlsbad, CA. *See, also,* PHARMINGEN products, vectors and systems, San Diego, CA.

Baculoviral expression systems may also be used with the present invention to produce high yields of biologically active protein. Vectors such as the CLONETECH, BacPak™ Baculovirus expression system and protocols are preferred which are commercially available. CLONTECH, Palo Alto, CA. Miller, L.K., et al., Curr. Op. Genet. Dev. 3:97 (1993); O'Reilly, D.R., et al., Baculovirus Expression Vectors: A Laboratory Manual, 127. Vectors such as the INVITROGEN, MaxBac™ Baculovirus expression system, insect cells, and protocols are also preferred which are commercially available. INVITROGEN, Carlsbad, CA.
*See* EXAMPLE VI.

**Example Host Cells**

Host cells transformed with a nucleotide sequence which encodes a potassium channel molecule of the present invention may be cultured under conditions suitable for the expression and recovery of the encoded protein from cell culture. Particularly preferred embodiments of the present invention are host cells transformed with a purified polynucleotide comprising a nucleic acid sequence encoding the polypeptide having the sequence substantially as depicted in SEQ ID NO:3 or a biologically active fragment thereof. Cells of this type or preparations made from them may be used to screen for pharmacologically active modulators of the biological activity of the potassium channel. Modulators thus identified will be used for the regulation of neurophysiology as defined herein.

Eukaryotic recombinant host cells are especially preferred as otherwise descibed herein or are well known to those skilled in the art. Examples include but are not limited to yeast, mammalian cells including but not limited to cell lines of human, bovine, porcine, monkey and rodent origin, and insect cells including but not limited to Drosophila and silkworm derived cell lines. Cell lines derived from mammalian species which may be suitable and which are commercially available, include but are not limited to, L cells L-M(TK-) (ATCC CCL 1.3), L cells L-M (ATCC CCL 1.2), 293 (ATCC CRL 1573), Raji (ATCC CCL 86), CV-1 (ATCC CCL 70), COS-1 (ATCC CRL 1650), COS-7 (ATCC CRL 1651), CHO-K1 (ATCC CCL 61), 3T3 (ATCC CCL 92), NIH/3T3 (ATCC CRL 1658), HeLa (ATCC CCL 2), C127I (ATCC CRL 1616), BS-C-1 (ATCC CCL 26) and MRC-5 (ATCC CCL 171).

STRATAGENE, hNT Neurons, human neurons are *commercially available for transfection, the study of ion channel genes, and drug screening,* La Jolla, CA. Pleasure, S.J., et al., J. Neuroscience, 12:1802 (1992); J. Neuroscience, 35:585 (1993); Hiraka, G., et al., J. Virol., 65:2732 (1991); Wertkin, R., et al., PNAS, 90:9513 (1993); Younkin, D.P., et al., PNAS, 90:2174 (1993).

The expression vector may be introduced into host cells expressing the novel neuronal potassium channel polypeptide *via* any one of a number of techniques including but not limited to transformation, transfection, lipofection, protoplast fusion, and electroporation. Commercially available kits applicable for use with the present invention for hererologous expression, including well-characterized vectors, transfection reagents and conditions, and cell culture materials are well-established and readily available. CLONTECH, Palo Alto, CA; INVITROGEN, Carlsbad, CA; PHARMINGEN, San Diego, CA; STRATAGENE, LaJolla, CA. The expression vector-containing cells are clonally propagated and individually analyzed to determine the level of the novel neuron potassium channel biomolecule production. Identification of host cell clones which express the polypeptide may be performed by several means, including but not limited to immunological reactivity with antibodies described herein, and/or the presence of host cell-associated specific potassium channel activity, and/or the ability to covalently cross-link specific substrate to the neuronal poypeptide with the bifunctional cross-linking reagent disuccinimidyl suberate or similar cross-linking reagents.

The neuron biomolecule of the present invention may also be expressed as a recombinant protein with one or more additional polypeptide domains added to facilitate protein purification. Such purification facilitating domains include, but are not limited to, metal chelating peptides such as histidine-tryptophan modules that allow purification on immobilized metals (Porath, J., Protein Exp. Purif. 3:263 (1992)), protein A domains that allow purification on immobilized immunoglobulin, and the domain utilized in the FLAGS extension/affinity purification system (Immunex Corp, Seattle WA). The inclusion of a cleavable linker sequences such as Factor XA or enterokinase (Invitrogen, San Diego CA) between the purification domain and the potassium channel coding region is useful to facilitate purification.

Systems such as the CLONTECH, TALON™ nondenaturing protein purification kit for purifying 6xHis-tagged proteins under native conditions and protocols are preferred which are commercially available. CLONTECH, Palo Alto, CA.

In addition, a host cell strain may be chosen for its ability to modulate the expression of the inserted sequences or to process the expressed protein in the desired fashion. Such modifications of the polypeptide include, but are not limited to, acetylation, carboxylation, glycosylation, phosphorylation, lipidation and acylation. Post-translational processing which cleaves a nascent form of the protein may also be important for correct insertion, folding and/or function. Different host cells such as CHO, HeLa, MDCK, 293, WI38, NIH-3T3, HEK293 etc., have specific cellular machinery and characteristic mechanisms for such post-translational activities and may be chosen to ensure the correct modification and processing of the introduced, foreign protein.

For long-term, high-yield production of recombinant proteins, stable expression is preferred. For example, cell lines which stably express the novel potassium channel poypeptide may be transformed using expression vectors which contain viral origins of replication or endogenous expression elements and a selectable marker gene. Following the introduction of the vector, cells may be allowed to grow for 1-2 days in an enriched media before they are switched to selective media. The purpose of the selectable marker is to confer resistance to selection, and its presence allows growth and recovery of cells which successfully express the introduced sequences. Resistant clumps of stably transformed cells can be proliferated using tissue culture techniques appropriate to the cell type.

The human neuron biomolecule described herein can be produced in the yeast *S. cerevisiae* following the insertion of the optimal cDNA cistron into expression vectors designed to direct the intracellular or extracellular expression of the heterologous protein. In the case of intracellular expression, vectors such as EmBLyex4 or the like are ligated to the beta subunit cistron. *See, e.g.,* Rinas, U., et al., Biotechnology, 8:543 (1990); Horowitz, B., et al., J. Biol. Chem., 265:4189 (1989). For extracellular expression, a potassium channel coding region, e.g., SEQ ID NO:2, is ligated into yeast expression vectors which may employ any of a series of well-characterized secretion signals. Levels of the expressed potassium channel molecule are determined by the assays described herein.

A variety of protocols for detecting and measuring the expression of the novel potassium channel, using either polyclonal or monoclonal antibodies specific for the protein are known in the art. Examples include enzyme-linked immunosorbent assay (ELISA), radioimmunoassay (RIA) and fluorescent activated cell sorting (FACS). A two-site, monoclonal-based immunoassay utilizing monoclonal antibodies reactive to two non-interfering epitopes may be employed. Well known competitive binding techniques may also be employed. See, e.g., Hampton, R., et al. (1990), Serological Methods - a Laboratory Manual, APS Press, St Paul Minn.; Maddox, D.E., et al., J. Exp. Med. 158:1211.

## ***in vitro* synthesis of capped mRNA**

The full-length cDNA (SEQ ID NO: 1), for example, may be used in standard procedures to synthesize biologically active mRNA for functional expression in heterologous cells, in *Xenopus* oocytes, for instance, or in various types of mammalian cells including human neurons. See, e.g., Goldin A, Methods Enzymol, 207:279 (1992); STRATAGENE, hNT Neurons, *commercially available for the study of ion channel genes,* La Jolla, CA.

The coding region for the novel potassium channel described herein (e.g., a region which comprises SEQ ID NO:2) which encodes the polypeptide having a bio-active sequence substantially as depicted in SEQ ID NO:3 or a biologically active fragment thereof may be cloned 3', for example, to a bacteriophage promoter, e.g., an SP6, T7 or T3 promoter. The PGEM vectors from Promega, Madison, WI, are examples of preferred vectors which may be used with the present invention. Standard vectors known in the art, such as pSP64T or pBSTA, which enhance stabililty of the message and increase specific expression in *Xenopus* oocytes, are especially preferred. Kreig and Melton, Nucleic Acids Res., 12:7057 (1984). These particular preferred vectors contain the Xenopus beta-globin 5' and 3' untranslated mRNA regions flanking the 5'end and a poly-A tail on the 3' end of the gene.

A plasmid vector DNA construct which contains an insert which encodes the novel potassium channel described herein or a biologically active fragment thereof (e.g., SEQ ID NO: 1, or a region which comprises SEQ ID NO:2, or a truncated version thereof) may be then cut with a restriction enzyme to linearize the construct 3' to the structural coding region. The linearized vector should be extracted with phenol-chloroform-isoamyl alcohol, precipitated with ethanol and re-suspended in RNAse-free water for use as a transcription template. The transcription reaction, for example, may be carried out as described *infra* to synthesize biologically active mRNA for subsequent *in vivo* or *in vitro* translation by methods which are well-known in the art. The mMessage mMachine™ kit, AMBION, Austin, TX, is especially preferred for synthesizing biologically active mRNA. Alternately, the mRNA transcripts may be used as probes for analysis of tissue distribution ny Northern analyses and or RNAse protection assays.

## **mRNA Synthesis**

| | |
|---|---|
| PGEM, Promega, Madison, WI | |
| 10x SP6/T7 buffer | 5 ul |
| 10X ATP, CTP, UTP (5 mM each) | 5 ul |
| 10x GTP (5 mM) | 1 ul |
| 10x GpppG (Cap; 5 mM) | 5 ul |
| DTT (1M) | 0.5 ul |
| Rnase inhibitor (40U/ul) | 1.25 ul |
| Water | 27 ul |
| Linearized DNA (1 ug/ul) | 1-5 ul |
| SP6 or T7 RNA polymerase (20U/ul) | 1-3 ul |
| TOTAL REACTION VOLUME | 50 ul |

Incubate at 37 degrees for 1-2 hours. Add 1 ul of RNAse-free Dnase to degrade template DNA. Digest for 10 minutes. Add 75 ul of water. Extract with phenol/CHCl3. Ethanol precipitate RNA. Store in aliqots at -70 degrees.

## **Functional Expression**

Biologically active mRNA can be introduced into heterologous cells for functional expression and analyses by methods well-known in the art. Synthetic mRNA from example constructs described *supra,* for example, may be injected into *Xenopus* oocytes for functional expression and analyses. Goldin, A., Methods Enzyml.,207:266, (1992). Hererologous potassium channels may be examined using standard two-electrode voltage clamp techniques. *See, e.g.,* Stuhmer, W., Methods in Enzymol., 207:319, (1992); Kohler, et al., Science, 273:1709 (1996). Potassium concentrations inside the cell may be altered, for example, by adding a potassium-ionophore, co-expression with a receptor that causes a rise in intracellular potassium. Potassium concentrations may alternately be altered by pulling inside-out patches and changing potassium concentrations in the bath medium. E.g., Grissmer, S., et al., Calcium-activated Potassium Channels in Resting and Activated Human T Lymphocytes, J. Gen. Physiol, 102:601 (1993). Standard biophysical parameters, such as activation, potassium dependence, single-channel conductance, inactivation, tail currents, potassium selectivity, and thorough pharmacology of various K channel blockers including TEA, Apamin, and others may also be tested.

Grissmer, S., et al., Calcium-activated Potassium Channels in Resting and Activated Human T Lymphocytes, J. Gen. Physiol, 102:601 (1993).

Alternatively, cRNA (synthetic mRNA from a cDNA construct) can be introduced into heterologous mammalian cells, for example, RBL cells (ATCC # CRL 1378), and 293 cells (ATCC # CRL 1573), may be transformed using standard art-methods. For example, the Eppendorf microinjection system may be used (Micromanipulator 5171 and Transjector 5242). Transformed cells may be analysed for K+ currents about 4 hours later using patch-clamp techniques which are well-documented. *E.g.,* Ikeda, et al., Pfueg. Arch. Eur. J. Physiol., 422:201 (1992); Grissmer, et al., J. Gen. Physiol., 102:601 (1993).

**Over-expression of the novel channel in cell-lines**

Transient and/or stable eucaryotic transfectant cells comprised of the coding region(s) described herein are contemplated for high-level expression of the novel potassium channel.

Eucaryotic transfectants are preferred embodiments of the present invention for employment in pharmacological target binding studies for the identification molecules which block the novel channel described herein *in vivo.* HEK cells are particularly preferred.

Transient expression of coding regions for the novel channel can be achieved by straight transfection into mammalian cells, by standard techniques. Omari, K. et al., J. Physiol., 499:369, (1997); Panyi, G. et al., J. Gen. Physiol., 107(3):409 (1996). High level transient expression may be achieved using standard viral systems, e.g., Baculovirus, Adenovirus, or Vaccinia virus. Channel numbers resulting from these systems are typically 5-500K per cell. Kamb, A., Methods Enzymol. 207:423 (1992); Sun, T. et al., Biochemistry, 33(33):9992 (1994); Spencer, R.H., et al., J. Biol. Chem., 272:2389 (1997).

Stable transfection of heterologous cells using sequences which encode the novel potassium channel described herein (SEQ ID NO:3) or biologically active variations or fragments thereof can be generated using, for example, NIH-3t3, L929, COS, HEK, or CHO cells. *See, e.g.,* EMBO, 11 (6):2033 (1992); Grissmer, et al., Mol. Pharm., 45:1227 (1994).

A preferred vector for use with the present invention is pcDNA/Neo, which is commercially available from INVITROGEN, Carlsbad, CA.

Cells, NIH-3t3, for example, are grown to 50% confluency in 60mm plates (media, and conditions are according to requirements of the particular cell line) and transfected with 5 ug of pure DNA comprising a coding region for the novel potassium channel, e.g. SEQ ID NO:2, in pCDNA/Neo using the Lipofection reagent, as described by the supplier (LIFE TECHNOLOGIES Gibco BRL, Bethesda, MD). After transfection, the cells are incubated at 37˚C, conditions for 3 days in medium with 10% FCS. Cells are trypsinized seeded onto 100mm dishes, and then selected with 300ug/ml of G418 (Neomycin). Only cells that have stable integration of the heterologous coding region will grow in the presence of G418, which is confered by the Neomycin-resistance gene in the plasmid. Isolated clones are processed for 2-3 rounds of purification and subjected to patch-clamp analysis for K+ currents.

Since the novel gene, e.g., SEQ ID NO:1 is highly expressed in differentiated neurons, and since potassium channels are potently blocked by particular ligands including but not limited to tetraethylammonium chloride (TEA), 4-aminopyridine (4-AP, as well as 2-AP and 3-AP), 3,4- and 2,3-diaminopyridine, $BaCl_2$, CsCl, strychnine, phencyclidine, pyridostigmine, 9-aminoacridine, DuP-996 (3,3-bis (4-pyridinylmethyl)-1-phenylindolin-2-one; linopiridine), clofilium, quinidine, aminoquinolines and quinine, various cell-lines that heterologously over-expresses the novel channel structural coding regions described herein can be used in radio-labeled binding assays to screen for pharmacologically active molecules which block the novel channel, using a radiolabelled ligand, as a measurable displacement entity in a binding assay or competitive binding assay. Peptide toxins including but not limited to stichodactylotoxin, apamin, charybdotoxin, kaliotoxin, and margotoxin may also be used as ligands in the binding assays described herein. *See,* for instance, EXAMPLE XI.

**Ligand-binding assay for high-throughput screening for modulators of the novel channel**

Embodiments of the present invention are cell-lines that heterologously over-expresses the calcium activated potassium channel coding region described herein (e.g., SEQ ID NO:1 or a biologically active truncated version thereof or a chimeric fusion) and their use in binding assays to identify pharmacologically active molecules which block the novel channel.

For example, a radio-labeled binding assay using a radiolabelled ligand may be used as previously decribed by Hill, R.J., Mol. Pharm., 48:98 (1995), and Deutsch, C., et al., J. Biol. Chem., 266:3668 (1991). Membrane preparations of cell-lines which over-express the novel potassium channel described herein are made by homogenizing the cells using a Polytron for 25 seconds at 13,000 RPM and spun at low speed (100 g) for 2 minutes. The supernatant is spun at high speed (50,000 g) for 10 minutes. The pellet is suspended in 1 ml of assay buffer (5 mM Nacl, 5 mM KCL, 10 mM HEPES, 6 mM glucose, pH 8.4) and diluted to 50 ug/ml.

To each well of a 96-well microtiter plate, 130 ul of assay buffer is added, as well as 20 ul of test molecule compound (test drug may be, for instance, a small molecule, peptide, analog or mimetic compound) /control assay buffer /non-specific (10 nM cold ligand) (not-labelled) 50 ul of membranes from cells which over-express the novel potassium channel at 50 ug/ml and 50 ul of radioligand (25 pM; NEN, 2200 Ci/mmol) are incubated for 20 minutes at 21˚C with mixing. Bound radiolabeled ligand is separated from free radiolabeled ligand in solution by filtering over pre-soaked GF/C Unifilters (Packard Instruments) and washing rapidly in ice-cold wash buffer. Upon drying, the filter plates are scintillation counted. Data from saturation experiments are subject to Scatchard analysis and linear regression. Deutsch, et al., J. Biol. Chem., 266:3668 (1991). Compounds that compete with the radio-labeled ligand for binding the novel potassium channel are identified which produce a reduction in specific counts.

In another embodiment, a scintillation proximity assay (SPA) that eliminates the need for filters, can be easily adapted for high throuput screening (HTS) assays. Hoffman, R., et al., Anal. Biochem., 20370 (1992); Kienuis, C.B.M., et al., J. Recept. Res., 12:389 (1992).

**$^{86}$Rb (or $^{42}$K) efflux assays in cells heterologously expressing the novel potassium channel polypeptide**

Cells are grown to confluence in DMEM +10% FCS in six well plates. Cells are incubated in HEPES buffer solution (in mM: NaCl 137, KCl 4.7, $MgSO_4$ 0.6, $CaCl_2$ 1.8, HEPES 20, Glucose 7.7) containing $^{86}$Rb (5 mCi/ml) overnight in a incubator at 37$^0$C. At the end of incubation HEPES containing $^{86}$Rb is aspirated and cells are washed with tracer free HEPES to remove extracellular $^{86}$Rb. Cells are incubated with tracer free HEPES for 3 minutes and at the end of 3 minutes HEPES is aspirated and fresh HEPES is added to the cells. After the basal release of $^{86}$Rb reaches a steady state the cells are exposed to HEPES containing higher KCl (20 mM) concentration for 6 minutes (3 min.X 2) followed by another solution containing 20 mM KCl and compound of choice for 12 minutes (3 min.X 4). At the end of the procedure 500 mL of NaOH is added to the wells and cells are scraped. The control cells are treated with HEPES containing 20 mM KC1 and vehicle (DMSO) for 12 min. At the end of experiment radioactivity in aspirated solutions and the cells are counted in a gamma counter. The efflux rate constants (k min$^{-1}$), which represents the radioactivity released per minute expressed as a percentage of the radioactivity remaining in the tissue, is calculated. Results are expressed as maximum % increase in efflux rate constant obtained as a difference between the peak increase in rate constant and the average rate constant during 10 minutes before candidate compound application. *See also* EXAMPLE XII.

Alternately, a cell-line (eg: HEK 293, CHO, COS or SF9) heterologously expressing a novel nucleic acid sequence described herein, e.g., SEQ ID NO:2 to yield the biologically active peptide SEQ ID NO:3, is incubated with $^{86}$RbCl (10 mCi/ml) for 18 hours at 37 degrees in 5% $CO_2$. Cells are washed in low-$K^+$ HBSS medium and resuspended at 5.5 X $10^6$ cells/ml in low-$K^+$ HBSS. Fifty ul of the cell suspension is incubated for 15 minutes at ambient temperature with 10 ul of candidate compound in a well of Millipore Multi-screen 96-well 0.65 um filtration plate (# MADV-N6550). The mixture is then incubated for 20 minutes at the same temperature with 125 ul of 140 mM $K^+$-HBSS added to initiate Rb efflux via membrane depolarization and filtered with a TV-1 vacuum transfer manifold (Pall Trinity Micro, Cortland, NY) into standard 96-well microtiter plates; 100 ul of the filtrate is removed and counted in a Beckman liquid scintillation counter (Palo Alto, CA) for one minute. Compounds that inhibit the potassium channel activity will have reduced Rb efflux. *See also* Example XII.

**Laser-Based Fluorescent Imaging Plate Reader (FLIPR)**

Since the novel potassium channel described herein has all the molecular features of a voltage-gated potassium channel, a fluroscent assay that detects changes in membrane potential in cells functionally expressing the novel biomolecule is another method for rapid screening of small molecules that modulate channel activity. Such an assay may be set up using fluorescent membrane potential sensing dyes such as Bis-oxynol. Brauner, et al., Biochimica et Biophysica Acta, 771:208 (1984). Cells expresssing SEQ ID NO:2, for example, are incubated with the dye and candidate compound modulator. Membrane depolarization is triggered by increasing potassium concentration external to the cells. Normally, the dye partition into the membrane upon sensing depolarization, leading to a change in fluorescence. Cells expressing SEQ ID NO:2 would open in response to depolarization and efflux potassium ions and cause a counteractive hyperpolarization and reduced efficiency of the dye to change fluorescence. The presence of ligands, either agonists or antagonists of the novel potassium channel would modulate changes in fluorescence which are detected optically. The FLIPR instrument is now commercially available through Molecular Devices Inc., Sunnyvale, CA, and can be adapted to a very rapid and sensitive 96-well screening assay. *See* EXAMPLE XIII.

**Various Screening Assays**

The present invention is also directed to methods for screening for compounds which modulate the biological activity of the novel potassium channel and/or the biological activity of neurons *in vivo.* Compounds which modulate these activities may be DNA, RNA, peptides, proteins, or non-proteinaceous organic molecules. Compounds may modulate the activity by increasing or attenuating the expression of DNA or RNA which encodes the potassium channel, or may antagonize or agonize the biological activity of the novel potassium channel itself. Compounds that modulate the expression of DNA or RNA encoding the human potassium channel or the function of the polypeptide may be detected by a variety of assays. The assay may be a simple "yes/no" assay to determine whether there is a change in expression or function. The assay may be made quantitative by comparing the expression or function of a test sample with the levels of expression or function in a standard sample.

The human brain-derived potassium channel described herein, its immunogenic fragments or oligopeptides can be used for screening therapeutic compounds in any of a variety of drug screening techniques. The fragment employed in such a test may be free in solution, affixed to a solid support, borne on a cell surface, or located intracellularly. The abolition of activity or the formation of binding complexes, between the potassium channel biomolecule and the agent being tested, may be measured. Accordingly, the present invention provides a method for screening a plurality of compounds for specific binding affinity with the potassium channel polypeptide or a fragment thereof, comprising providing a plurality of compounds; combining a polypeptide of the present invention or a fragment thereof with each of a plurality of compounds for a time sufficient to allow binding under suitable conditions; and detecting binding of the trans-activator molecule, or fragment thereof, to each of the plurality of compounds, thereby identifying the compounds which specifically bind the human brain-derived biomolecule.

Methods of identifying compounds that modulate the activity of a potassium channel polypeptide are generally preferred, which comprise combining a candidate compound modulator of a potassium channel biological activity with a polypeptide of a potassium channel having the sequence substantially as depicted in SEQ ID NO:3, and measuring an effect of the candidate compound modulator on the biological activity of the potassium channel (e.g., physical binding interaction, ability to pass K+ ions, neurophysiological effect on neurons).

A further method of identifying compounds that modulate the biological activity of a potassium channel, comprises combining a candidate compound modulator of a potassium channel biological activity with a host-cell expressing a potassium channel polypeptide having the sequence substantially as depicted in SEQ ID NO:3, and measuring an effect of the candidate compound modulator on the biological activity of the potassium channel (e.g., physical binding interaction, ability to pass K+ ions, measurable biophysical effect, neurophysiological effect on neurons).

Another preferred method is one of identifying compounds that modulate neurophysiology, comprising combining a candidate compound modulator of neurophysiology with a polypeptide of a potassium channel having the sequence substantially as depicted in SEQ ID NO: 3, and measuring an effect of the candidate compound modulator on a biological activity of the potassium channel.

Methods of identifying compounds that modulate the activity of a potassium channel and/or modulate or regulate neurophysiology, are also preferred which comprise combining a candidate compound modulator with a host-cell expressing a potassium channel polypeptide (or *capable* of expressing a potassium channel polypeptide via e.g., inducible expression) having the sequence substantially as depicted in SEQ ID NO:3, and measuring an effect of the candidate compound modulator on a biological activity or physiological effect of the potassium channel. Preferred cellular assays for modulators fall into two general categories:

1) direct measurement of the physical potassium channel biological activity, and 2) measurement of physiological or neurophysiological effect. These methods can employ the endogenous brain-derived potassium channel descibed herein, or overexpressed recombinant potassium channel polypeptide in heterologous cells, including human neurons.

In order to purify a potassium channel poypeptide to measure a biological binding activity, the source may be a whole cell lysate, prepared by one to three freeze-thaw cycles in the presence of standard protease inhibitors. The potassium channel may be partially or completely purified by standard protein purification methods. The potassium channel polypeptides described herein may be purified by affinity chromatography using specific antibody described herein or by ligands specific for an epitope tag engineered into the recombinant molecule moreover described herein. The preparation may then be assayed for binding activity as described.

Purified polypeptides comprising the amino acid sequence substantially as depicted in SEQ ID NO:3 are especially preferred embodiments of the present invention.

Compounds which are identified generally according to methods described, referenced, and contemplated herein that modulate the activity of a potassium channel (preferably regulate physiology, most preferably regulate neurophysiology) are especially preferred embodiments of the present invention.

An especially preferred embodiment of the present invention is a method for treatment of a patient in need of such treatment for a condition which is mediated by the novel human brain-derived potassium channel described herein comprising administration of a therapeutically effective amount of a potassium channel modulating compound identified by a method described herein.

Another especially preferred embodiment of the present invention is a method for treatment of a patient in need of such treatment for a condition which is mediated by dysfunctional neurons, or a neurophysiological disorder, comprising administration of a therapeutically effective amount of a potassium channel modulating compound identified by a method described herein.

A further especially preferred embodiment of the present invention is a method for treatment of a patient in need of such treatment for a condition which is mediated by neurophysiology comprising administration of a therapeutically effective amount of a compound modulator of neurophysiology identified by a method described herein.

**Yeast 2-Hybrid System**

In another embodiment of the invention, a nuleic acid sequence which encodes a potassium channel molecule substantially as depicted in SEQ ID NO:3 or a biologically active fragment thereof may be ligated to a heterologous sequence to encode a fusion protein. For example, for screening compounds for modulation of biological activity, further described *infra,* it may be useful to encode a chimeric potassium channel molecule as described herein for expression in hererologous host cells. Chimeric constructs may also be used to express a 'bait', according to methods well known using a yeast two-hybrid system, to identify accessory native peptides that may be associated with the novel neuronal biomolecule described herein. Fields, S., et al., Trends Genet., 10:286 (1994); Allen, J.B., et al., TIBS, 20:511 (1995). A yeast two-hybrid system has been described wherein protein:protein interactions can be detected using a yeast-based genetic assay via reconstitution of transcriptional activators. Fields, S., Song, O., Nature 340:245 (1989). The two-hybrid system used the ability of a pair of interacting proteins to bring a transcription activation domain into close proximity with a DNA-binding site that regulates the expression of an adjacent reporter gene. Commercially available systems such as the CLONTECH, Matchmaker™ systems and protocols may be used with the present invention. CLONTECH, Palo Alto, CA. *See also,* Mendelsohn, A.R., Brent, R., Curr. Op. Biotech., 5:482 (1994); Phizicky. E.M., Fields, S., Microbiological Rev., 59(1):94 (1995); Yang, M., et al., Nucleic Acids Res., 23(7):1152 (1995); Fields, S., Stemglanz, R., TIG, 10(8):286 (1994); and US Patents 5,283,173, *System to Detect Protein-Protein Interactions,* and 5,468,614.

Modified screening systems, for instance, can be practiced either with a positive readout or with a negative readout such as that in the recently developed versions of "Reverse Y2H" approach. *See, e.g.,* Vidal M, Braun P, Chen E, Boeke JD, Harlow E (1996) Genetic characterization of a mammalian protein-protein interaction domain by using a yeast reverse two-hybrid system, Proc Natl Acad Sci U S A 17;93(19):10321-10326; Vidal M, Brachmann RK, Fattaey A, Harlow E, Boeke JD (1996) Reverse two-hybrid and one-hybrid systems to detect dissociation of protein-protein and DNA-protein interactions. Proc Natl Acad Sci U S A 17;93(19):10315-10320; White MA (1996) The yeast two-hybrid system: forward and reverse, Proc Natl Acad Sci U S A 17;93(19):10001-10003; Leanna CA, Hannink M (1996), The reverse two-hybrid system: a genetic scheme for selection against specific protein/protein interactions, Nucleic Acids Res 1;24(17):3341-3347.

**Antibodies**

Monospecific antibodies to the neuronal biomolecule of the present invention are purified from mammalian antisera containing antibodies reactive against the polypeptide or are prepared as monoclonal antibodies reactive with a human brain-derived potassium channel polypeptide using the technique of Kohler and Milstein, Nature, 256:495 (1975). Monospecific antibody as used herein is defined as a single antibody species or multiple antibody species with homogenous binding characteristics for the novel human brain-derived potassium channel. Homogenous binding as used herein refers to the ability of the antibody species to bind to a specific antigen or epitope, such as those associated with the novel transcription activator, as described. Human neuronal potassium channel specific antibodies are raised by immunizing animals such as mice, rats, guinea pigs, rabbits, goats, horses and the like, with rabbits being preferred, with an appropriate concentration of the human neuronal potassium channel either with or without an immune adjuvant.

Preimmune serum is collected prior to the first immunization. Each animal receives between about 0.1 mg and about 1000 mg of neuronal potassium channel polypeptide associated with an acceptable immune adjuvant. Such acceptable adjuvants include, but are not limited to, Freund's complete, Freund's incomplete, alum-precipitate, water in oil emulsion containing Corynebacterium parvum and tRNA. The initial immunization consists of a neuronal potassium channel polypeptide in, preferably, Freund's complete adjuvant at multiple sites either subcutaneously (SC), intraperitoneally (IP) or both. Each animal is bled at regular intervals, preferably weekly, to determine antibody titer. The animals may or may not receive booster injections following the initial immunization. Those animals receiving booster injections are generally given an equal amount of the antigen in Freund's incomplete adjuvant by the same route. Booster injections are given at about three week intervals until maximal titers are obtained. At about 7 days after each booster immunization or about weekly after a single immunization, the animals are bled, the serum collected, and aliquots are stored at about - 20° C.

Monoclonal antibodies (mAb) reactive with the brain-derived potassium channel polypeptide are prepared by immunizing inbred mice, preferably Balb/c, with a potassium channel polypeptide. The mice are immunized by the IP or SC route with about 0.1 mg to about 10 mg, preferably about 1 mg, of the novel neuronal potassium channel polypeptide in about 0.5 ml buffer or saline incorporated in an equal volume of an acceptable adjuvant, as discussed above. Freund's complete adjuvant is preferred. The mice receive an initial immunization on day 0 and are rested for about 3 to about 30 weeks. Immunized mice are given one or more booster immunizations of about 0.1 to about 10 mg of neuronal potassium channel polypeptide in a buffer solution such as phosphate buffered saline by the intravenous (IV) route. Lymphocytes, from antibody positive mice, preferably splenic lymphocytes, are obtained by removing spleens from immunized mice by standard procedures known in the art. Hybridoma cells are produced by mixing the splenic lymphocytes with an appropriate fusion partner, preferably myeloma cells, under conditions which will allow the formation of stable hybridomas. Fusion partners may include, but are not limited to: mouse myelomas P3/NS1/Ag 4-1; MPC-11; S-194 and Sp 2/0, with Sp 2/0 being preferred. The antibody producing cells and myeloma cells are fused in polyethylene glycol, about 1000 molecular weight, at concentrations from about 30% to about 50%. Fused hybridoma cells are selected by growth in hypoxanthine, thymidine and aminopterin supplemented Dulbecco's Modified Eagles Medium (DMEM) by procedures known in the art. Supernatant fluids are collected from growth positive wells on about days 14, 18, and 21 and are screened for antibody production by an immunoassay such as solid phase immunoradioassay (SPIRA) using the human neuronal potassium channel polypeptide as the antigen. The culture fluids are also tested in the Ouchterlony precipitation assay to determine the isotype of the mAb. Hybridoma cells from antibody positive wells are cloned by a technique such as the soft agar technique of MacPherson, Soft Agar Techniques, in Tissue Culture Methods and Applications, Kruse and Paterson, Eds., Academic Press, 1973.

Monoclonal antibodies are produced in vivo by injection of pristane primed Balb/c mice, approximately 0.5 ml per mouse, with about 2 x $10^6$ to about 6 x $10^6$ hybridoma cells about 4 days after priming. Ascites fluid is collected at approximately 8-12 days after cell transfer and the monoclonal antibodies are purified by techniques known in the art.

*In vitro* production of the anti- human neuronal potassium channel polypeptide mAb is carried out by growing the hydridoma in DMEM containing about 2% fetal calf serum to obtain sufficient quantities of the specific mAb. The mAb are purified by techniques known in the art.

## Diagnostic Assays

Antibody titers of ascites or hybridoma culture fluids are determined by various serological or immunological assays which include, but are not limited to, precipitation, passive agglutination, enzyme-linked immunosorbent antibody (ELISA) technique and radioimmunoassay (RIA) techniques. Similar diagnostic assays are used to detect the presence of the novel potassium channel biomolecule in body fluids or tissue and cell extracts.

Diagnostic assays using the human neuron potassium channel polypeptide specific antibodies are useful for the diagnosis of conditions, disorders or diseases characterized by abnormal expression of the potassium channel or expression of genes associated with abnormal cell growth or abnormal neurophysiology. Diagnostic assays for the neural biomolecule of this invention include methods utilizing the antibody and a label to detect the human potassium channel polypeptide in human body fluids, cells, tissues or sections or extracts of such tissues. The polypeptides and antibodies of the present invention may be used with or without modification. Frequently, the polypeptides and antibodies will be labeled by joining them, either covalently or noncovalently, with a reporter molecule, a myriad of which are well-known to those skilled in the art.

A variety of protocols for measuring the potassium channel polypeptide, using either polyclonal or monoclonal antibodies specific for the respective protein are known in the art. Examples include enzyme-linked immunosorbent assay (ELISA), radioimmunoassay (RIA) and fluorescent activated cell sorting (FACS). A two-site, monoclonal-based immunoassay utilizing monoclonal antibodies reactive to two non-interfering epitopes on the human brain-derived potassium channel polypeptide is preferred, but a competitive binding assay may be employed. These assays are described, among other places, in Maddox, D.E. et al., J. Exp. Med. 158:1211 (1983); Sites, D.P., et al., Basic and Clinical Immunology, Ch.22, 4th Ed., Lange Medical Publications, Los Altos, CA (1982); U.S. Patents No. 3,654,090, No. 3,850,752; and No. 4,016,043.

In order to provide a basis for the diagnosis of disease, normal or standard values for the human potassium channel polypeptide expression must be established. This is accomplished by combining body fluids or cell extracts taken from normal subjects, either animal or human, with antibody to the human neuron channel biomolecule under conditions suitable for complex formation which are well known in the art. The amount of standard complex formation may be quantified by comparing it with a dilution series of positive controls where a known amount of antibody is combined with known concentrations of purified potassium channel polypeptide. Then, standard values obtained from normal samples may be compared with values obtained from samples from subjects potentially affected by a disorder or disease related to the channel biomolecule expression. Deviation between standard and subject values establishes the presence of the disease state.

Kits containing potassium channel nucleic acid, antibodies to a channel polpeptide, or protein may be prepared.

Such kits are used to detect heterologous nucleic acid which hybridizes to potassium channel nucleic acid, or to detect the presence of protein or peptide fragments in a sample. Such characterization is useful for a variety of purposes including, but not limited to, forensic analyses and epidemiological studies.

The DNA molecules, RNA molecules, recombinant protein and antibodies of the present invention may be used to screen and measure levels of the novel potassium channel DNA, RNA or protein. The recombinant proteins, DNA molecules, RNA molecules and antibodies lend themselves to the formulation of kits suitable for the detection and typing of the novel human potassium channel biomolecule. Such a kit would comprise a compartmentalized carrier suitable to hold in close confinement at least one container. The carrier would further comprise reagents such as recombinant potassium channel or anti-potassium channel antibodies suitable for detecting the novel potassium channel biomolecule. The carrier may also contain a means for detection such as labeled antigen or enzyme substrates or the like.

Polynucleotide sequences which encode the novel potassium channel may be used for the diagnosis of conditions or diseases with which the expression of the novel neurophysiological biomolecule is associated. For example, polynucleotide sequences encoding a potassium channel may be used in hybridization or PCR assays of fluids or tissues from biopsies to detect expression of the biomolecule. The form of such qualitative or quantitative methods may include Southern or Northern analysis, dot blot or other membrane-based technologies; PCR technologies; dip stick, pin, chip and ELISA technologies. All of these techniques are well known in the art and are the basis of many commercially available diagnostic kits. Such assays may also be used to evaluate the efficacy of a particular therapeutic treatment regime in animal studies, in clinical trials, or in monitoring the treatment of an individual patient. Once disease is established, a therapeutic agent is administered and a treatment profile is generated. Such assays may be repeated on a regular basis to evaluate whether the values in the profile progress toward or return to the normal or standard pattern. Successive treatment profiles may be used to show the efficacy of treatment over a period of several days or several months.

Polynucleotide sequences which encode the novel human potassium channel may also be employed in analyses to map chromosomal locations, e.g., screening for functional association with disease markers. Moreover the sequences described herein are contemplated for use to identify human sequence polymorphisms and possible association with disease as well as analyses to select optimal sequence from among possible polymorphic sequences for the design of compounds to modulate the biological activity and therefore regulate physiological disorders, most preferably neurophysiological disorders *in vivo.* Furthermore the sequences are contemplated as screening tools for use in the identification of appropriate human subjects and patients for therapeutic clinical trials.

## Purification *via* Affinity Columns

It is readily apparent to those skilled in the art that methods for producing antibodies may be utilized to produce antibodies specific for potassium channel polypeptide fragments, or the full-length nascent human potassium channel polypeptide. Specifically, it is readily apparent to those skilled in the art that antibodies may be generated which are specific for the fully functional biomolecule or fragments thereof.

Potassium channel polypeptide antibody affinity columns are made by adding the antibodies to Affigel-10 (Biorad), a gel support which is activated with N hydroxysuccinimide esters such that the antibodies form covalent linkages with the agarose gel bead support. The antibodies are then coupled to the gel via amide bonds with the spacer arm. The remaining activated esters are then quenched with 1M ethanolamine HCl (pH 8). The column is washed with water followed by 0.23M glycine HCl (pH 2.6) to remove any non-conjugated antibody or extraneous protein. The column is then equilibrated in phosphate buffered saline (pH 7.3) with appropriate detergent and the cell culture supernatants or cell extracts, for example, containing human potassium channel polypeptide made using appropriate membrane solubilizing detergents are slowly passed through the column. The column is then washed with phosphate buffered saline/detergent until the optical density falls to background, then the protein is eluted with 0.23M glycine-HCl (pH 2.6) /detergent. The purified potassium channel polypeptide is then dialyzed against phosphate buffered saline/detergent.

Recombinant potassium channel molecules can be separated from other cellular proteins by use of an immunoaffinity column made with monoclonal or polyclonal antibodies specific for full length nascent human potassium channel polypeptide, or polypeptide fragments of the biomolecule.

Potassium channel polypeptides described herein may be used to affinity purify biological effectors from native biological materials, e.g. disease tissue. Affinity chromatography techniques are well known to those skilled in the art. A potassium channel peptide described herein or an effective fragment thereof, is fixed to a solid matrix, e.g. CNBr activated Sepharose according to the protocol of the supplier (Pharmacia, Piscataway, NJ), and a homogenized/buffered cellular solution containing a potential molecule of interest is passed through the column. After washing, the column retains only the biological effector which is subsequently eluted, e.g., using 0.5M acetic acid or a NaCl gradient.

## Antisense Molecules

The cDNA sequence SEQ ID NO:1 provided herein, may be used in another embodiment of the invention to study the physiological relevance of the novel potassium channel in cells by knocking out expression of the endogenous gene by use of anti-sense constructs.

To enable methods of down-regulating expression of the novel potassium channel of the present invention in mammalian cells, an example antisense expression construct containing the complement DNA sequence to the sequence substantially as depicted in SEQ ID NO:2 can be readily constructed for instance using the pREP10 vector (Invitrogen Corporation). Transcripts are expected to inhibit translation of the wild-type potassium channel mRNA in cells transfected with this type construct. Antisense transcripts are effective for inhibiting translation of the native gene transcript, and capable of inducing the effects (e.g., regulation of neurophysiological disorders) herein described. Translation is most effectively inhibited by blocking the mRNA at a site at or near the initiation codon. Thus, oligonucleotides complementary to the corresponding 5' -terminal region of the potassium channel mRNA transcript are preferred. Secondary or tertiary structure which might interfere with hybridization is minimal in this region. Moreover, sequences that are too distant in the 3' direction from the initiation site can be less effective in hybridizing the mRNA transcripts because of a "read-through" phenomenon whereby the ribosome appears to unravel the antisense/sense duplex to permit translation of the message. Oligonucleotides which are complementary to and hybridizable with any portion of the novel potassium channel mRNA are contemplated for therapeutic use.

U.S. Patent No. 5,639,595, *Identification of Novel Drugs and Reagents,* issued Jun. 17, 1997, thoroughly describes methods of identifying oligonucleotide sequences that display *in vivo* activity. Expression vectors containing random oligonucleotide sequences derived from previously known polynucleotides are transformed into cells. The cells are then assayed for a phenotype resulting from the desired activity of the oligonucleotide. Once cells with the desired phenotype have been identified, the sequence of the oligonucleotide having the desired activity can be identified. Identification may be accomplished by recovering the vector or by polymerase chain reaction (PCR) amplification and sequencing the region containing the inserted nucleic acid material.

Nucleotide sequences that are complementary to the novel potassium channel polypeptide encoding polynucleotide sequence can be synthesized for antisense therapy. These antisense molecules may be DNA, stable derivatives of DNA such as phosphorothioates or methylphosphonates, RNA, stable derivatives of RNA such as 2'-O-alkylRNA, or other oligonucleotide mimetics. U.S. Patent No. 5,652,355, *Hybrid Oligonucleotide Phosphorothioates,* issued July 29, 1997, and U.S. Patent No. 5,652,356, *Inverted Chimeric and Hybrid Oligonucleotides,* issued July 29, 1997, describe the synthesis and effect of physiologically-stable antisense molecules. Potassium channel antisense molecules may be introduced into cells by microinjection, liposome encapsulation or by expression from vectors harboring the antisense sequence. Antisense therapy may be particularly useful for the treatment of diseases where it is beneficial to modulate the biological activity of the potassium channel described herein.

## Gene Therapy

A potassium channel polypeptide described herein may administered to a subject *via* gene therapy. A polypeptide of the present invention may be delivered to the cells of target organs in this manner. Conversely, potassium channel polypeptide *antisense* gene therapy may be used to modulate the expression of the polypeptide in the cells of target organs and hence regulate biological activity. The potassium channel polypeptide coding region can be ligated into viral vectors which mediate transfer of the trans-activator polypeptide nucleic acid by infection of recipient host cells. Suitable viral vectors include retrovirus, adenovirus, adeno-associated virus, herpes virus, vaccinia virus, polio virus and the like. *See, e.g.,* U.S. Patent No. 5,624,820, *Episomal Expression Vector for Human Gene Therapy,* issued April 29, 1997.

Nucleic acid coding regions of the present invention are incorporated into effective eukaryotic expression vectors, which are directly administered or introduced into somatic cells for gene therapy (a nucleic acid fragment comprising a coding region, preferably mRNA transcripts, may also be administered directly or introduced into somatic cells). *See, e.g.,* U.S. Patent No. 5,589,466, issued Dec. 31, 1996. Such nucleic acids and vectors may remain episomal or may be incorporated into the host chromosomal DNA as a provirus or portion thereof that includes the gene fusion and appropriate eukaryotic transcription and translation signals, i.e, an effectively positioned RNA polymerase promoter 5' to the transcriptional start site and ATG translation initiation codon of the gene fusion as well as termination codon(s) and transcript polyadenylation signals effectively positioned 3' to the coding region. Alternatively, the novel potassium channel polypeptide DNA can be transferred into cells for gene therapy by non-viral techniques including receptor-mediated targeted DNA transfer using ligand-DNA conjugates or adenovirus-ligand-DNA conjugates, lipofection membrane fusion or direct microinjection. These procedures and variations thereof are suitable for *ex vivo,* as well as *in vivo* human gene therapy according to established methods in this art.

## PCR Diagnostics

The nucleic acid sequence, oligonucleotides, fragments, portions or antisense molecules thereof, may be used in diagnostic assays of body fluids or biopsied tissues to detect the expression level of the novel human brain-derived potassium channel molecule. For example, sequences designed from the cDNA sequence SEQ ID NO: or sequences comprised in SEQ ID NO:2 can be used to detect the presence of the mRNA transcripts in a patient or to monitor the modulation of transcripts during treatment.

One method for amplification of target nucleic acids, or for later analysis by hybridization assays, is known as the polymerase chain reaction ("PCR") or PCR technique. The PCR technique can be applied to detect sequences of the invention in suspected samples using oligonucleotide primers spaced apart from each other and based on the genetic sequence, e.g., SEQ ID NO: 1, set forth herein. The primers are complementary to opposite strands of a double stranded DNA molecule and are typically separated by from about 50 to 450 nucleotides or more (usually not more than 2000 nucleotides). This method entails preparing the specific oligonucleotide primers followed by repeated cycles of target DNA denaturation, primer binding, and extension with a DNA polymerase to obtain DNA fragments of the expected length based on the primer spacing. One example embodiment of the present invention is a diagnostic composition for the identification of a polynucleotide sequence comprising the sequence substantially as depicted in SEQ ID NO:2 comprising the PCR primers substantially as depicted in EXAMPLE II. The degree of amplification of a target sequence is controlled by the number of cycles that are performed and is theoretically calculated by the simple formula 2n where n is the number of cycles. *See, e.g.,* Perkin Elmer, PCR Bibliography, Roche Molecular Systems, Branchburg, New Jersey; CLONTECH products, Palo Alto, CA; U.S. Patent No. 5,629,158, *Solid Phase Diagnosis of Medical Conditions,* issued May 13, 1997.

## Compositions

Pharmaceutically useful compositions comprising sequences pertaing to the novel potassium channel polypeptide, DNA, RNA, antisense sequences, or the human polypeptide itself, or variants and analogs which have biological activity or otherwise compounds which modulate cell physiology identified by methods described herein , may be formulated according to known methods such as by the admixture of a pharmaceutically acceptable carrier. Examples of such carriers and methods of formulation may be found in Remington's Pharmaceutical Sciences (Maack Publishing Co, Easton, PA). To form a pharmaceutically acceptable composition suitable for effective administration, such compositions will contain an effective amount of the protein, DNA, RNA, or compound modulator.

Therapeutic or diagnostic compositions of the invention are administered to an individual in amounts sufficient to treat or diagnose human physiological disorders or neurophysiological disorders. The effective amount may vary according to a variety of factors such as the individual's condition, weight, sex and age. Other factors include the mode of administration.

The term "chemical derivative" describes a molecule that contains additional chemical moieties which are not normally a part of the base molecule. Such moieties may improve the solubility, half-life, absorption, etc. of the base molecule. Alternatively the moieties may attenuate undesirable side effects of the base molecule or decrease the toxicity of the base molecule. Examples of such moieties are described in a variety of texts, such as *Remington's Pharmaceutical Sciences.*

Pharmaceutical compositions suitable for use in the present invention include compositions wherein the active ingredients are contained in an effective amount to achieve the intended purpose. The determination of an effective dose is well within the capability of those skilled in the art. The therapeutically effective dose can be estimated initially either in cell culture assays, eg, of neoplastic cells, or in animal models, usually mice, rabbits, dogs, or pigs. The animal model is also used to achieve a desirable concentration range and route of administration. Such information can then be used to determine useful doses and routes for administration in humans. A therapeutically effective dose refers to that amount of protein or its antibodies, antagonists, or inhibitors which ameliorate the symptoms or condition. The exact dosage is chosen by the individual physician in view of the patient to be treated.

Compounds identified according to the methods disclosed herein may be used alone at appropriate dosages defined by routine testing in order to obtain optimal modulation of a potassium channel biological activity and/or physiological condition, or its activity while minimizing any potential toxicity. In addition, co-administration or sequential administration of other agents may be desirable.

The pharmaceutical compositions may be provided to the individual by a variety of routes such as subcutaneous, topical, oral and intramuscular. Administration of pharmaceutical compositions is accomplished orally or parenterally. Methods of parenteral delivery include topical, intra-arterial (directly to the tissue), intramuscular, subcutaneous, intramedullary, intrathecal, intraventricular, intravenous, intraperitoneal, or intranasal administration. The present invention also has the objective of providing suitable topical, oral, systemic and parenteral pharmaceutical formulations for use in the novel methods of treatment of the present invention. The compositions containing compounds identified according to this invention as the active ingredient for use in the modulation of physiological conditions can be administered in a wide variety of therapeutic dosage forms in conventional vehicles for administration. For example, the compounds can be administered in such oral dosage forms as tablets, capsules (each including timed release and sustained release formulations), pills, powders, granules, elixirs, tinctures, solutions, suspensions, syrups and emulsions, or by injection. Likewise, they may also be administered in intravenous (both bolus and infusion), intraperitoneal, subcutaneous, topical with or without occlusion, or intramuscular form, all using forms well known to those of ordinary skill in the pharmaceutical arts. An effective but non-toxic amount of the compound desired can be employed as a potassium channel modulating agent.

The daily dosage of the products may be varied over a wide range from 0.01 to 1,000 mg per adult human/per day. For oral administration, the compositions are preferably provided in the form of scored or unscored tablets containing 0.01, 0.05, 0.1, 0.5, 1.0, 2.5, 5.0, 10.0, 15.0, 25.0, and 50.0 milligrams of the active ingredient for the symptomatic adjustment of the dosage to the patient to be treated. An effective amount of the drug is ordinarily supplied at a dosage level of from about 0.0001 mg/kg to about 100 mg/kg of body weight per day. The range is more particularly from about 0.001 mg/kg to 10 mg/kg of body weight per day. Even more particularly, the range varies from about 0.05 to about 1 mg/kg. Of course the dosage level will vary depending upon the potency of the particular compound. Certain compounds will be more potent than others. In addition, the dosage level will vary depending upon the bioavailability of the compound. The more bioavailable and potent the compound, the less compound will need to be administered through any delivery route, including but not limited to oral delivery. The dosages of the potassium cahennel modulators are adjusted when combined to achieve desired effects. On the other hand, dosages of these various agents may be independently optimized and combined to achieve a synergistic result wherein the pathology is reduced more than it would be if either agent were used alone. Those skilled in the art will employ different formulations for nucleotides than for proteins or their inhibitors. Similarly, delivery of polynucleotides or polypeptides will be specific to particular cells and conditions.

## Example 1

## Identification of Full-Length cDNA

The cDNA SEQ ID NO: 1 was identified from an initial database search for novel KvQT channels. The partial sequence of hKvLQT1, Genbank Accession number U40990, was used in a tblastn search against a proprietary database to identify a homologous nucleic acid sequence which was subsequently used to search a public domain database and identified Merck-WashU clone yn72gl 1 (Genbank accession number H23701). The tissue source for the Merck-WashU clone was a human brain library. Tissue was acquired 17 hours after death from a 55-year-old male who died of a ruptured aortic aneurysm. Starting RNA was made from a pool of brain tissues, including the frontal, parietal, temporal, and occipital cortex from the left and right hemispheres, subcortical white matter, basal ganglia, thalamus, cerebellum, midbrain, pons, and medulla. The cDNA synthesis was initiated using a NotI-oligo(dT) primer. Double-stranded cDNA was size-selected, ligated to EcoRI adaptors (Pharmacia), digested with NotI, and cloned into the NotI and EcoRI sites of a modified pT7T3 vector (Pharmacia). The library was constructed by Bento Soares and went through two rounds of normalization. Soares, B., et al., PNAS, 91:9228 (1994). Full-length information was obtained using 5' and 3' RACE-PCR techniques (rapid amplification of cDNA ends) from the human brain cDNA library. Schaefer, Anal. Biochem., 227: 255 (1995).

## Example 2

## Full-Length Cloning:

The 3' end of the novel cDNAsequence described herein was amplified from 0.25 ng Human Brain, Whole Marathon Ready cDNA™ (Clontech, Palo Alto, CA; Cat.# 7400-1) in a 50 mL reaction using Advantage™ KlenTaq DNA Polymerase Mix (Clontech Cat.# 8417-1) according to manufacturer's recommendations. Marathon Ready CDNA™ is a double stranded, adaptor ligated cDNA used in Rapid Amplification of cDNA Ends (RACE). Primer set sequences are: 5' AACCTCTCGCGCACAGACCTGCA 3' (forward, corresponding to base positions 1058-1080 of SEQ ID NO:1) and 5' CCATCCTAATACGACTCACTATAGGGC 3' (reverse, corresponding to cDNA adaptor, refer to user manual, Clontech Cat# PT1156-1). Cycling paramters were according to user manual, program 1 (briefly, touchdown PCR with 4 min. extensions). A 5 mL aliquot was re-amplified in a 50 mL reaction using Advantage™ KlenTaq DNA Polymerase Mix according to manufacturer's recommendations. Primer sequences are: 5' CTCCACGTGGCAGTACTACGAGC 3' (forward, corresponding to base positions 1081-1103 of SEQ ID NO:1) and 5' ACTCACTATAGGGCTCGAGCGGC 3' (reverse, corresponding to cDNA adaptor, refer to user manual). Cycling parameters were according to user manual, program 1 (briefly, touchdown PCR with 4 min. extensions). An aliquot of the PCR run on a 1% agarose/1xTAE gel, with EtBr 0.5 ug/mL A product of approx. 1.2 kb was cut out and isolated with QIAGEN Gel Extraction Kit (QIAGEN, Santa Clarita, CA; Cat.#28704). The gel eluate was ligated into pT7Blue T-Vector using the Regular pT7BlueT-Vector Kit plus Ligase (Novagen, Madison, WI; Cat.#69836-1) and NovaBlue cells were transformed with ligation reaction according to manufacturer's recommendations. Four colonies were sequenced, and a primer was designed to re-amplify the RACE product from 0.5 ng Human Brain, Whole Marathon Ready cDNA™ and to confirm RACE sequencing data. Advantage™ KlenTaq DNA Polymerase Mix was used in a 50 mL reaction according to manufacturer's recommendations. DMSO was included at a final concentration of 5%. Cycling parameters were according to user manual, program 1 (briefly, touchdown PCR with 4 min. extensions). Primer set sequences are 5' AACCTCTCGCGCACAGACCTGCA 3' (forward, corresponding, base positions 1058-1080 of SEQ ID NO:1) and 5' GACCCAAGGCCGCAGGTCCTCACC 3' (reverse, corresponding to base positions 2998-3030 of SEQ ID NO:1).

SEQ ID NO: 1 specific first strand cDNA was constructed using 5 mg Human Brain Total RNA (Clontech Cat. # 64020-1) and SUPERSCRIPT™ Preamplification System for First Strand cDNA synthesis (Life Technologies, Gaithersburg, MD; Cat.# 18089-011). Manufacturer's protocol 5.1 - First Strand cDNA Synthesis of Transcripts with High GC Content - was followed. Primer sequence used: 5' GACCCAAGGCCGCAGGTCCTCACC 3' (reverse, corresponding to base positions 2998-3030 of SEQ ID NO:1). A 5 mL aliquot was amplified in a 50 mL reaction using Advantage™ KlenTaq DNA Polymerase Mix according to manufacturer's recommendations. DMSO was included at a final concentration of 5%. Cycling parameters were according to user manual, program 1 (briefly, touchdown PCR with 4 min. extensions). Primer sequences:

5'GCCAGGCACCATGGTGCAGAAGTCG 3'(forward, corresponding to base positions 1-25 of SEQ ID NO:1) and 5'CAAAACCTCGGAGGCACCGTGCTG 3' (reverse, corresponding to base positions 2614-2637 of SEQ ID NO:1). The reaction was purified with QIAGEN PCR Purification Kit (QIAGEN Cat.# 28104) and ligated into pCR®II-TOPO vector using TOPO TA Cloning® Kit Dual Promoter (Invitrogen, Carlsbad, CA; Cat.# K4600-01) and TOP 10 cells were transformed with ligation reaction according to manufaturer's recommendations. Four colonies were selected for insert sequencing in both directions using the ABI PRISM™ 377 automated sequencer. SEQ ID NO: results from this coding-region sequencing data and the 3'UTR data from RACE product sequencing.

## Example 3

### Maintanance of Cells and Preparation of RNA for Northern Analyses Isolation of glial cells

Human fetal brain tissues: (16-22-week-old fetuses) were obtained commercially from the Anatomic Gift Foundation (Woodbine, GA). Cerebral hemispheres were placed in chilled, sterile, calcium-free, magnesium-free Hanks Balanced Salt Solution (HBSS, # 14170-112, Life Technologies/Gibco-BRL, Gaithersburg, MD). Meninges were removed with sterile forceps and tissue was dissociated initially by repeated trituration through sterile pipets. Tissue was incubated with 0.05% Trypsin/0.53 mM EDTA (# 25300-054, Life Technologies /Gibco-BRL, Gaithersburg, MD) and 0.15 mg/ml DNAse (# D-5025, Sigma Chemical Co., St. Louis, MO) at 37°C for 45 minutes with gentle shaking. 10% Fetal Bovine Serum (FBS, # 160001-036, Gibco-BRL/Life Technologies, Gaithersburg, MD) was added to the suspension to stop trypsinization. The cells were then passed through 210 μm and 149 μm polypropylene mesh (# 08-670-188 and # 08-670-189, Fisher Scientific, Pittsburgh, PA) sequentially. The filtrate was washed twice and resuspended in complete media [DMEM (with high glucose, L-glutamine and HEPES), Gibco # 12430-054; 100 U-μg/ml penicillin/streptomycin, Gibco # 15070-030; 10% FBS, Gibco # 160001-036]. Cells were plated at density of 80 million/75 $cm^2$ flask (# 12-565-52, Fisher Scientific, Pittsburgh, PA). Cultures were incubated at 37°C, 5% $CO_2$ for 2 weeks (with one media change after one week in culture to remove cellular debris). Mature cultures consisted of astrocytes, neurons, microglia and oligodendrocytes.

Pure astrocyte cultures were achieved by removing complete media (containing floating microglia) and trypsinizing the cultures 3 times which effectively removed neurons, oligodendrocytes and attached microglia. Astrocytes were lysed in Buffer RLT (QIAGEN) with 0.01 % b-ME (Sigma Cat.# M7154) within 24 hours and frozen at -80°C until use.

### IMR-32 cells:

IMR-32 human neuroblastoma (ATCC #CCL-127; Rockville, MD, U.S.A.) cells were cultured in MEM (Specialty Media Cat.# SLM-034-13), supplemented with 10% heat-inactivated FBS (JRH Bioscience Cat. #, 12126-78-H) and 2mM 1-glutamine (Cellgro Cat.# 20-005-LI) at 37°C in a 5% $CO_2$ humidified atmosphere. The medium was changed twice a week. Cells were differentiated with 1mM dibutyryl cAMP (Aldrich Cat.# 85-196-5) and 2.5μM 5-bromodeoxyuridine (Sigma B-9285) to the cultures. Cells were lysed in Buffer RLT (QIAGEN) with 0.01% b-ME (Sigma Cat.# M7154) and frozen at -80°C until use.

Lysed cells were thawed on ice, and processed through a QIAshredder (QIAGEN Cat.# 79654) to homogenize. Total RNA was then prepared with the RNeasy Mini Kit (QIAGEN Cat.#74103) according to manufacturer's directions. Concentration was determined by absorbance at 260 nm.

Human Brain, Human Fetal Brain and Rat Brain Total RNA (Clontech Cat.#s 64020-1,64019-1,64060-1) were precipitated and resuspended for use according to manufacturer's recommendations. Twenty ug each of total RNA from Human Brain, Human Fetal Brain, Rat Brain, Imr-32 (undifferentiated), Imr-32 (differentiated), and Human Fetal Astrocyte along with 5 mg RNA Ladder (Life Technologies Cat.# 15620-016) were run on a 1% agarose/2.2M Formaldehyde/1x MOPS denaturing gel at 50V for 4.5 hr. RNA was transfered overnight to positively charged nylon membrane (Hybond™ N+, Amersham #RPN203B), according to manufacturer's protocol. The RNA was UV crosslinked to the membrane using Stratagene's Stratalinker for 40 sec.

Two multiple-tissue Northern Blots (Cat # 7760-1 and 7759-1) and a blot with RNA from different regions of human brain (Cat # 7755-1) were purchased from Clontech, Inc., each lane contained approximately 2 mg poly A+ RNA.

**Probes Used:**

Probe 1: novel brain derived potassium channel specific: Corresponds to base positions 1257 to 1461 of SEQ ID NO:2 which spans the coding region for the C-terminal tail of the novel poypeptide

Probe 2: "common" probe: Corresponds to base positions 989 to 1109 of SEQ ID NO:2 which spans the coding region for the first 40 amino acids of the C-terminal tail that is common to both KvQT2 and HNSPC.

Probe 1 was generated by amplifying 1 ng Human Brain, Whole Marathon Ready cDNA in a 50 mL reaction using Advantage™ KlenTaq DNA Polymerase Mix according to manufacturer's recommendations. DMSO was included at a final concentration of 5%. Primer set sequences are: 18F and 19R. A 201 bp product was gel isolated and quantified by absorbance at 260 nm. Forty five ng of amplicon was labeled by primer extension with a reverse primer that will yield an antisense ssDNA probe. Final reaction conditions: 50 mL, 1 x reaction buffer A , 5% DMSO, 200 nM 19R, 200 $\mu$M dATP, dTTP, dGTP, 5 U Taq DNA polymerase (Fisher), and 50 mCi a$^{32}$P-dCTP (Dupont NEN). Reaction was incubated at 94°C for 1 min and 72°C for 1 min, repeated for a total of 4 cycles. Labeled product was purified from unincorporated radioactive nucleotide using ProbeQuant™ /G-50 Micro Columns (Pharmacia) according to manufacturer's recommendations.

Probe 2 was generated by amplifying SEQ ID NO:2 in a 50 mL reaction using Advantage™ KlenTaq DNA Polymerase Mix according to manufacturer's recommendations. Primer set sequences are: 5' AGAAGAGGCGGAACCCGGCAGCAG 3' (forward, corresponding to base positions 989-1012 of SEQ ID NO:2) and 5' GGCACGGTGACCGTTCGCTCGTAG 3' (reverse, corresponding to base positions 1084-1109 of SEQ ID NO:2). The reaction was purified with QIAGEN PCR Purification Kit, according to manufacturer's recommendations. The 121 bp amplicon was labeled by primer extension with the reverse primer that will yield an antisense ssDNA probe. Final reaction conditions: 50 mL, 1x reaction buffer A ,100 ng amplicon, 200 nM reverse primer, 200 mM dATP, dTTP, dGTP, 5 U Taq DNA polymerase (Fisher), and 50 mCi a$^{32}$P-dCTP (Dupont NEN). Reaction was incubated at 94°C for 1 min and 72°C for 1 min, repeated for a total of 4 cycles. Labeled product was purified from unincorporated radioactive nucleotide using ProbeQuant™ /G-50 Micro Columns (Pharmacia) according to manufacturer's recommendations.

**Example 4**

**Hybridizations**

Radioactive probes were used to hybridize the two multiple tissue blots the homemade blot andthe Human Brain blot described above. Blots were rinsed in 2xSSC and then prehybridized in NorthernMAX™ Prehybridization/Hybridization Solution at 42°C for 1 hr. The solution was then replaced with the same with the addition of 1-2 x $10^7$ cpm labeled probe, and hybridized overnight at 42°C. The blots were then washed stringently: 2x SSC, 0.5% SDS, 5 min., 2x SSC, 0.1% SDS, 5 min., 0.1x SSC/0.5% SDS 30 min., 37°C, twice and briefly with 0.1 x SSC. Blots were then exposed between 24 and 48 hours to Biomax MS-1 imaging film (Kodak) at -80°C with two intensifying screens.

**Example 5**

**Generation of Expression Construct**

SEQ ID NO: 1 cDNA was excised from the pCR®II-TOPO vector (*supra*) using Eco RI sites and subcloned into pCDNA3 mammalian vector (Invitrogen). For expression in *Xenopus* oocytes (Timpe, et al., Nature, 331:143 (1988)), the cDNA was cut from pCR®II-TOPO vector using EcoRV and Hind III sites and subcloned into pKGEM vector (a modified PGEM vector from Promega containing Xenopus b-globin 5' and 3' UTR sequences flanking the multiple cloning site to increase message stablility). These constructs can be used to obtain functional and biophysical and pharmacological profiles of the novel brain-derived potassium channel using standard patch-clamp and/or oocyte techniques. The novel neuron-specific K+ channel can readily be expressed in mammalian cells (e.g., HEK 293, Cos, CHO, RBL-1, etc.)

by transient or stable transfection, or by microinjection of RNA into Xenopus oocytes for thorough biophysical and pharmacological characterization using standard patch-clamp (Hammill, O. et al., Pfugers Arch. 391: 85 (1981) or two-electrode voltage clamp techniques (Soreq, H. and Seidman., S. Methods Enzymol., 207:225 (1992); Goldstein, A., et al., PNAS, 83:7503 (1986).

**Example 6**

**Baculoviral Overexpression System for Screening Preparation**

(Generation of recombinant baculoviral expression vectors and gene expression with the BAC-TO-BAC Expression System, Life Technologies, Gaithersburg, MD Cat. # 10359-016 BAC to BAC Baculovirus Expression System)

SEQ ID NO:2 is cloned into pFASTBAC1, and the recombinant plasmid is transformed into DH10BAC competent cells which contain the bacmid with a mini-attTn7 target site and the helper plasmid. The mini-Tn7 element on the pFASTBAC1 plasmid can transpose to the mini-attTn7 target site on the bacmid in the presence of transposition proteins provided by the helper plasmid. Colonies containing recombinant bacmids are identified by disruption of the lacZα gene. High molecular weight mini-prep DNA is prepared from selected E. coli clones containing the recombinant bacmid, and this DNA is then used to transfect insect cells.

**Cloning Into pFASTBAC1**

The donor vector, pFASTBAC1, contains the polyhedrin promoter followed by an extensive MCS which extends from BamH I (4032) to Hind III (4137). The BamH I site is 37 bp downstream from the original ATG of the polyhedrin gene, which has been mutated to ATT. Therefore, to successfully express a foreign protein, the foreign DNA fragment must contain its own ATG followed by an open reading frame (ORF). The foreign DNA fragment must be cloned into pFASTBAC1 in the correct orientation with respect to the polyhedrin promoter (i.e., the 5' end of the gene must be inserted into the first selected site of the MCS).

Prepare pFASTBAC1 and the foreign DNA fragment by digesting 500 ng to 1 μg DNA with the selected restriction endonuclease(s) under the appropriate conditions. If only one site in the vector is chosen as the cloning site, dephosphorylate the vector under the appropriate conditions. DNA fragments can be purified by agarose gel electrophoresis and the fragments of interest can be recovered from the gel by using a GLASSMAX® cartridge or an equivalent purification. Ligate the prepared vector and insert fragments under the appropriate conditions.

**Cloning**

For this initial cloning, do not use the DH10BAC cells in the system. DH5α or DH10B competent cells can be used. Plate the transformation mix onto LB agar plates containing 100 μg/ml ampicillin.

For analysis of a directional cloning experiment, 6 colonies are sufficient to screen; 12 or more may need to be analyzed for a nondirectional cloning strategy. Prepare plasmid DNA from overnight cultures using a mini-preparation procedure (e.g., Schecter, A.I., et al., Nature 312:513 (1984) and verify correct insertion of the gene of interest by restriction endonuclease digestion or PCR analysis.

**Transposition**

1. Prepare Luria Agar plates containing:

    50 μg/ml kanamycin,
    7 μg/ml gentamycin;
    10 μg/ml tetracycline;
    300 μg/ml Bluo-gal and
    40 μg/ml IPTG.
    See additional protocols for formulations (Sections 5.1 and 5.2)

2. Thaw the DH10BAC competent cells on ice.
3. Dispense 100 μl of the cells into 15-ml polypropylene tubes.
4. Add approximately 1 ng recombinant donor plasmid (in 5 μl) and gently mix the DNA into the cells by tapping the side of the tube/

5. Incubate the mixture on ice for 30 min.
6. Heat shock the mixture by transferring to 42˚C water bath for 45 s.
7. Chill the mixture on ice for 2 min.
8. Add 900 μl S.O.C. medium to the mixture.
9. Place the mixture in a shaking incubator at 37˚C with medium agitation for 4 h.
10. Serially dilute the cells, using S.O.C. medium, to $10^{-1}$, $10^{-2}$, $10^{-3}$, (i.e., 100 μl of transposition mix: 900 μl of S.O.C. medium = $10^{-1}$ dilution, use this to further dilute 10-fold to give $10^{-2}$ dilution, and similarly for $10^{-3}$ dilution).
11. Place 100 μl of each dilution on the plates and spread evenly over the surface.
12. Incubate for at least 24 h at 37˚C (Blue colonies may not be visible prior to 24 h).

**Isolation of Recombinant DNA**

White colonies contain the recombinant bacmid, and therefore, are selected for isolation of recombinant bacmid DNA. Before isolating DNA, candidate colonies are streaked to ensure they are truly white.

1. Select white colonies from a plate with approximately 100 to 200 colonies. NOTE: This number facilitates differentiation between blue and white colonies.
2. Pick - 10 white candidates and streak to fresh plates to verify the phenotype. Incubate overnight at 37˚C.
3. From a single colony confirmed as having a white phenotype on plates containing Bluo-gal and IPTG, set up a liquid culture for isolation of recombinant bacmid DNA.

The following protocol is specifically developed for isolating large plasmids (> 100 kb), and is adapted for isolating bacmid DNA.

1. Using a sterile toothpick, inoculate a single, isolated bacterial colony into 2 ml LB medium supplemented with 50 μg/ml kanamycin, 7 μg/ml gentamicin, and 10 μg/ml tetracycline. A 15-ml snap-cap polypropylene tube is suitable. Grow at 37˚C to stationary phase (up to 16 h) shaking at 250 to 300 rpm.
2. Transfer 1 ml of culture to a 1.5-ml microcentrifuge tube and centrifuge at 14,000 x g for 1 min.
3. Remove the supernatant by vacuum aspiration and resuspend (by gently vortexing, or pipetting up and down, if necessary) each pellet in 0.3 ml of Solution I [15 mM Tris-HCl (pH 8.0), 10 mM EDTA, 100 μg/ml Rnase A]. Add 0.3 ml of Solution II (0.2 N NaOH, 1% SDS) and gently mix. Incubate at room temperature for 5 min. Note: The appearance of the suspension should change from very turbid to almost translucent.
4. Slowly add 0.3 ml of 3 M potassium acetate (pH 5.5), mixing gently during addition. A thick white precipitate of protein and genomic *E. coli* DNA will form. Place the sample on ice for 5 to 10 min.
5. Centrifuge for 10 min. at 14,000 x g. During the centrifugation, label another microcentrifuge tube and add 0.8 ml absolute isopropanol to it.
6. Gently transfer the supernatant to the tube containing isopropanol. Avoid any white precipitate material. Mix by gently inverting tube a few times and place on ice for 5 to 10 min.
At this stage, the sample can be stored at -20˚C overnight.
7. Centrifuge the sample for 15 min. at 14,000 x g at room temperature.
8. Remove the supernatant and add 0.5 ml 70% ethanol to each tube. Invert the tube several times to wash the pellet. Centrifuge for 5 min. at 14,000 x g at room temperature. (Optional: repeat step 8.)
9. Remove as much of the supernatant as possible. Note: The pellet may become dislodged from the bottom of the tube, so it is better to carefully aspirate the supernatant than to pour it.
10. Air dry the pellet briefly, 5 to 10 min., at room temperature and dissolve the DNA in 40 μl TE. Allow the solution to sit in the tube with occasional gentle tapping of the bottom of the tube. The DNA is generally ready for use within 10 min., as long as the pellets are not overdried.
11. Store the DNA at -20˚C. However, avoid repeated freeze/thaw cycles to avoid a drastic reduction in transfection efficiency.

Preparations of bacmid DNA may be analyzed by agarose gel electrophoresis to confirm the presence of high molecular weight DNA.

**Transfection of Sf9 Cells with Recombinant Bacmid DNA**

1. Seed 9 x $10^5$ cells per 35-mm well (of a 6-well plate) in 2 ml of Sf-900 II SFM containing penicillin/streptomycin at 0.5X final concentration (50 units/ml penicillin, 50 up/ml streptomycin).
2. Allow cells to attach at 27˚C for at least 1 h.
3. Prepare the following solutions in 12- x 75-mm sterile tubes:

   Solution A; For each transfection, dilute ~5 up of mini-prep bacmid DNA into 100 µl Sf-900 II SFM without antibiotics.

   Solution B; For each transfection, dilute ~6 ul $_{CELL}$FECTIN Reagent into 100 µl Sf-900 II SFM without antibiotics.

4. Combine the two solutions, mix gently, and incubate for 15 to 45 min. at room temperature.
5. Wash the cells once with 2 ml of Sf-900 II SFM without antibiotics.
6. For each transfection, add 0.8 ml of Sf-900 II SFM to each tube containing the lipid-DNA complexes. Mix gently. Aspirate wash media from cells and overlay 1 ml of the diluted lipid-DNA complexes onto the cells.
7. Incubate cells for 5 h in a 27˚C incubator.
8. Remove the transfection mixtures and add 2 ml of Sf-900 II SFM containing antibiotics. Incubate cells in a 27˚C incubator for 48 h.
9. Assay cells for protein activity 48 to 72 h after the start of transfection; harvest virus at 72 h.

**Harvest/Storage of Recombinant Baculovirus**

1. When harvesting virus from the transfection, transfer the supernatant (2 ml) to a sterile, capped tube. Clarify by centrifugation for 5 min. at 500 x g and transfer the virus-containing supernant to a fresh tube.
2. From the initial transfection, viral titers of 2 x $10^7$ to 4 x $10^7$ pfu/ml can be expected.
3. Store the virus at 4˚C, protected from light. For long term storage of virus, the addition of fetal bovine serum (FBS) to a final concentration of at least 2% FBS is recommended. Storage of an aliquot of the viral stock at -70˚C is also recommended.
4. For determining the viral titer, a plaque assay can be performed. See Section 5.12, *viral Plaque Assay,* for plaquing procedures.
5. For amplifying viral stocks, infect a suspension or monolayer culture at a Multiplicity of Infection (MOI) of 0.01 to 0.1. Use the following formula:

$$\text{Innoculum required (ml):} \quad \frac{\text{desired MOI (pfu/ml) x (total number of cells)}}{\text{titer of virual innoculum (pfu/ml)}}$$

For example, infect a 50-ml culture at 2 x $10^6$ cells/ml with 0.5 ml of a viral stock that is 2 x $10^7$ pfu/ml, for an MOI of 0.1.

Harvest virus at 48 h post-infection. This will result in approximately 100-fold amplification of the virus.

**Infection of insect cells with recombinant baculovirus particles**

Optimal infection conditions for insect cells can vary. A starting point for infection is an MOI of 5 to 10. For more information, please refer to reference 2. It is recommended that several experiments be performed for each protein to be expressed.

***MOI optimization:*** Infect a population of cells at varying MOIs (e.g., 1, 2, 5, 10) and assay protein expression upon harvesting the cells (or media, if the protein is secreted).

***Time course:*** Infect cells at a constant MOI. Harvest cells (or media) at the following time intervals: 24 h, 48 h, 72 h, and 96 h. Assay for expression.

**Preparing the cells for transfection**

1. Twenty-four hours before transfection, inoculate a 60-mm tissue culture dish with 4 x $10^5$-11 x $10^5$ exponentially growing cells. The cells should be 60-80% confluent at the time of transfection.

2. Grow the cells overnight in 5 ml of the appropriate culture medium.

**Preparing the complex formation solution**

1. Transfer 900 μl of sterile serum-free, antibiotic-free DMEM (DMEM-SA) to a polystyrene tube.
2. Add 35-100 μl of LipoTAXI transfection reagent. Tap the side of the tube to mix.
3. Add 7 μg of the control plasmid to the control reaction and 5-10 μg of the experimental DNA to the experimental reaction. For stable transfections, prepare a negative control.
4. Mix gently (do not vortex) and incubate for 15-30 minutes at room temperature.

**Adding the activated solution**

1. Remove the standard culture medium from the tissue culture dish by aspiration.
2. Add 1.5 ml of DMEM (serum optional) to the transfection mixture in the polystyrene tube and then transfer this entire mixture (~2.5 ml) dropwise to the tissue culture dish while swirling the dish.
3. Incubate for 4-6 hours using standard growth conditions (i.e., 37˚C and 5% $CO_2$ in a humidified incubator).
4. Add 2.5 ml of DMEM containing serum (DMEM+S) at twice the normal serum concentration to the tissue culture dish and incubate overnight.
5. Replace the medium with 5.0 ml of fresh, complete medium.
6. Incubate the cells for 24-72 hours depending on the cell type, reporter system, and promoter activity or proceed to *Performing a Stable Transfection.*

***Performing a Stable Transfection***

1. Split the cells from step 6 of Adding the Activated Solution to the desired ratio (at least 1:10) after the 24-hour incubation and then incubate overnight.
2. Apply selection antibiotics dropwise to the tissue culture dish, swirling the dish between drops, at a concentration appropriate to the cell line.
3. Replace the medium and apply fresh selection antibiotics every 4-7 days (approximately two times per week).
4. Stable colonies form within 1-2 weeks. Cells from the negative DNA control dish die off.

**Transfecting Suspension Cells with the LipoTAXI Mammalian Transfection Kit**

***Preparing the Cells for Transfection***

1. Seed 4 x $10^6$-10 x $10^6$ cells per 60-mm dish in 700 μl of DMEM-SA.

***Note** The optimal ratio of LipoTAXI transfection reagent to DNA must be determined for each plasmid and cell line.*

***Preparing the Complex Formation Solution***

1. Transfer 900 μl of DMEM-SA to a polystyrene tube.
2. Add 35-100 μl of LipoTAXI transfection reagent. Tap the side of the tube to mix.
3. Add 10 μg of the control plasmid to the control reaction and 7-15 μg of the experimental DNA to the experimental reaction. For stable transfections, prepare a negative control.

4. Mix gently (do not vortex) and incubate for 15-30 minutes at room temperature.

***Adding the Activated Solution***

1. Add 800 μl of DMEM (serum optional) to the transfection mixture in the polystyrene tube and then transfer this entire mixture (~1.8 ml) dropwise to the tissue culture dish while swirling the dish.
2. Incubate for 4-6 hours using standard growth conditions (i.e., 37˚C and 5% $CO_2$ in a humidified incubator).
3. Add 3 ml of fresh, complete DMEM to the tissue culture dish.
4. Incubate the cells for 24-72 hours depending on the cell type, reporter system, and promoter activity or proceed to step 10 if performing a stable transfection.

***Performing a Stable Transfection***

1. After 48 hours, seed a fresh tissue culture dish to be used for selection at no greater than one-third the density of the transfected cells from step 9.
2. Apply selection antibiotics dropwise to the tissue culture dish, swirling the dish between drops, at a concentration appropriate to the cell line.
3. Replace the medium and apply fresh selection antibiotics every 4-7 days (approximately two times per week).
4. Stable colonies form within 1-2 weeks. Cells from the negative DNA control dish die off.

**Harvesting The Transfected Suspended Cells**

1. Transfer the transfected cells into 15-ml centrifuge tubes and spin for 5 minutes at 200 x g to pellet the cells.
2. Remove and discard the supernatant, add 1 ml of PBS to the cell pellet, and transfer the resulting cell suspension to a 1.7-ml microcentrifuge tube.
3. Spin the tube in a microcentrifuge at 200 x g for 5 minutes to pellet the cells.
4. Remove and discard the supernatant, add 1 ml of lysis buffer to the cell pellet, and place at -20˚C until frozen.
5. Thaw the cell extract and spin the tube in a microcentrifuge at 12,000 x g for 5 minutes to pellet the cell debris.
6. Transfer the supernatant to a new microcentrifuge tube and store at -20˚C or proceed to *B-Galactosidase Assay.*

**Example 7**

**$CaPO_4$ Transfection Protocol**

The following protocol is optimized for 100-mm culture dishes and all volumes are for single transfection.

1. Inoculate 100-mm culture dishes with exponentially growing cells at 5 x $10^5$ cells per dish no more than 24 hours before the transfection. These cells should be grown overnight in 10 ml of the appropriate medium to a confluency of approximately 10-20%. The RPMI series of media cannot be used for $CaPO_4$ transfection. The excess positive charge in this media will cause a dense precipitate to form.
Preparation of the DNA Precipitate Solution (per 100-mm culture dish)
*NOTE: The optimal amount of DNA will vary depending on the cell type being transfected For the control plasmid pWLneo* (Life Technologies, Gaithersburg, MD), *use 10 μg/dish, (10-30 μg of circular DNA/dish is recommended in most cases).*
2. Dilute desired amount of DNA with distilled, deionized water to 450 μl.
3. Allow mixture to incubate 10-20 minutes at room temperature.
4. Gently mix the solution to ensure adequate suspension. Add this to the culture dropwise and swirl plate gently to distribute evenly.
5. Incubate cells for 12-24 hours. Depending upon cell type, optimal incubation time may vary. A fine precipitate will be seen after incubation.
***NOTES:*** *If spare incubator space is available, transfection efficiency can be improved 2-3 times by using lower $CO_2$ concentrations (2-4% $CO_2$).*

*Return to normal CO2 concentrations after precipitation removal in the following step (step 6).*
6. After 12-24 hours incubation, remove medium and rinse culture twice using PBS (without Ca or Mg), or medium without serum. Apply fresh, complete medium (serum-containing), and allow the cells to incubate 24 hours under a $CO_2$ concentration optimal for the cell line.
7. Split cells at desired rations (at least 1:10) and incubate an additional 24 hours before applying selection of stable transfectants.

## Example 8

## DEAE-Dextran Transfection Protocol

The following protocol has been optimized for 100-mm culture dishes and all volumes are for a single transfection. For the control plasmid pSV2 Cat (Life Technologies, Gaithersburg, MD) use 2 μg/100 mm dish.

## DEAE-Dextran Transfection (per 100-mm Culture Dish)

1. Exponentially growing cells should be seeded at a density which will yield -100% confluency within 72 hours. Transfection should be performed between 6-24 hours after seeding.
2. Mix DNA (1-2 μg/100-mm dish) with phosphate buffered saline (1xPBS) to a final volume of 170 μl.
3. Dilute 85 μl of Solution #3 with 85 μl of 1xPBS.
4. Combine the PBS-DNA mixture with the Solution #3-PBS mixture.
5. Remove media from cultures and rinse twice with PBS.
6. Add the DNA mixture (from step 4) dropwise to the center of the culture and swirl gently to distribute the solution evenly. Incubate for 15 minutes at room temperature (-25°C) without $CO_2$.
7. Remove the solution and *gently* rinse the culture with PBS (cells will be poorly attached at this point).
8. Add 10 ml of fresh, complete media to the culture and allow to grow under optimal culture conditions.

## Selection

When using the G418 antibiotic selection method, it is important to remember that no all mammalian cell lines are equally sensitive to G418. The minimal lethal concentration can range from 100 μg/ml to 1 mg/ml. Therefore, the concentration to be used for selection must be determined for *each* cell line *before* the experimental can begin.
Since many cell lines have already been subjected to this type of selection, it may be useful to consult the available literature. If no information about the sensitivity of a particular cell line is available, a simple way to determine its sensitivity is to grow cultures in a multiwell late with a range of G418 concentrations between the individual wells. The optimal concentration is the lowest one that kills *all* of the cells within 10-14 days. (Rapidly dividing cells may be killed more readily since the antibiotic appears to act mainly on dividing cells.)
In some cases, it may be possible to reduce the concentration of G418 after the initial selection and still maintain the selectable marker gene. For example, NIH 3T3 cells are generally selected in 400 μg/ml G418 and the presence of the neo gene can be maintained in 250 μg/ml.

## Clone Isolation

After the selection process progresses to the point where colonies are visible, it is important to isolate the colonies in a manner that obtains the maximum number of cells (increasing the likelihood the clone will survive), and to minimize the changes of contaminating one colony with cells from another. To help achieve both these goals, it is recommend use cloning rings when isolating colonies. The rings may be made of any autoclavable material, and have an internal diameter of 5-10 mm. Sterilize them along with a pair of medium forceps and some form of adhesive (vacuum grease works well).
Before isolating the colonies, prepare 24-well microtiter dishes to receive the new clones. The volume used for the new clones should be kept to a minimum, since many types of cells appear to require the support of other cells of the same type in order to grow in culture. This support may involve some kind of soluble growth factor which may become ineffective if diluted too much. In cases where cell growth is initially very sparse (<5% of the dish surface), it is often helpful to add growth factors at higher concentrations than usual.
***NOTE:*** *For example, if a cell type grows wells in media supplemented with 10% fetal calf serum under normal culture conditions, it may require 20% fetal calf serum to grow well when the cells are seeded at a very low density. It may*

*also be useful to remove media from a more densely populated culture and, after sterile filtering, use it to supplement the growth of medium of the sparsely populated cultures.*

To isolate single colonies using cloning rings, the culture containing the colonies should be washed twice in PBS, then all fluid should be removed from the plate. The rings should be handled only with sterile forceps.

1. Dip the rings in the sterile adhesive and lightly dab them against a dry, sterile surface to spread the adhesive evenly around the outer edges.
2. Apply the rings around the colonies to be harvested (work quickly to prevent drying out).
3. Add 4-5 drops of trypsin to each ring with a sterile, cotton-plugged Pasteur pipet.
4. Wait ~30 seconds per colony, then break up the colony by pipetting the trypsin up and down 2-3 times.
5. Transfer the trypsinized cells to a 24-well plate.
6. Remove some of the media from the 24-well late, rinse the inside of the ring to remove any residual cells and transfer to the dish.
7. After ~ 2 hours, examine the plate with a microscope to determine if the cells have attached. If they have attached, replace the media in the 24-well plate with fresh, complete media in order to remove any remaining trypsin.

During the early stages of new clone growth, it is best to change the media as infrequently as possible (about once a week for very sparsely populated cultures), which helps maintain any soluble factors. It is best to add exogenous growth factors to the media, instead of replacing the old media with media containing fresh growth factors.

Once the small cultures have grown to confluency, they may be passed to larger culture vessels (usually 6-well microtiter plates first) and treated the same way as other cells of the same type (the original selection should be maintained throughout the life of the new clone).

## Example 9

### Membrane Preparation of Transfected HEK Cells for Screening Compounds

Membranes are prepared when the HEK cells in each T-150 (or T-175 or T-225) flask are confluent.

The preparation of membranes from each flask of cells is as follows:

1. Pour off the cell culture media and save (since it always contains some cells that have just been dislodged from handling the flask).
2. Rinse the confluent layer of cells with about 8 mls of phosphate buffered saline (PBS), 37˚C. This removes residual fetal calf serum which can inhibit the trypsin used in step 3. Save the PBS from the rinse, too, to capture any other cells that are dislodged.
3. Add about 5 ml of trypsin-EDTA, 37˚C. Slowly rock the flask back and forth to let the trypsin wash over the whole layer of cells. In about 30 sec, some of the cells will come loose; tap the flask firmly on the lab bench to dislodge the rest of the cells. Quickly add fresh medium to the flash to stop the trypsin reaction. Add about 10ml medium for every 5ml of trypsin. Pipet the trypsin/cells/medium into a centrifuge tube. Rinse the flask with about 5 ml more medium and combine with other tubes.
4. Wash the cells by spinning at 250xg, 8 min. Resuspend the individual cell pellets in a small volume of assay buffer, and combine them into one tube.
5. Homogenize the cells using a Polytron, 25 sec., 13000 RPM
6. LOW SPEED SPIN: 800 RPM (~100xg) 2 min.; save the supernatant; discard the pellet
7. HIGH SPEED SPIN: transfer the sup into high speed tubes, and spin 20,000 RPM (50,000xg), 10 min. Discard the supernatant; add 1 ml of assay buffer on top of pellet
8. Freeze on dry ice; cap the tubes and store at -80 C.

Dilute the membranes to 50ug/ml, and use 2.5ug in each well (50ul of 50ug/ml)

## Example 10

### Radiolabelled Ligand Competitive Binding in Membranes from Transfected HEK cells 96 well microtiter plate method; separation of bound and free by filtration over GF/C filters

Radiolabelled ligands for use in this example include but are not limited to tetraethylammonium chloride (TEA), 4-aminopyridine (4-AP, as well as 2-AP and 3-AP), 3,4-and 2,3-diaminopyridine, $BaCl_2$, CsCl, strychnine, phencyclidine, pyridostigmine, 9-aminoacridine, DuP-996 (3,3-bis (4-pyridinylmethyl)-1-phenylindolin-2-one; linopiridine), clofilium, quinidine, aminoquinolines and quinine.

| Assay Buffer: | Wash Buffer: |
|---|---|
| 5mM NaCl<br>5mM KCl<br>10mM HEPES<br>6mM glucose<br>bring to pH 8.4 with TRIS base | 200mM NaCl<br>20mM HEPES<br>pH 8.0 with Tris base |

membranes: transfected HEK cell membranes
Use 2.5ug protein per well in the assay

misc. 96 well microtiter plates, Falcon, round bottom, non tissue culture treated
96 well Unifilters, GF/C, Packard Instrument Co.
Microscint-20 scintillation fluor, Packard
Top Count scintillation counter, Packard
Polyethylenimine, 0.6% for soaking Unifilters
Radiolabelled ligand, e.g. TEA, 2200Ci/mmol
nonspecific binding defined with 10nM cold Ligand (e.g., TEA)

Assay: 130ul assay buffer
20ul test compound or control or nonspecific
50ul membranes at 50ug/ml (2.5ug protein added to each well)
50ul Radioligand [final] 25 pM
250ul total volume

Assay Method:

Place 130ul per well in 96well plate
Add 20ul of candidate compound (drug), or control solution (more buffer or test compound diluent), or nonspecific (defined with 10nM cold Ligand)
Add the previously prepared membranes to the assay plate, and pre-incubate membranes, plus buffer, test compounds, etc. for 5 min, 21˚C, mixing on a Titer-Tek plate mixer
Add 50ul Radioligand, [final] 25pM, and incubate for 20 min. 21˚C, mixing on the plate mixer
Separate bound from free Radioligand by filtering over the presoaked GF/C Unifilters;
wash twice rapidly using ice cold wash buffer
Let Unifilter plates dry overnight
When dry, add 25ul Microscint-20 to Unifilters, and count in Top Count (Packard) scintillation counter

**Example 11**

**Radioligand Binding Assays**

Radiolabelled ligands for use in this example include but are not limited to tetraethylammonium chloride (TEA), 4-aminopyridine (4-AP, as well as 2-AP and 3-AP), 3,4- and 2,3-diaminopyridine, $BaCl_2$, CsCl, strychnine, phencyclidine, pyridostigmine, 9-aminoacridine, DuP-996 (3,3-bis (4-pyridinylmethyl)-1-phenylindolin-2-one; linopiridine), clofilium, quinidine, aminoquinolines and quinine.

Neurons (commercially available for culture (and for transfection and transformation), as well as postmitotic human CNS cells, for instance, from STRATAGENE, La Jolla, CA) are incubated in 12 x 75-mm polystyrene tubes with a radioligand (~1-2 x $10^3$ Ci/mmol). Unless otherwise noted, cells are suspended in isotonic sucrose medium (Medium I) containing 10 mM NaHepes, 5mM KCl, 5 mM NaCl, and 6 mM glucose, pH 8.4, and incubated with the radioligand for 1 h at room temperature on a rotary shaker. Nonspecific binding is determined in the presence of 10 nM native cold radioligand. Stock cell suspensions are diluted to give a final cell concentration of 2.5 x $10^5$ cells/ml in a total volume of 400 μl. At the end of the incubation period, samples are diluted with 4 ml of ice-cold Quench solution, which contain 200 mM NaCl, 20 mM Hepes (free acid), titrated to pH 8.0 with Trisbase. Quenched samples are filtered through GF/C glass microfiber filters, that had been presoaked in 0.6% polyethylenimine, and washed twice with ice-cold Quench solution. Triplicate samples are run for each experimental point. Standard deviation of the mean is typically less than 5%. Different cell preparations may produce somewhat different ratios of nonspecific/total radioligand binding. Stock solutions of the radioligand are prepared in 100 mM NaCl, 20 mM Tris-HCl, pH 7.4, 0.1% bovine serum albumin.

*Analysis of Data*-Data from saturation experiments are subjected to Scatchard analysis, and linear regression is performed to yield to equilibrium dissociation constant ($K_4$) and maximum receptor concentration ($B_{max}$). Correlation coefficients for these determinations are typically greater than 0.95. Data from competition experiments are analyzed by the standard method of Cheng and Prusoff to determine $K_i$ values. The dissociation rate constant ($\kappa_1$) is determined directly from a first order plot of ligand dissociation *versus* time. The rate of ligand association ($k_1$) is determined from the equation $k_1$ -$k_{obe}$($[LR]_e$/([L][LR]$_{max}$)), where [L] is the concentration of ligand, $[LR]_e$ is the concentration of the complex at equilibrium, $[LR]_{max}$ is the maximum number of receptors present, and $k_{obe}$ is the slope of the pseudo-first order plot In ($[LR]_e$/{$[L]_e$- $[LR]_t$}] *versus* time. Association and dissociation rate of the radioligand are also determined by measuring the kinetics of radiolabeled entity binding at different ligand concentrations, determining $k_{obe}$ at each concentration of ligand from semilogarithmic representations of these data, and determining $k_1$ and $k_{-1}$ from the slope and y intercept, respectively, of the plot of $k_{obe}$ *versus* ligand concentration.

The Radioligand Binding Assays may be used with the present invention substanstially as described by Deutsch, C., et al., J. of Biological Chemistry, 266: No. 6, 3668-3674 (1991).

## Example 12

### $^{86}$Rb efflux Drug Screening Assay

Depolarization of human neuroblastoma cells by high concentrations of extracellular potassium ions, leads to the activation of the voltage-gated potassium channels. The activity of such potassium channels is demonstrated to be effectively and rapidly monitored by tracking the efflux of $^{86}$Rb from pre-loaded target cells in response to the depolarizing stimulus. The inclusion of compounds with unknown activity in the assay medium, results in the identification of novel blockers of the potassium channel described herein. *See,* Toral, J., et al., Use of Cultured Human Neuroblastoma Cells in Rapid Discovery of the Voltage-gated Potassium-channel Blockers, J. Pharm. Pharmacol., 46:731(1994). Blocking of individual K+ channels by a candidate compound results in a significant decrease in $^{86}$Rb efflux which can be readily detected by this assay. Toral, J., *et* al. have successfully used this assay to discover a number of novel chemical structures capable of blocking the voltage-gated potassium channels in neurons and cardiocytes. The potassium-channel blocking activity of these compounds has been verified by electrophysiological techniques, as well as by $^{86}$Rb efflux from cultured mammalian cells transfected with cloned neuronal voltage-gated potassium channels.

The functional high-volume $^{86}$Rb efflux assay is performed in 96-well microtitre plates, it represents a rapid and high-volume primary screening method for the detection and identification of potassium-channel blockers. The application is described for detecting blockers of the potassium channel in cultured human neuroblastoma cells. Toral, J., et al., Use of Cultured Human Neuroblastoma Cells in Rapid Discovery of the Voltage-gated Potassium-channel Blockers, J. Pharm. Pharmacol., 46:731(1994).

Highly purified human NT2 neuron cells and hNT post mitotic cenral nervous system cells for differentiation toward neuronal phenotype, are available from STRATAGENE, La Jolla, CA, for transfection to allow the study of potassium channel genes and assays described herein.

*Buffers and Reagents*

### Buffer

MOPS-PSS, pH 7.4 (NaCl 120 mM; KCl 7.0 mM; $CaCl_2$ 2.0 mM; $MgCl_2$ 1.0 mM; ouabain 10μM; 4-morpholinepropanesulphonic acid, MOPS 20mM).

### Depolarizing Solution

MOPS-PSS containing KCl (80mM) replacing the equivalent concentrations of NaCl.

Candidate Compounds are dissolved at a stock concentration of 10-100 mM, either in MOPS-PSS or dimethylsulphoxide (DMSO), and are subsequently diluted in the incubation buffer to the desired concentration. Candidate compounds are dissolved in MOPS-PSS containing bovine serum albumin (0.1@ w/v) at 50-500μM stock concentration.

### Cell Culture and $^{86}$Rb loading

Human neuroblastoma cells TE671 are obtained from American Type Culture Collection (HTB 139) and are maintained at 37°C in Dulbecco's Modified Eagle's Medium (DMEM) supplemented with 10% foetal bovine serum, 4.5

$gL^{-1}$ glucose and 2.0 mM L-glutamine. Cells are plated and loaded with $^{86}Rb$ in 96-well microtitre plates as described by Daniel, S., et al., J. Pharmacol. Methods, 25:185 (1991).

**$^{86}$Rb efflux Assay Procedure**

The growth medium in the microtitre plate is discarded by a sharp flicking of the plate. The adherent cell layer is washed three times with 200μL MOPS-PSS using a 12-channel pipetter. The cells are incubated for 30 min at room temperature, either with 200 μL MOPS-PSS, or 20 μL of the depolarizing solutions, in the presence or absence of a candidate compound potassium-channel blocker. Supernatant (150 μL) from each well is removed and counted. Cell layer is solubilized in 200 μL 0.1 % Tween 20 in water and 150 μL is also counted in a Packard 2200 CA liquid scintillation counter. All supernatants are counted in 7.0 mL distilled water.

The percent efflux is calculated as follows:

$$\%\ \text{total efflux} = \frac{(\text{counts min}^{-1}\ \text{in supernatant})}{(\text{counts min}^{-1}\ \text{in supernatant} + \text{counts min}^{-1}\ \text{in cell extract})} \times 100$$

and the value of percent net efflux is calculated as:

$$\%\ \text{net efflux} = \%\ \text{total efflux} - \%\ \text{basal efflux}$$

where % total efflux is that induced by the depolarizing solution containing 100 mM KCl. The basal efflux is the efflux (leak) of $^{86}Rb$ observed in the physiological saline, MOPS-PSS.

**Example 13**

**FLIPR™ Optical Screening System**

**Molecular Devices Corporation, Sunnyvale, CA**

FLIPR (FLuorescent Imaging Plate Reader) was initially designed to perform as a large-volume screening tool for measurements of membrane potential of cells in 96 well microtiter plates. FLIPR is applied to fluorescent assays, such as in the measurement of particular intra-cellular ions and intracellular pH. FLIPR may also be used in non-fluorescent assays.

In fluorescent mode, FLIPR works by illuminating the bottom of a 96 well microplate and measuring the fluorescence from all 96 wells simultaneously using a cooled CCD camera. The excitation optics in FLIPR illuminate the plate from the bottom and the emission optics read the plate from the bottom. This requires that the microplate that is optically measured in the machine has a clear bottom.

The typical measurement assay consists of using one 96 well plate with cells and one (or two) 96 well plate with the candidate compounds to be screened.

The detection optics of FLIPR are based on cooled CCD (charge coupled device) technology. With each kinetic update, the system takes a picture of the bottom of a microplate, recording a signal for all the individual wells simultaneously. Enhanced sensitivity for cell-based assays is accomplished via optical detection, which allows for signal isolation on a cell mono-layer. This technique is very important for assays where background fluorescence is present (such as from extra-cellular dye). In order to make FLIPR an effective screening tool, the instrument includes an accurate 96 well pipettor which can aspirate and dispense from two separate fluid addition microplates into a third microplate being optically tested. The entire system is controlled via a PC and Windows-based software interface. The interface provides flexible tools for assay development, quality control and data management.

Measurements are recorded for both voltage gated and ligand gated calcium channels on many different cell lines including CHO, HEK, A10, ECV50, primarily cultured neuronal cells, and others. Visible wavelength indicators, namely Fluo-3 and Calcium Green-1 are redily used.

| Description | Suggested Supplier | Phone Number | Item Number |
|---|---|---|---|
| Black wall plates, clear bottom, tissue culture treated, sterile | Corning/Costar | 800-492-1110 | 3603 |
| | Packard Instrument | 800-856-0734<br>203-639-2404 | 6005182 |
| Nunc V-bottom 96 well plate | Fisher | 800-766-7000 | 12-565-216 Nunc part #249128 |
| Non-sterile lids for Nunc plates | PGC Scientific | 800-424-3300 | 5-6112-21 |
| Sterile basin for multichannel pipettor | Fisher | 800-766-7000 | 13-681-101 |
| Non-sterile basin for multichannel pipettor | Fisher | 800-766-7000 | 13-681-100 |
| Autotip, 96 tip rack, black tips, non-sterile, 200μl | Robins Scientific | 800-752-8585 | 1043-24-O |
| Hank's Balanced Salt Solution | Gibco | 800-828-6686 | 14065-056 |
| HEPES buffer solution 1X | Irvine Scientific | 800-437-5706 | 9319 |
| Probenecid, crystalline | Sigma | 800-325-3010 | P8761 |
| Fluo-3 AM ester | Molecular Probes | 800-438-2209<br>541-465-8300 | F-1241 |
| Ca-Green AM ester | Molecular Probes | | C-3011 |
| Pluronic acid | Molecular Probes | | P-6867 |
| DiBac(4)3, bis oxonol dye for membrane potential | Molecular Probes | | B-438 |
| BCECF AM ester for intracellular pH | Molecular Probes | | B-1150 |

**Components Needed for Dye Loading Cells:**

250mM Probenecid:

**prepare fresh each day**
710 mg Probenecid
5ml 1.0N NaOH - solubilize in
5ml Hanks' + 20mM Hepes

1 mM Dye:

Molecular Probes Fluo3,AM F-1241
Calcium Green 1,AM C-3011
1 mg Fluo3,AM or Ca-Green1,AM

443 μl MDSO - solubilize dye
443 μl 20% Pluronic acid in DMSO

Store solution in aliquots at -20˚C.
Can be frozen and thawed several times.

Alternatively, prepare a 2mM dye solution that you keep frozen, and a 20% pluronic acid solution that you keep at room temperature, and mix the 2 solutions at a 1:1 ratio before use.

20% Pluronic acid:

Molecular Probes P-6867
Weigh in a tube and solubilize in DMSO at 37˚C,
for example: 400mg/2ml DMSO.
Let cool to room temperature and aliquote. Store at room temperature.

DMSO:

Use DMSO with low water content
For example; Sigma D2650 in 5 ml ampules

Other items needed and used in a cell culture facility:

- 12 channel pipettoro 50-200 μL, example Brinkman, part #5008130-3
- Sterile pipette tips 200 μL, example E+K Scientific (tel: 408-378-2013),part #3507-R965) (10 racks of 96 tips)
- Aspirator to remove medium from plate wells. Example: 12-pin manifold aspirator, part #851388, or 8-pin manifold aspirator, part #951381 (from Wheaton Science Products (tel: 800-225-1437)
- 5 mL, 10 mL, 25 mL sterile serological pipettes
- Rechargeable pipettor for 2-25 mL pipettes
- Sterile tissue culture water
- Gloves
- Culture medium to grow cells
- EDTA and Trypsin/EDTA to life cells
- Hemacytometer and counter
  Sterile test tubes 15 mL and 50 mL
- 1 N NaOH solution

## FLIPR Test

### *Membrane Potential*

FLIPR was developed basically for the measurement of membrane potential using the voltage sensitive dye DiBAC$(4)_3$ (Molecular Probes B-438). The assay protocol was developed by Dr. Vince Groppi of Upjohn-Pharmacia in Kalamazoo, Michigan.

### Cell Culture

One criterion necessary for quality data from FLIPR is that the cells measured are at the bottom of the well. This does not necessary mean the cells are adherent. This necessity is due to the optical detection in FLIPR which enhances sensitivity to fluorescence from the bottom of the microplate well. Non-adherent cells can also be used, however not in suspension. Generally non-adherent cells are spun down, forming a pseudo-monolayer at the bottom of the plate.

Some cell types require a coating (e.g. Poly-D-Lysine) in order to insure adherence. Typical protocols would involve coating the microplates in a sterile environment before use.

The following procedure is used to prepare ceils which overexpress the novel potassium channel described herein, e.g. SEQ ID NO:2, for FLIPR

Day 1: Stock cultures of transformed or transfected heterologous expression cells are detached from T75 flasks with 1 X trypsin/EDTA. Typically 1 T75 flask will contain $2x10^6$ cells. The heterologous expression cells are diluted to approximately 0.25 x $10^5$ cells/ml and 0.2 ml of cells are distributed to each well of a 96 well plate. In this way each well is plated with 0.5 x $10^4$ A10 cells. In this manner 1 T75 flask will generate about four confluent 96 well plates after growing for 3 days.

Day 3: The heterologous expression cells are carefully fed with fresh medium. Care is taken not to disrupt the monolayer.

Day 4: Cells are ready for analysis in FLIPR.

Following this procedure, one should establish a uniform monolayer of cells in each well of a 96 well plate. If the monolayer appears non-uniform in any way, then the experiment is terminated and fresh cultures are prepared.

Having followed the above procedure to Day 4 (see previous section) it is now time to prepare the plates for testing in FLIPR. Based on their reluctance to overgrow, the A 10 cells can probably be used successfully on Days 4-6.

## Calibration

FLIPR can incorporate a calibration scheme using positive and negative control wells. The negative controls are typically additions of a non-stimulating fluid buffer. The negative controls are used to make sure the instrument as well as the assay is working properly, yielding flat baselines and no response where there should not be any. The positive control wells are used to monitor changes in signal gain. The gain calibration is useful to compare all of the data within a single run to known positives, thereby calibrating the plate.

## Preparation of Solutions (membrane potential assay)

The following protocol is intended to provide enough solutions to test approximately 2 microplates:

1.) Prepare a 20mM solution of HEPES Buffer in EBSS by adding 10 mL of 1M HEPES to 490 ml of EBSS. This solution is EBSS + H. Warm to 37 deg. C by incubation.

2.) Prepare 100 ml of EBSS + H containing 5 μM DiBAC, by adding 50μl of 10 mM DiBAC to 100 ml of EBSS + H. This solution is EBSS + H + D. Keep at 37 deg. C. This solution will be used in the negative control wells, to wash the cells and also as the media in solution with the cells during testing. Approximately 60 ml will be used per testplate. The 10 mM DiBAC stock is made up from the powder in DMSO. For the DiBAC 10 mM stock: DiBAC normally comes in 25 mg quantities and it is useful to make around ~ 20 300 μl aliquots in DMSO for freezing and later use.

3.) Prepare 50 ml of EBSS + H containing 10 μM DiBAC, by adding 50 μl of 10 mM DiBAC to 50 ml of EBSS + H. This solution is EBSS + H + D. Keep at 37 deg C. This 10 μM dye (2X) solution will be used in FLIPR to presoak the pipette tips during a run. DiBAC will absorb into plastic, and it is the stimulus compounds are prepared in DiBAC thereby maintaining the dye concentration in the optically measured microplate wells containing the cells.

4.) Prepare 100 ml of EBSS + H + D containing 300 mM KCl by adding 7.5 ml of 4M KC1 to 92.5 ml of EBSS = H and 50 ml of 10 mM DiBAC. This solution is EBSS + H + D + 300KC1. Keep at 37 deg C. This 10X KCl solution will be used for the positive control wells (20 ml addition to 180 ml: making the final concentration 30 mM KCl) in the addition plate.

## Preparation of Cell Plate

1.) Remove growth media from cells with a multi-well pipettor. If using a 8 well pipettor do no more than 4 rows at a time to insure that the cells do not dry out. Add 250 μl of pre-warmed EBSS + H + D to the cells. Do this for the entire plate. This work should be carried out on a hot plate set to 37 deg C, to minimize temperature fluctuations. Do two washes with 250 μl of EBSS + H + D.
2.) Again doing 4 rows at a time, remove the 250 μl of EBSS + H + D and replace with 180 μl of fresh pre-warmed EBSS + H + D. Make sure that pipettor tips are pre-soaked and be consistent when handling dye washes to avoid variable dilution.
3.) After the cell plate is washed place it in the incubator to equilibrate to 37 deg C.
4.) The cells should be tested in FLIPR within 2 hours of the final wash.

**Preparation of Candidate Compound (drug) Addition Plate**

1.) Place 220-250 μl of EBSS + H + D in the negative control wells. The pipettor will go down to the 50 μl fluid level and will typically only aspirate 20 μl per run, therefore this plate can be used several times. Clear microplates can be used for the stimulus plates. Using the FLIPR 96 well pipetter with a flat bottom plate will result in a dead volume of about 50 μl whereas a v-bottomed plate can have dead volumes as low as 10-20 μl.
2.) Place 220-250 μl of EBSS + H + D +300KCl in the designed positive control wells.
3.) If the unknowns are in a solvent, be sure to include the same concentration of solvent in the negative control wells. In our experience try to stay below 0.1% DMSO in possible.
4.) Place the drug addition plate in the incubator and equilibrate to 37 deg C.

***Typical Measurement Sequence for Membrane Potential***

**Start-Up Procedure**

1.) Turn on the laser water flow and electrical main disconnect.
2.) Turn on the laser. Typically, the laser will require about 15 to 30 minutes to stabilize. Coherent recommends a 30-minute warm-up stabilization period for the laser. They also recommend (due to tube lifetime) turning the laser off only if you plan on being down for more than 3 hours, otherwise leave the laser on.
3.) Turn on the computer, monitor and camera controller. Make sure that the CCD camera is at temperature before taking data with the camera. Proper temperature is indicated by a lit green a "status" light on the camera controller. Normally camera cool-down takes about 5 minutes.
4.) Power up the FLIPR enclosure. Make sure the PC is booted up *before* powering up the FLIPR enclosure. The system may lock up otherwise.
5.) Turn on the regulator for 80 psi air source.
6.) Clear any interlocks by making sure the cell drawer is in and the pipette tips are up.
7.) Start the airflow for the humidity chamber (temperature dependent assays only).
8.) Start the FLIPR code by double clicking on the FLIPR control software icon. The pipettor will also go through the normal homing cycle on start-up. If temperature control is desired, and it not set up in the setup.flp file as default on, turn it on via the Setup "heaters" pull down option. It will take approximately 30 minutes for the temperature sensors to stabilize. Recall that the temperature control option requires the low-pressure regulated air source be turned on also.
9.) Wait for the temperature warning indicators to clear on the display window if temperature control is being used.

**Data Collection**

Because of the strong temperature dependence of DiBAC, it is a good idea to immediately place the cells in the FLIPR cell incubation chamber after removing them from the incubator.
*Once the cells are place in solution with DiBAC, they should be tested within 2 hours.*
To reduce the temperature stabilization time, the cell plate and the addition plate should be removed from the incubator and put into the cell incubation chamber in FLIPR as quickly as possible. The more careful the operator is in handling the plates, the quicker the DiBAC temperature stabilizes and the quicker the fluorescent baselines stabilizes.
The operator then needs to set up the parameters to be used in the experiment. Choosing the Experiment option under the Setup pull-down menu does this.

**Typical System Setup for Measurements of Membrane Potential**

System gain parameters:

Assuming the laser is set to around 300mW

Since DiBAC is bright, the F/stop should be set around F5.6, i.e. stopped down a little Camera Exposure time around 0.4 seconds (how to set this is described below).

General Setup Parameters:

a.) Exposure Length: (camera integration time): usually around 0.4 seconds
b.) Filter #1 (standard filter)
c.) Presoak tips (usually checked, usually from left tray)
d.) Multiple additions
e.) Automation: user's choice of options

Under the First Sequence Definition

a.) Sample Interval (typically 20 seconds)
b.) Sample count (depends on length of experiment desired), usually 15 minutes is enough for DiBAC, therefore a 20 second updates, and 15 minute total run time, 45 samples would be taken.
c.) Second interval (usually not necessary with DiBAC, generally only used for fast updates)
d.) Fluid addition checked active
e.) Fluid volume to add (usually 20 μl, as DiBAC is temperature sensitive and smaller addition volumes are preferred.
f.) Dispense speed (depends on cell type, 20 μl/sec is slow, 80 μl is fast, better mixing the faster the mix speed, depends on what the cells will withstand without being relocated on the bottom of the plate.

Under Pipetting

a.) Mix volume (generally 40 μl)
b.) Number of mixes (generally 3, do mixing because of adding a small volume to a large volume, as well as the kinetics of DiBAC are slow enough to allow for omitting between updates.
c.) Tip position, uncheck, i.e. tips are out of well during data collection, since DiBAC experiment is slow, the pipettor has time to go up and down into the fluid between sample points, by unchecking this box one can insure that compound does not leach out into the cell plate prior to the pre-programmed delivery.
d.) Fluid Addition, generally for DiBAC this will be checked, that is well will remove the same amount of fluid which was added, in this example 20 μl. This insures that there is no fluid height dependence associated with adding a very fluorescent background component, that being the DiBAC in solution with the addition compounds. This may or may not be necessary. With this box unchecked, the fluid will be added, changing the total well volume to 200 μl (assuming 20 μl to 180 μl already added in the cell plate).

Once the experiment has been defined, the user will perform a signal level check using the "light bulb" or Run "Signal Test" option. This takes a camera exposure suing the current exposure time defined in the Setup Experiment window and displays the signal counts for all 96 wells of the plate. It also displays some statistics of the average, minimum and maximum signal counts for the exposure. Since the total dynamic range of the camera is 65,000 counts is it a good idea to work with signal counts in the range of 25,000 to 30,000 DiBAC. At these gain levels, a good physiological DiBAC signal (e.g. a membrane depolarization) will be on the order of 5,000 counts. Thus starting with a basal fluorescent range of 25 to 30,000 counts helps to insure that the individual camera pixels will not saturate during the data collection.
If the signal counts are too high or low, shortening or lengthening respectively, the camera exposure interval is recommended until the correct signal level is achieved. If exposure times less than 0.4 seconds are required to come down to the desired video levels, then turn down the laser source power or alternatively, stop down the camera aperture. Each F/stop increase of the camera cuts the detection sensitivity by a factor of two. General ranges for the system gain parameters are as follows:
Laser power: run between 150mW and 1 Watt, it is advised to leave around 300 mW so laser warms up at a consistent place every day, increase power when more gain is needed.
Camera F/stop (run between F/2 and F/22), note that each stop is a factor of 2 in sensitivity with F/2 being the largest aperture, with the most sensitivity. This is the easiest thing to change to et rid of light, for example when running the DiBAC assay.
Camera Exposure time: Run above 0.2 seconds, for kinetic (multiple frame) data. This is because at very short exposure times the mechanical jitter in the opening and closing of the camera shutter can add noise to the temporal fluorescent traces. This is not an issue for a single (non-kinetic) exposure.

After the signal level is set, the operator is ready to start a baseline stability check. This is usually a good idea to insure that the fluorescent baselines are under thermal equilibrium resulting in flat baseline fluorescence. This can be done by starting an experiment, usually with 20 second time updates, with the pipetting deactivated. This is more of an issue with DiBAC assays than with intracellular calcium assays. Depending on the temperature of the fluid when the cell plate with placed in FLIPR, stabilization can take a few minutes or up to 20 minutes.

Assuming the baselines are flat, then it is time to start the assay. Hitting the stop icon at any time can stop the baseline. Once the user is satisfied that the baselines are flat enough, the pipettor should be re-activated in the Experimental Setup window; "First sequence" dialog page.

The experiment will continue, with the real-time display updating the measured fluorescent data values, until the total number of measurements has been completed. At this point the pipettor will return to the pipette load station on the right side of FLIPR.

Once the run is completed the user is then ready to export (or process) the data. This basically converts the raw data files, which have already been compressed and stored on the hard disk, into fluorescent signal counts per well. Exporting of data is covered in detail in Section 3.5.

## Notes

After dye loading, several washes in a non-fluorescent salt buffer will be required to reduce signal artifacts associated with the presence of extra-cellular dye as well as background associated with fluorescent entities in media. The main artifact present with improper washing is a sharp signal decline upon addition of the stimulus due to the dilution of the strong background component. Recall that generally the compounds are mixed up in a non-fluorescent salt solution, e.g. Hanks, Earles etc. and that large volume additions are made (e.g. 50 μl to 100 μl) to insure rapid mixing.

The cell wash buffer should be the same solution used with the cells during testing. Likewise, this same buffer should be used to prepare the stimulus compounds to avoid unwanted pH, osmolarity, or morphology changes in the cells during a kinetic experiment. Typically balanced salt solutions like HBS, PBS or EBSS will be used. *It is very important that the compounds are prepared in "exactly" the same buffer as the cells are washed and sitting in prior to running the experiment.*

A typical protocol would be to load in dye for 1.5 hours, wash 3.4 times with buffer (200 μl per well), for example using a gentle washer such as the Denley CellWash. Exactly how many washes are necessary will be cell type dependent. Sometimes a wait period between washes (two before, two after) is good to allow the cells to re-equilibrate to a lower extra-cellular dye concentration, before doing the final wash. If the cells are only slightly adherent, there will be a trade-off between washing the cells thoroughly to remove extra-cellular dye, and washing cells off the plates.

The pipettor dispense speed should generally be about 50 μl/second. However, if the cells are only slightly adherent, the user may need to reduce the dispense speed. A slow dispense speed is on the order of 20 μl/second, and a fast dispense speed is around 80 μl/sec. These values must be experimentally determined for each cell type, but generally it is preferable to dispense as fast as possible to enhance mixing. The trade-off is that the pipetting speed cannot be so forceful so as to dislodge the cells at the bottom of the well. This will in effect removed the fluorescent cells from the field of view of the camera pixels, thereby causing large decreases (artifacts) in the fluorescent traces upon the fluid addition.

At the beginning of a data run, it is useful to perform a "signal test" to check the dye loading level in the cells. When establishing loading level protocols, it is useful not to load dye into the entire microplate to get a reading on the background fluorescent counts. These unloaded wells should be treated just like the loaded wells in terms of wash steps etc. The dominant background component (assuming a salt solution is used to the buffer during the experiment) will be fluorescence from the plastic microplates. Most salt buffer solutions have negligible fluorescence at excitation wavelengths of 488 nm.

Work with starting fluorescent levels (above background) of about 10,000 to 20,000 counts (recall saturation is 65,000 counts) is recommended for basal ion levels. Again, depending on loading levels, this should be achievable with approximately 0.300 W of laser power, 0.4 sec of integration time and an F/stop setting of F/2.

## Specific Dye Loading Protocol for Heterologous Overexpressor Cells

1.) Plate cells to confluence: need to be adherent
2.) Make ~2mM dye stock in anhydrous DMSO
3.) Make 1mM dye stock by diluting with 20% w/v pluronic acid (in our experience very necessary)
4.) Make 4 μM dye loading stock by adding 40 μl of 1 mM stock to 10 mls of loading buffer or media.This will do about 1 plate.

5.) Remove growth media and serum from plated cells. Alternatively, if loading in media and serum, just add dye/media/serum to existing media/serum. This removes an aspirate step, however dye concentration if reduced.

6.) Load cells with 4 μM dye (~100 ul per well) for 1.0 to 1.5 hours)

7.) Wash cells with buffer solution twice. You want a good wash so use volumes of at least 200 μl per well.

8.) Re-incubate cells for 15 minutes.

9.) Wash twice more with buffer, leaving 100-150 μl in wells. The actual volume left depends on the amount of dilution desired in the preparation of the stimulus plate. It is important that the final volumes be consistent so as to produce good well to well consistency. It may be appropriate to do 4 buffer washes consecutively, without reincubation. This is usually empirically determined based on whether or not a dye leakage artifact is present in the negative control wells.

**Example 14**

**Functional expression of the brain-derived potassium channel subunit (SEQ ID NO:3) yields currents which are suppressed by HNSPC (SEO ID NO:5)**

**Cloning SEQ ID NO:2 and SEQ ID NO:4 into mammalian expression vectors:** EcoRI fragments containing the coding region sequences SEQ ID NO: 2 and SEQ ID NO:4 were each cut from a pCR2.1 vector (Invitrogen, Carlsbad, CA) and ligated into the EcoRI site of a pcDNA3 vector (Invitrogen). Transformed bacterial colonies were screened for orientation by PCR, using vector and gene-specific primers. Clones were confirmed by DNA seuqencing.

**Generation of an N-terminal Enhanced Green Flurosencent Protein (EGFP) fusion construct of SEQ ID NO: 2:** The EcoRI fragment of SEQ ID NO:2 in a pCR2.1 vector (*supra*) was ligated into the EcoRI site of pEGFPC1 (Clontech, Palo Atlo, CA (Cormack, B.P. et al., Gene, 173:33 (1996))) to generate an N-terminal EGFP-fusion construct of SEQ ID NO:2. Transformed bacterial colonies were screened for sense orientation by PCR and confirmed by DNA sequencing.

**transfection:** HEK293 cells (ATCC, Rockville, MD) were transfected as follows: 70 ul LipoTAXI (Stratagene, La Jolla, CA) was added to a polystyrene tube and gently mixed. Two (2) ug pEFGPC1 (control vector used to trace the transfected cells) and 5 ug of one of the constructs (SEQ ID NO:2 in pcDNA3 or SEQ ID NO:4 in pcDNA3) was added to the lipid/media mixture and incubated for 20 minutes. Two (2)mls of OPTI-MEM I was then added and mixed gently. Cells to be transfected were washed once with OPTI-MEM I, and the 3 ml of lipid-DNA mixture were added to the washed cells and incubated at 37°C 5%$CO_2$ for 4-6 hours. Cells were then refed with 10% Heat Inactivated FBS (Biowhittaker, Walkersville, MD) DMEM (Mediatech 10-017-CV). For co-expression experiments, 2 ug pEGFPC1(control vector), 5 ug of pcDNA3QT2L ((SEQ ID NO:2 in pcDNA3) and 20 ug pcDNA3QT2S (SEQ ID NO:4 in pcDNA3) were used in this protocol. Cells were diluted and replated onto 35mm dishes 24-48 hours prior to patch clamping.

<u>COS-7 cells (ATCC, Rockville, MD) were transfected as follows:</u> 25 ul DMRIE-C Reagent (Gibco BRL/Life Technologies, Gaithersburg, MD) were added to 2 ml of OPTI-MEM I (Gibco BRL/Life Technologies, Gaithersburg, MD), mixed gently and incubated for 20 minuets. Two (2) ug pEGFPC1QT2L (EGFP fused to the N-terminus of SEQ ID NO: 2) and 7.3 ug pcDNA3 or 9.3 ug pcDNA3QT2S (SEQ ID NO:4 in pcDNA3) (for a 5 molar excess over hKvQT2L (SEQ ID NO:2) construct) were added to lipid/media mixture, mixed gently and incubated for 20 minutes. Two (2) mls of OPTI-MEM I was then added to this and mixed gently. Cells to be transfected were washed once with OPTI-MEM I, and the 4 ml of lipid-DNA mixture were added to the washed cells and incubated at 37°C 5%$CO_2$ for 4-6 hours. Cells were then refed with 10% Heat Inactivated FBS (Biowhittaker, Walkersville, MD) DMEM (Mediatech 10-017-CV). Cells were trypsinized, diluted and replated onto 35mm dishes 24-48 hours prior to patch clamping.

**Electrophysiology:** All recordings were obtained using the conventional whole cell configuration (Hammill, O. P., et al., Pfluger Arch., 391, 85-100, 1981). Cells were transfected with 5 ug pcDNA3 or 2 ug pEGFPQT2L (EGFP fused to the N-terminus of SEQ ID NO:2) 24-48 hours prior to experiments. Extracellular solution was (mM): 160 NaCl, 4.5 KCl, 1 $MgCl_2$, 2 $CaCl_2$ 5 HEPES, 5 glucose (pH: 7.4 by NaOH, ~325 mOsm). In K+ selectivity experiments, equimolar $Na^+$ was substituted by $K^+$. Pipette solution was (mM): 150 K-aspartate, 2 $MgCl_2$, 1 $CaCl_2$, 5 HEPES, 1.6 EGTA (pH: 7.2 by KOH, ~305 mOsm). Voltage protocols were implemented and data acquired using pClamp 6.0 software (Axon Instr., CA). Solution changes and drug application with a local perfusion system.

To generate FIG.13 cells were transfected with 2μg pEGFPQT2L(SEQ ID N0:2) and a 5 or 10 fold molar excess of empty vector (pcDNA3) or pcDNA3QT2S(SEQ ID NO:4) 24-48 hours prior to recordings. Currents were elicited by 850 ms voltage steps incremented by 10 mV from -40 mV to 70 mV from a holding potential of -60 mV. Currents are normalized to cell capacitance to yield current densities. Solid red lines are monoexponential fits to data.

**Example 15**

**Detection of expression of SEQ ID NO:4 in human brain tumors**

A human tumor blot containing diseased and normal tissues was purchased from Invitrogen, Carlsbad, CA (Cat # D5030-01; lot # 607337). The commercially available blot contained total RNA isolated from tumor tissue from four different donors; tumor and normal tissue were excised at the same operational site. Twenty micrograms of total RNA was loaded per lane and run on a 1% denaturing formaldehyde gel. The gel was vacuum blotted to a nylon membrane and fixed by UV irradiation and baking. A $^{32}$P-labeled DNA probe corresponding to nucleotide positions 989-1109 of Seq ID NO: 2 (probe 2) was generated (*supra*) and hybridized to the blot at 42˚C in NorthernMax Prehyb/Hyb Soln. (Ambion Inc., Austin, TX). High stringency washes were performed twice in 2X SSC/0.5% SDS for 5 min; 2X SSC/0.1% SDS for 5 min., 0.1%SSC/0.5% SDS for 30 min at 37 degrees as per Sambrook et al, Cold Spring Harbor, Molecular Cloning (1989). The blot was exposed to BioMax MS-1 film (Kodak, Rochester, NY) for 5-7 days at -80˚C with two intensifying screens.

Various modifications and variations of the described methods and system of the invention will be apparent to those skilled in the art without departing from the scope of the invention. Although the invention has been described in connection with specific preferred embodiments, it should be understood that the invention as claimed should not be unduly limited to such specific embodiments. Indeed, various modifications of the described modes for carrying out the invention which are obvious to those skilled in molecular biology or related fields are intended to be within the scope of the following claims.

## Claims

1. A purified polynucleotide comprising a nucleic acid sequence encoding the polypeptide of SEQ ID N0:3, said polypeptide possessing the ability to allow transmembrane potassium ion flow and/or transport.

2. The polynucleotide of claim 1 wherein the polynucleotide sequence comprises the sequence of SEQ ID NO:2.

3. An expression vector comprising the polynucleotide of claim 1 or 2.

4. A host cell transformed with the expression vector of claim 3.

5. A purified polypeptide of SEQ ID NO:3, said polypeptide possessing the ability to allow transmembrane potassium ion flow and/or transport.

6. A method for producing cells which express a polypeptide of SEQ ID NO:3, said polypeptide possessing the ability to allow transmembrane potassium ion flow and/or transport, said method comprising:

   a) transforming suitable host cells with a polynucleotide comprising a nucleic acid that encodes a polypeptide of SEQ ID NO:3, said polypeptide possessing the ability to allow transmembrane potassium ion flow and/or transport; and
   b) selecting cells expressing the polypeptide encoded by the introduced nucleic acid.

7. A method for producing a polypeptide of SEQ ID NO:3, said polypeptide possessing the ability to allow transmembrane potassium ion flow and/or transport, said method comprising:

   a) culturing a host cell according to claim 4 under conditions suitable for the expression of said polypeptide; and
   b) recovering said polypeptide from the host cell culture.

8. A method of identifying compounds that modulate the biological activity of a potassium channel, comprising:

   a) combining a candidate compound modulator of biological activity with a polypeptide of SEQ ID NO:3, said polypeptide possessing the ability to allow transmembrane potassium ion flow and/or transport; and
   b) measuring an effect of the candidate compound modulator on the transmembrane potassium ion flow and/or transport ability of the polypeptide.

9. A method of identifying compounds that modulate the biological activity of a potassium channel comprising:

   a) combining a candidate compound modulator of a potassium channel biological activity with a host-cell expressing a polypeptide of SEQ ID NO:3, said polypeptide possessing the ability to allow transmembrane potassium ion flow and/or transport; and
   b) measuring an effect of the candidate compound modulator on the transmembrane potassium ion flow and/or transport ability of the polypeptide referred to in step (a).

10. A method of claim 8 or 9, wherein a compound identified as being one that modulates the biological activity of a potassium channel is a modulator of neurophysiology.

11. Use of a purified polypeptide of SEQ ID NO:3, or host cells expressing said polypeptide, or a preparation made from said cells in a screen for compounds that modulate the transmembrane potassium ion flow and/or transport ability of said polypeptide.

## Patentansprüche

1. Gereinigtes Polynukleotid, umfassend eine für das Polypeptid der SEQ ID NO:3 codierende Nukleinsäuresequenz, wobei das Polypeptid die Fähigkeit besitzt, den Transmembranfluß und/oder -transport von Kaliumionen zu gestatten.

2. Polynukleotid nach Anspruch 1, wobei die Polynukleotidsequenz die Sequenz der SEQ ID NO:2 umfaßt.

3. Expressionsvektor, umfassend das Polynukleotid nach Anspruch 1 oder 2.

4. Wirtszelle, transformiert mit dem Expressionsvektor nach Anspruch 3.

5. Gereinigtes Polypeptid der SEQ ID NO:3, wobei das Polypeptid die Fähigkeit besitzt, den Transmembranfluß und/oder -transport von Kaliumionen zu gestatten.

6. Verfahren zur Herstellung von Zellen, die ein Polypeptid der SEQ ID NO:3 exprimieren, wobei das Polypeptid die Fähigkeit besitzt, den Transmembranfluß und/oder -transport von Kaliumionen zu gestatten, wobei man in dem Verfahren

   a) geeignete Wirtszellen mit einem eine für ein Polypeptid der SEQ ID NO:3 codierende Nukleinsäure umfassenden Polynukleotid transformiert, wobei das Polypeptid die Fähigkeit besitzt, den Transmembranfluß und/oder -transport von Kaliumionen zu gestatten, und
   b) das von der eingeführten Nukleinsäure codierte Polypeptid exprimierende Zellen selektioniert.

7. Verfahren zur Produktion eines Polypeptids der SEQ ID NO:3, wobei das Polypeptid die Fähigkeit besitzt, den Transmembranfluß und/oder -transport von Kaliumionen zu gestatten, wobei man in dem Verfahren

   a) eine Wirtszelle gemäß Anspruch 4 unter für die Expression des Polypeptids geeigneten Bedingungen kultiviert und
   b) das Polypeptid aus der Wirtszellkultur gewinnt.

8. Verfahren zur Identifizierung von Verbindungen, die die biologische Aktivität eines Kaliumkanals modulieren, wobei man

   a) einen Kandidatenverbindungsmodulator von biologischer Aktivität mit einem Polypeptid der SEQ ID NO:3 kombiniert, wobei das Polypeptid die Fähigkeit besitzt, den Transmembranfluß und/oder -transport von Kaliumionen zu gestatten, und
   b) eine Wirkung des Kandidatenverbindungsmodulators auf die Fähigkeit des Polypeptids zum Transmembranfluß und/oder -transport von Kaliumionen mißt.

9. Verfahren zur Identifizierung von Verbindungen, die die biologische Aktivität eines Kaliumkanals modulieren, wobei man

   a) einen Kandidatenverbindungsmodulator einer biologischen Kaliumkanalaktivität mit einer ein Polypeptid der SEQ ID NO:3 exprimierenden Wirtszelle kombiniert, wobei das Polypeptid die Fähigkeit besitzt, den Transmembranfluß und/oder -transport von Kaliumionen zu gestatten, und
   b) eine Wirkung des Kandidatenverbindungsmodulators auf die Fähigkeit des unter Schritt (a) genannten Polypeptids zum Transmembranfluß und/oder -transport von Kaliumionen mißt.

10. Verfahren nach Anspruch 8 oder 9, wobei es sich bei einer als Verbindung, die die biologische Aktivität eines Kaliumkanals moduliert, identifizierten Verbindung um einen Modulator der Neurophysiologie handelt.

11. Verwendung eines gereinigten Polypeptids nach SEQ ID NO:3 oder das Polypeptid exprimierender Wirtszellen oder einer aus den Zellen hergestellten Präparation bei einer Suche nach Verbindungen, die die Fähigkeit des Polypeptids zum Transmembranfluß und/oder -transport von Kaliumionen modulieren.

## Revendications

1. Polynucléotide purifié comprenant une séquence d'acide nucléique codant pour le polypeptide représenté par la SEQ ID n° 3, ledit polypeptide ayant la capacité de permettre le flux et/ou le transport transmembranaire d'ions potassium.

2. Polynucléotide selon la revendication 1, dans lequel la séquence polynucléotidique comprend la séquence représentée par la SEQ ID n° 2.

3. Vecteur d'expression comprenant le polynucléotide selon la revendication 1 ou 2.

4. Cellule hôte transformée avec le vecteur d'expression selon la revendication 3.

5. Polypeptide purifié représentée par la SEQ ID n° 3, ledit polypeptide ayant la capacité de permettre le flux et/ou le transport transmembranaire d'ions potassium.

6. Procédé pour produire des cellules qui expriment un polypeptide représentée par la SEQ ID n° 3, ledit polypeptide ayant la capacité de permettre le flux et/ou le transport transmembranaire d'ions potassium, ledit procédé comprenant :

a) la transformation de cellules hôtes appropriées avec un polynucléotide comprenant un acide nucléique qui code pour un polypeptide représenté par la SEQ ID n° 3, ledit polypeptide ayant la capacité de permettre le flux et/ou le transport transmembranaire d'ions potassium ; et
b) la sélection de cellules exprimant le polypeptide codé par l'acide nucléique introduit.

7. Procédé pour produire un polypeptide représenté par la SEQ ID n° 3, ledit polypeptide ayant la capacité de permettre le flux et/ou le transport transmembranaire d'ions potassium, ledit procédé comprenant :

a) la mise en culture d'une cellule hôte selon la revendication 4, dans des conditions convenant à l'expression dudit polypeptide ; et
b) la récupération dudit polypeptide à partir de ladite culture de cellules hôtes.

8. Procédé d'identification de composés qui modulent l'activité biologique d'un canal potassique, comprenant :

a) la combinaison d'un composé candidat modulateur de l'activité biologique avec un polypeptide représenté par la SEQ ID n° 3, ledit polypeptide ayant la capacité de permettre le flux et/ou le transport transmembranaire d'ions potassium ; et
b) la mesure d'un effet du composé candidat modulateur de la capacité de flux et/ou de transport transmembranaire d'ions potassium présentée par le polypeptide.

9. Procédé d'identification de composés qui modulent l'activité biologique d'un canal potassique comprenant :

a) la combinaison d'un composé candidat modulateur d'une activité biologique de canal potassique avec une cellule hôte exprimant un polypeptide représenté par la SEQ ID n° 3, ledit polypeptide ayant la capacité de permettre le flux et/ou le transport transmembranaire d'ions potassium ; et
b) la mesure d'un effet du composé candidat modulateur sur la capacité de flux et/ou de transport transmembranaire d'ions potassium présentée par le polypeptide auquel on se réfère à l'étape (a).

10. Procédé selon la revendication 8 ou 9, dans lequel un composé, identifié comme étant l'un des composés qui module l'activité biologique d'un canal potassique, est un modulateur de neurophysiologie.

11. Utilisation d'un polypeptide purifié représenté par la SEQ ID n° 3, ou de cellules hôtes exprimant ledit polypeptide, ou d'une préparation élaborée à partir desdites cellules, dans le cadre d'un criblage de composés qui modulent la capacité de flux et/ou de transport transmembranaire d'ions potassium présentée par ledit polypeptide.

**FIG.1**

Human Brain-Derived Potassium Channel cDNA · SEQ ID NO:1

GCCAGGCACC**ATG**GTGCAGAAGTCGCGCAACGGCGGCGTATACCCCGGCCCGAG
CGGGGAGAAGAAGCTGAAGGTGGGCTTCGTGGGGCTGGACCCCGGCGCGCCCGA
CTCCACCCGGGACGGGGCGCTGCTGATCGCCGGCTCCGAGGCCCCCAAGCGCGG
CAGCATCCTCAGCAAACCTCGCGCGGGCGGCGCGGGCGCCGGGAAGCCCCCAA
GCGCAACGCCTTCTACCGCAAGCTGCAGAATTTCCTCTACAACGTGCTGGAGCGG
CCGCGCGGCTGGGCGTTCATCTACCACGCCTACGTGTTCCTCCTGGTTTTCTCCTG
CCTCGTGCTGTCTGTGTTTTCCACCATCAAGGAGTATGAGAAGAGCTCGGAGGGG
GCCCTCTACATCCTGGAAATCGTGACTATCGTGGTGTTTGGCGTGGAGTACTTCGT
GCGGATCTGGGCCGCAGGCTGCTGCTGCCGGTACCGTGGCTGGAGGGGGCGGCT
CAAGTTTGCCCGGAAACCGTTCTGTGTGATTGACATCATGGTGCTCATCGCCTCCA
TTGCGGTGCTGGCCGCCGGCTCCCAGGGCAACGTCTTTGCCACATCTGCGCTCCG
GAGCCTGCGCTTCCTGCAGATTCTGCGGATGATCCGCATGGACCGGCGGGGAGGC
ACCTGGAAGCTGCTGGGCTCTGTGGTCTATGCCCACAGCAAGGAGCTGGTCACTG
CCTGGTACATCGGCTTCCTTTGTCTCATCCTGGCCTCGTTCCTGGTGTACTTGGCA
GAGAAGGGGGAGAACGACCACTTTGACACCTACGCGGATGCACTCTGGTGGGGC
CTGATCACGCTGACACCATTGGCTACGGGGACAAGTACCCCCAGACCTGGAACG
GCAGGCTCCTTGCGGCAACCTTCACCCTCATCGGTGTCTCCTTCTTCGCGCTGCCT
GCAGGCATCTTGGGGTCTGGGTTTGCCCTGAAGGTTCAGGAGCAGCACAGGCAGA
AGCACTTTGAGAAGAGGCGGAACCCGGCAGCAGGCCTGATCCAGTCGGCCTGGA
GATTCTACGCCACCAACCTCTCGCGCACAGACCTGCACTCCACGTGGCAGTACTA
CGAGCGAACGGTCACCGTGCCCATGTACAGTTCGCAAACTCAAACCTACGGGGCC
TCCAGACTTATCCCCCCGCTGAACCAGCTGGAGCTGCTGAGGAACCTCAAGAGTA
AATCTGGACTCGCTTTCAGGAAGGACCCCCCGCCGGAGCCGTCTCCAAGCCAGAA
GGTCAGTTTGAAAGATCGTGTCTTCTCCAGCCCCCGAGGCGTGGCTGCCAAGGGG
AAGGGGTCCCCGCAGGCCCAGACTGTGAGGCGGTCACCCAGCGCCGACCAGAGC
CTCGAGGACAGCCCCAGCAAGGTGCCCAAGAGCTGGAGCTTCGGGGACCGCAGC
CGGGCACGCCAGGCTTTCCGCATCAAGGGTGCCGCGTCACGGCAGAACTCAGAA

**FIG.1 (continued)**

GAAGCAAGCCTCCCCGGAGAGGACATTGTGGATGACAAGAGCTGCCCCTGCGAG
TTTGTGACCGAGGACCTGACCCCGGGCCTCAAAGTCAGCATCAGAGCCGTGTGTG
TCATGCGGTTCCTGGTGTCCAAGCGGAAGTTCAAGGAGAGCCTGCGGCCCTACGA
CGTGATGGACGTCATCGAGCAGTACTCAGCCGGCCACCTGGACATGCTGTCCCGA
ATTAAGAGCCTGCAGTCCAGAGTGGACCAGATCGTGGGGCGGGGCCCAGCGATC
ACGGACAAGGACCGCACCAAGGGCCCGGCCGAGGCGGAGCTGCCCGAGGACCCC
AGCATGATGGGACGGCTCGGGAAGGTGGAGAAGCAGGTCTTGTCCATGGAGAAG
AAGCTGGACTTCCTGGTGAATATCTACATGCAGCGGATGGGCATCCCCCCGACAG
AGACCGAGGCCTACTTTGGGGCCAAAGAGCCGGAGCCGGCGCCGCCGTACCACA
GCCCGGAAGACAGCCGGGAGCATGTCGACAGGCACGGCTGCATTGTCAAGATCG
TGCGCTCCAGCAGCTCCACGGGCCAGAAGAACTTCTCGGCGCCCCCGGCCGCGCC
CCCTGTCCAGTGTCCGCCCTCCACCTCCTGGCAGCCACAGAGCCACCCGCGCCAG
GGCCACGGCACCTCCCCCGTGGGGGACCACGGCTCCCTGGTGCGCATCCCGCCGC
CGCCTGCCCACGAGCGGTCGCTGTCCGCCTACGGCGGGGGCAACCGCGCCAGCAT
GGAGTTCCTGCGGCAGGAGGACACCCCGGGCTGCAGGCCCCCCGAGGGGACCCT
GCGGGACAGCGACACGTCCATCTCCATCCCGTCCGTGGACCACGAGGAGCTGGA
GCGTTCCTTCAGCGGCTTCAGCATCTCCCAGTCCAAGGAGAACCTGGATGCTCTC
AACAGCTGCTACGCGGCCGTGGCGCCTTGTGCCAAAGTCAGGCCCTACATTGCGG
AGGGAGAGTCAGACACCGACTCCGACCTCTGTACCCCGTGCGGGCCCCCGCCACG
CTCGGCCACCGGCGAGGGTCCCTTTGGTGACGTGGGCTGGGCCGGGCCCAGGAA
G**TGA**GGCGGCGCTGGGCCAGTGGACCCGCCCGCGGCCCTCCTCAGCACGGTGCC
TCCGAGGTTTTGAGGCGGGAACCCTCTGGGGCCCTTTTCTTACAGTAACTGAGTGT
GGCGGGAAGGGTGGGCCCTGGAGGGGCCCATGTGGGCTGAAGGATGGGGGCTCC
TGGCAGTGACCTTTTACAAAAGTTATTTTCCAACAGGGGCTGGAGGGCTGGGCAG
GGCCCTGTGGCTCCAGGAGCAGCGTGCAGGAGCAAGGCTGCCCTGTCCACTCTGC
TCAGGGCCGCGGCCGACATCAGCCCGGTGTGAGGAGGGGCGGGAGTGATGACGG
GGTGTTGCCAGCGTGGCAACAGGCGGGGGGTTGTCTCAGCCGAGCCCAGGGGAG
GCACAAAGGGCAGGCCTGTTCCCTGAGGACCTGCGCAAAGGGCGGGCCTGTTTG
GTGAGGACCTGCGGCCTTGGGTC

## FIG.2

Human Brain-Derived Potassium Channel DNA Structural Region · SEQ ID NO:2

ATGGTGCAGAAGTCGCGCAACGGCGGCGTATACCCCGGCCCGAGCGGGGAGAAG
AAGCTGAAGGTGGGCTTCGTGGGGCTGGACCCCGGCGCGCCCGACTCCACCCGG
GACGGGGCGCTGCTGATCGCCGGCTCCGAGGCCCCCAAGCGCGGCAGCATCCTC
AGCAAACCTCGCGCGGGCGGCGCGGGCGCCGGGAAGCCCCCCAAGCGCAACGCC
TTCTACCGCAAGCTGCAGAATTTCCTCTACAACGTGCTGGAGCGGCCGCGCGGCT
GGGCGTTCATCTACCACGCCTACGTGTTCCTCCTGGTTTTCTCCTGCCTCGTGCTG
TCTGTGTTTTCCACCATCAAGGAGTATGAGAAGAGCTCGGAGGGGGCCCTCTACA
TCCTGGAAATCGTGACTATCGTGGTGTTTGGCGTGGAGTACTTCGTGCGGATCTG
GGCCGCAGGCTGCTGCTGCCGGTACCGTGGCTGGAGGGGGCGGCTCAAGTTTGCC
CGGAAACCGTTCTGTGTGATTGACATCATGGTGCTCATCGCCTCCATTGCGGTGCT
GGCCGCCGGCTCCCAGGGCAACGTCTTTGCCACATCTGCGCTCCGGAGCCTGCGC
TTCCTGCAGATTCTGCGGATGATCCGCATGGACCGGCGGGGAGGCACCTGGAAGC
TGCTGGGCTCTGTGGTCTATGCCCACAGCAAGGAGCTGGTCACTGCCTGGTACAT
CGGCTTCCTTTGTCTCATCCTGGCCTCGTTCCTGGTGTACTTGGCAGAGAAGGGGG
AGAACGACCACTTTGACACCTACGCGGATGCACTCTGGTGGGGCCTGATCACGCT
GACCACCATTGGCTACGGGGACAAGTACCCCCAGACCTGGAACGGCAGGCTCCTT
GCGGCAACCTTCACCCTCATCGGTGTCTCCTTCTTCGCGCTGCCTGCAGGCATCTT
GGGGTCTGGGTTTGCCCTGAAGGTTCAGGAGCAGCACAGGCAGAAGCACTTTGA
GAAGAGGCGGAACCCGGCAGCAGGCCTGATCCAGTCGGCCTGGAGATTCTACGC
CACCAACCTCTCGCGCACAGACCTGCACTCCACGTGGCAGTACTACGAGCGAACG
GTCACCGTGCCCATGTACAGTTCGCAAACTCAAACCTACGGGGCCTCCAGACTTA
TCCCCCCGCTGAACCAGCTGGAGCTGCTGAGGAACCTCAAGAGTAAATCTGGACT
CGCTTTCAGGAAGGACCCCCCGCCGGAGCCGTCTCCAAGCCAGAAGGTCAGTTTG
AAAGATCGTGTCTTCTCCAGCCCCCGAGGCGTGGCTGCCAAGGGGAAGGGGTCCC
CGCAGGCCCAGACTGTGAGGCGGTCACCCAGCGCCGACCAGAGCCTCGAGGACA

**FIG.2 (continued)**

GCCCCAGCAAGGTGCCCAAGAGCTGGAGCTTCGGGGACCGCAGCCGGGCACGCC
AGGCTTTCCGCATCAAGGGTGCCGCGTCACGGCAGAACTCAGAAGAAGCAAGCC
TCCCCGGAGAGGACATTGTGGATGACAAGAGCTGCCCCTGCGAGTTTGTGACCGA
GGACCTGACCCCGGGCCTCAAAGTCAGCATCAGAGCCGTGTGTGTCATGCGGTTC
CTGGTGTCCAAGCGGAAGTTCAAGGAGAGCCTGCGGCCCTACGACGTGATGGAC
GTCATCGAGCAGTACTCAGCCGGCCACCTGGACATGCTGTCCCGAATTAAGAGCC
TGCAGTCCAGAGTGGACCAGATCGTGGGGCGGGGCCCAGCGATCACGGACAAGG
ACCGCACCAAGGGCCCGGCCGAGGCGGAGCTGCCCGAGGACCCCAGCATGATGG
GACGGCTCGGGAAGGTGGAGAAGCAGGTCTTGTCCATGGAGAAGAAGCTGGACT
TCCTGGTGAATATCTACATGCAGCGGATGGGCATCCCCCCGACAGAGACCGAGGC
CTACTTTGGGGCCAAAGAGCCGGAGCCGGCGCCGCCGTACCACAGCCCGGAAGA
CAGCCGGGAGCATGTCGACAGGCACGGCTGCATTGTCAAGATCGTGCGCTCCAGC
AGCTCCACGGGCCAGAAGAACTTCTCGGCGCCCCCGGCCGCGCCCCCTGTCCAGT
GTCCGCCCTCCACCTCCTGGCAGCCACAGAGCCACCCGCGCCAGGGCCACGGCAC
CTCCCCCGTGGGGGACCACGGCTCCCTGGTGCGCATCCCGCCGCCGCCTGCCCAC
GAGCGGTCGCTGTCCGCCTACGGCGGGGGCAACCGCGCCAGCATGGAGTTCCTGC
GGCAGGAGGACACCCCGGGCTGCAGGCCCCCCGAGGGGACCCTGCGGGACAGCG
ACACGTCCATCTCCATCCCGTCCGTGGACCACGAGGAGCTGGAGCGTTCCTTCAG
CGGCTTCAGCATCTCCCAGTCCAAGGAGAACCTGGATGCTCTCAACAGCTGCTAC
GCGGCCGTGGCGCCTTGTGCCAAAGTCAGGCCCTACATTGCGGAGGGAGAGTCA
GACACCGACTCCGACCTCTGTACCCCGTGCGGGCCCCCGCCACGCTCGGCCACCG
GCGAGGGTCCCTTTGGTGACGTGGGCTGGGCCGGGCCCAGGAAGTGA

**FIG.3**

Human Brain-Derived Potassium Channel Peptide [Residue Sequence] · SEQ ID NO:3

MVQKSRNGGVYPGPSGEKKLKVGFVGLDPGAPDSTRDGALLIAGSEAPKRGSILSKP
RAGGAGAGKPPKRNAFYRKLQNFLYNVLERPRGWAFIYHAYVFLLVFSCLVLSVFST
IKEYEKSSEGALYILEIVTIVVFGVEYFVRIWAAGCCCRYRGWRGRLKFARKPFCVIDI
MVLIASIAVLAAGSQGNVFATSALRSLRFLQILRMIRMDRRGGTWKLLGSVVYAHSK
ELVTAWYIGFLCLILASFLVYLAEKGENDHFDTYADALWWGLITLTTIGYGDKYPQT
WNGRLLAATFTLIGVSFFALPAGILGSGFALKVQEQHRQKHFEKRRNPAAGLIQSAW
RFYATNLSRTDLHSTWQYYERTVTVPMYSSQTQTYGASRLIPPLNQLELLRNLKSKS
GLAFRKDPPPEPSPSQKVSLKDRVFSSPRGVAAKGKGSPQAQTVRRSPSADQSLEDSP
SKVPKSWSFGDRSRARQAFRIKGAASRQNSEEASLPGEDIVDDKSCPCEFVTEDLTPG
LKVSIRAVCVMRFLVSKRKFKESLRPYDVMDVIEQYSAGHLDMLSRIKSLQSRVDQI
VGRGPAITDKDRTKGPAEAELPEDPSMMGRLGKVEKQVLSMEKKLDFLVNIYMQRM
GIPPTETEAYFGAKEPEPAPPYHSPEDSREHVDRHGCIVKIVRSSSSTGQKNFSAPPAAP
PVQCPPSTSWQPQSHPRQGHGTSPVGDHGSLVRIPPPPAHERSLSAYGGGNRASMEFL
RQEDTPGCRPPEGTLRDSDTSISIPSVDHEELERSFSGFSISQSKENLDALNSCYAAVAP
CAKVRPYIAEGESDTDSDLCTPCGPPPRSATGEGPFGDVGWAGPRK*

**FIG.4**

Yokoyama *et al* cDNA Sequence (HNSPC) (Genbank accession # D82346)

SEQ ID NO:4

CGCGGAGCGAGGTGGCCGCAGCGTCTCCGCGCGCGGCCCAAGCCCGGCAGGAGT
GCGGAACCGCCGCCTCGGCCATGCGGCTCCCGGCCGGGGGGCCTGGGCTGGGGC
CCGCGCCGCCCCCCGCGCTCCGCCCCCGCTGAGCCTGAGCCCGACCCGGGGCGCC
TCCCGCCAGGCACC**ATG**GTGCAGAAGTCGCGCAACGGCGGCGTATACCCCGGCC
CGAGCGGGGAGAAGAAGCTGAAGGTGGGCTTCGTGGGGCTGGACCCCGGCGCGC
CCGACTCCACCCGGGACGGGGCGCTGCTGATCGCCGGCTCCGAGGCCCCCAAGC
GCGGCAGCATCCTCAGCAAACCTCGCGCGGGCGGCGCGGGCGCCGGGAAGCCCC
CCAAGCGCAACGCCTTCTACCGCAAGCTGCAGAATTTCCTCTACAACGTGCTGGA
GCGGCCGCGCGGCTGGGCGTTCATCTACCACGCCTACGTGTTCCTCCTGGTTTTCT
CCTGCCTCGTGCTGTCTGTGTTTTCCACCATCAAGGAGTATGAGAAGAGCTCGGA
GGGGGCCCTCTACATCCTGGAAATCGTGACTATCGTGGTGTTTGGCGTGGAGTAC
TTCGTGCGGATCTGGGCCGCAGGCTGCTGCTGCCGGTACCGTGGCTGGAGGGGGC
GGCTCAAGTTTGCCCGGAAACCGTTCTGTGTGATTGACATCATGGTGCTCATCGC
CTCCATTGCGGTGCTGGCCGCCGGCTCCCAGGGCAACGTCTTTGCCACATCTGCG
CTCCGGAGCCTGCGCTTCCTGCAGATTCTGCGGATGATCCGCATGGACCGGCGGG
GAGGCACCTGGAAGCTGCTGGGCTCTGTGGTCTATGCCCACAGCAAGGAGCTGGT
CACTGCCTGGTACATCGGCTTCCTTTGTCTCATCCTGGCCTCGTTCCTGGTGTACTT
GGCAGAGAAGGGGGAGAACGACCACTTTGACACCTACGCGGATGCACTCTGGTG
GGGCCTGATCACGCTGACCACCATTGGCTACGGGGACAAGTACCCCCAGACCTGG
AACGGCAGGCTCCTTGCGGCAACCTTCACCCTCATCGGTGTCTCCTTCTTCGCGCT
GCCTGCAGGCATCTTGGGGTCTGGGTTTGCCCTGAAGGTTCAGGAGCAGCACAGG
CAGAAGCACTTTGAGAAGAGGCGGAACCCGGCAGCAGGCCTGATCCAGTCGGCC
TGGAGATTCTACGCCACCAACCTCTCGCGCACAGACCTGCACTCCACGTGGCAGT
ACTACGAGCGAACGGTCACCGTGCCCATGTACAGGTACCGCCGCCGGGCACCTGC
CACCAAGCAACTGTTTCATTTTTATTTCCATTTGTTCT**TAA**ACCCCACTTTTTGT
TGTTCATTATTTTGATTGATTTTTTTTCTTTAAAATGTATTTTTCACAAAGG

**FIG.5**

Yokoyama *et al* amino acid sequence (HNSPC) . SEQ ID NO:5

MVQKSRNGGVYPGPSGEKKLKVGFVGLDPGAPDSTRDGALLIAGSEAPKRGSILSKP
RAGGAGAGKPPKRNAFYRKLQNFLYNVLERPRGWAFIYHAYVFLLVFSCLVLSVFST
IKEYEKSSEGALYILEIVTIVVFGVEYFVRIWAAGCCCRYRGWRGRLKFARKPFCVIDI
MVLIASIAVLAAGSQGNVFATSALRSLRFLQILRMIRMDRRGGTWKLLGSVVYAHSK
ELVTAWYIGFLCLILASFLVYLAEKGENDHFDTYADALWWGLITLTTIGYGDKYPQT
WNGRLLAATFTLIGVSFFALPAGILGSGFALKVQEQHRQKHFEKRRNPAAGLIQSAW
RFYATNLSRTDLHSTWQYYERTVTVPMYRYRRRAPATKQLFHFLFSICS*

Sanguinetti *et al* amino acid sequence (HKvLQT1) (Genbank Accession U40990, U71077)

SEQ ID NO:6

**FIG.6**

METRGSRLTGGQGRVYNFLERPTGWKCFVYHFAVFLIVLVCLIFSVLSTIEQYAALAT
GTLFWMEIVLVVFFGTEYVVRLWSAGCRSKYVGLWGRLRFARKPISIIDLIVVVASM
VVLCVGSKGQVFATSAIRGIRFLQILRMLHVDRQGGTWRLLGSVVFIHRQELITTLYIG
FLGLIFSSYFVYLAEKDAVNESGRVEFGSYADALWWGVVTVTTIGYGDKVPQTWVG
KTIASCFSVFAISFFALPAGILGSGFALKVQQKQRQKHFNRQIPAAASLIQTAWRCYAA
ENPDSSTWKIYIRKAPRSHTLLSPSPKPKKSVVVKKKKFKLDKDNGVTPGEKMLTVP
HITCDPPEERRLDHFSVDGYDSSVRKSPTLLEVSMPHFMRTNSFAEDLDLEGETLLTPI
THISQLREHHRATIKVIRRMQYFVAKKKFQQARKPYDVRDVIEQYSQGHLNLMVRIK
ELQRRLDQSIGKPSLFISVSEKSKDRGSNTIGARLNRVEDKVTQLDQRLALITDMLHQ
LLSLHGGSTPGSGGPPREGGAHITQPCGSGGSVDPELFLPSNTLPTYEQLTVPRRGPDE
GS

SEQ ID NO:6

# FIG.7

Alignment Report of FIG 7.meg, using Clustal method with PAM250 residue weight table.
Monday, November 24, 1997 10:45 AM

Page 1

```
                10        20        30        40        50
1   MVQKSRNGGVYPGPSGEKKLKVGFVGLDPGAPDSTRDGALLIAGSEAPKR SEQ ID NO 3
1   MVQKSRNGGVYPGPSGEKKLKVGFVGLDPGAPDSTRDGALLIAGSEAPKR SEQ ID NO 5
1   M----------------------------------------------ETR SEQ ID NO 6

                60        70        80        90       100
51  GSILSKPRAGGAGAGKPPKRNAFYRKLQNFLYNVLERPRGWAFIYHAYVF SEQ ID NO 3
51  GSILSKPRAGGAGAGKPPKRNAFYRKLQNFLYNVLERPRGWAFIYHAYVF SEQ ID NO 5
5   GSKLT-------GGQGR-------------VYNFLERPTGWCFVYHFAVF SEQ ID NO 6

               110       120       130       140       150
101 LLVFSCLVLSVFSTIKEYEKSSEGALYILEIVTIVVFGVEYFVRIWAAGC SEQ ID NO 3
101 LLVFSCLVLSVFSTIKEYEKSSEGALYILEIVTIVVFGVEYFVRIWAAGC SEQ ID NO 5
35  LIVLVCLIFSVLSTIEQYAALATGTLFWMEIVLVVFFGTEYVVRLWSAGC SEQ ID NO 6

               160       170       180       190       200
151 CCRYRGWRGRLKFARKPFCVIDIMVLIASIAVLAAGSQGNVFATSALRSL SEQ ID NO 3
151 CCRYRGWRGRLKFARKPFCVIDIMVLIASIAVLAAGSQGNVFATSALRSL SEQ ID NO 5
85  RSKYVGLWGRLRFARKPISIIDLIVVVASMVVLCVGSKGQVFATSAIRGI SEQ ID NO 6

               210       220       230       240       250
201 RFLQILRMIRMDRRGGTWKLLGSVVYAHSKELVTAWYIGFLCLILASFLV SEQ ID NO 3
201 RFLQILRMIRMDRRGGTWKLLGSVVYAHSKELVTAWYIGFLCLILASFLV SEQ ID NO 5
135 RFLQILRMLHVDRQGGTWRLLGSVVFIHRQELITTLYIGFLGLIFSSYPV SEQ ID NO 6

               260       270       280       290       300
251 YLAEKGENDH-----FDTYADALWWGLITLTTIGYGDKYPQTWNGRLLAA SEQ ID NO 3
251 YLAEKGENDH-----FDTYADALWWGLITLTTIGYGDKYPQTWNGRLLAA SEQ ID NO 5
185 YLAEKDAVMESGRVEFGSYADALWWGVVTVTTIGYGDKVPQTWVGKTIAS SEQ ID NO 6

               310       320       330       340       350
296 TFTLIGVSFFALPAGILGSGFALKVQEQHRQKHFEKRRNPAAGLIQSAWR SEQ ID NO 3
296 TFTLIGVSFFALPAGILGSGFALKVQEQHRQKHFEKRRNPAAGLIQSAWR SEQ ID NO 5
235 CFSVFAISFFALPAGILGSGFALKVQQKQRQKHFNRQIPAAASLIQTAWR SEQ ID NO 6

               360       370       380       390       400
346 FYATNLSRTDLHSTWQYYERTVTVPMYSSQTQTYGASRLIPPLNQLELLR SEQ ID NO 3
346 FYATNLSRTDLHSTWQYYERTVTVPMYR---------------------- SEQ ID NO 5
285 CYAAEMFDS---STWKIYIRKAP----RSHT-------LLSPSPKPKKSV SEQ ID NO 6

               410       420       430       440       450
396 NLKSKSGLAFRKDPPPEPSPSQKVSLKDRVFSSPRGVAAKGKGSPQA--Q SEQ ID NO 3
374 --------------YR---------------------------------- SEQ ID NO 5
321 VVKKEK---PKLDKDNGVTPGEKMLTVPRITCDPPEERRLDHFSVDGYDS SEQ ID NO 6

               460       470       480       490       500
444 TVRRSPSADQSLEDSPSKVPKSWSFGDRSRARQAFRIKGAASRQNSEEAS SEQ ID NO 3
376 --RRAPATKQ---------------------------------------- SEQ ID NO 5
368 SVRKSPTL---LEVSMPHFMRTNSFA--------------------EDLD SEQ ID NO 6
```

# FIG.7 (continued)

Alignment Report of FIG 7.meg, using Clustal method with PAM250 residue weight table.
Monday, November 24, 1997 10:45 AM

Page 2

```
                                 510       520       530       540       550
494 LPGEDIVDDKSCPCEFVTEDLTPGLKVSIRAVCVNRFLVSKRKFKESLRP SEQ ID NO 3
384 ---------------------------------LFHFL------------ SEQ ID NO 5
395 LEGETLLT----PITHISQ-LREHHRATIKVIRRNQYFVAKKKFQQARKP SEQ ID NO 6

                                 560       570       580       590       600
544 YDVMDVIEQYSAGHLDNLSRIKSLQSRVDQIVGRGP---AITDKDRTKGP SEQ ID NO 3
389 -------------------------------------------------- SEQ ID NO 5
440 YDVRDVIEQYSQGHLNLMVRIKELQRRLDQSIGKPSLFISVSEKSKDRGS SEQ ID NO 6

                                 610       620       630       640       650
591 AEAELPEDPSNNGRLGKVEKQVLSNEKKLDFLVNIYMQRNGIPPTETEAY SEQ ID NO 3
389 -------------------------------------------------- SEQ ID NO 5
490 N--------TIGARLNRVEDKVTQLDQRLALITDHLHQLLSLH------- SEQ ID NO 6

                                 660       670       680       690       700
641 FGAKEPEPAPPYHSPEDSREHVDRHGCIVKIVRSSSSTGQKNFSAPPAAP SEQ ID NO 3
389 -------------------------------------------------- SEQ ID NO 5
525 -GQSTPGSGGP--------------------------------------- SEQ ID NO 6

                                 710       720       730       740       750
691 PVQCPPSTSWQPQSHPRQGHGTSPVGDHGSLVRIPPPPAHERSLSAYGGG SEQ ID NO 3
389 -------------------------------------------------- SEQ ID NO 5
535 ---------------PREG------GAH-----ITQPCG--------SGQ SEQ ID NO 6

                                 760       770       780       790       800
741 NRASHEPLRQEDTPGCRPPEGTLRDSDTSISIPSVDHEELERSFSGFSIS SEQ ID NO 3
389 ----------------------------------------------FSI- SEQ ID NO 5
551 SVDPELFL-----------PSNTLP-----------------TYEQLTV- SEQ ID NO 6

                                 810       820       830       840       850
791 QSKENLDALNSCYAAVAPCAKVRPYIAEGESDTDSDLCTPCQPPPRSATG SEQ ID NO 3
392 -------------------------------------CS.           SEQ ID NO 5
572 --------------------------------------------PRRGPD SEQ ID NO 6

                                 860
841 EGPPGDVGNAGPRK.                                    SEQ ID NO 3
394                                                    SEQ ID NO 5
578 EGS.                                               SEQ ID NO 6
```

Decoration 'Decoration #1': Box residues that match the Consensus exactly.

FIG.8

SEQ ID NO 3.pad
Monday, November 24, 1997 11:22 AM

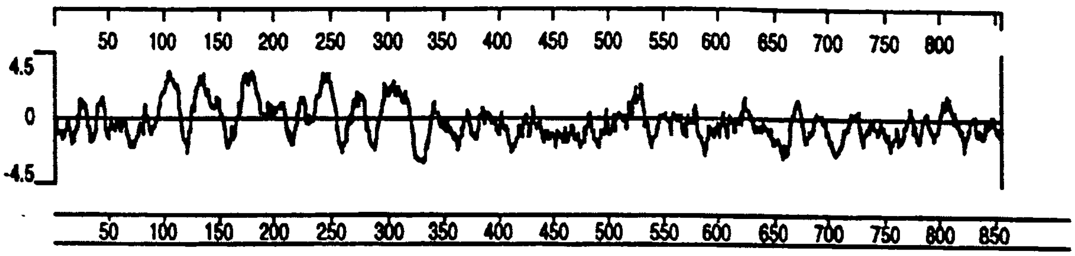


SEQ ID NO 5.pad
Monday, November 24, 1997 11:21 AM

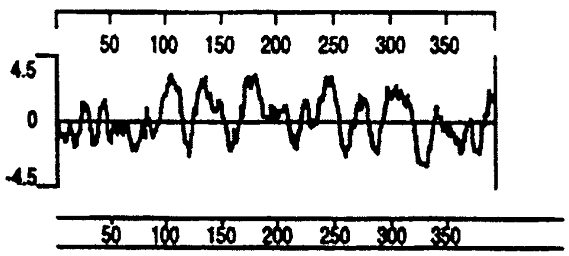


SEQ ID NO 6.pad
Monday, November 24, 1997 11:21 AM

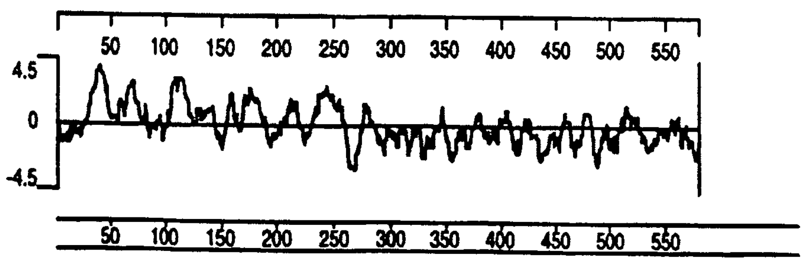

**FIG.9**

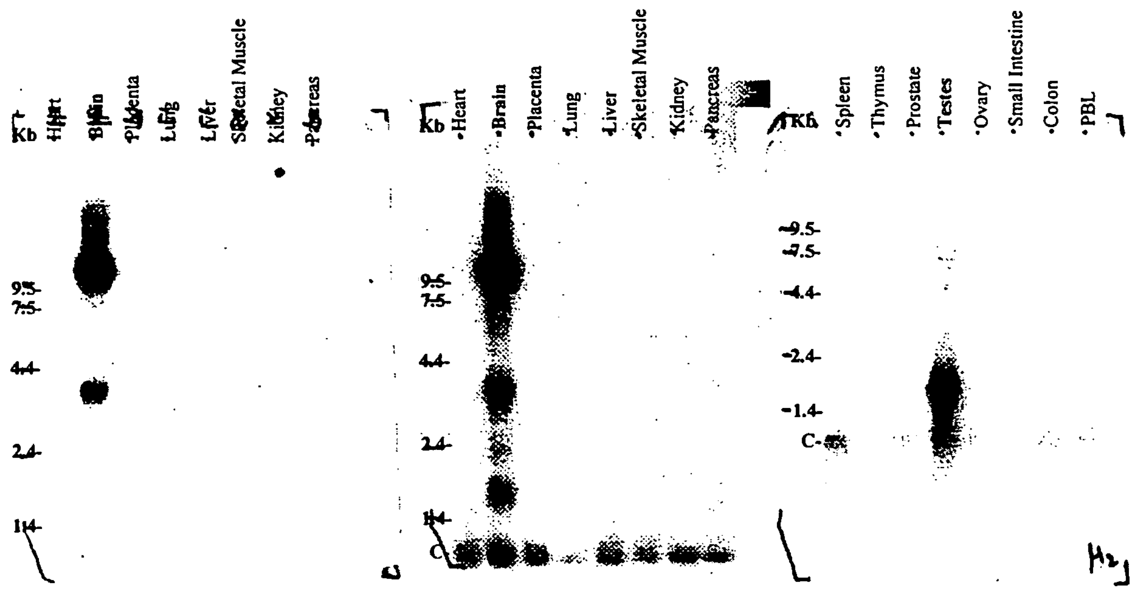


Multiple Tissue Northern I
Probe 1

Multiple Tissue Northern I
Probe 2

Multiple Tissue Northern II
Probe 2

PBL=Peripheral Blood Leukocytes
C=700 bp housekeeping cyclophilin transcript, used for normalization of RNA loading

FIG.10

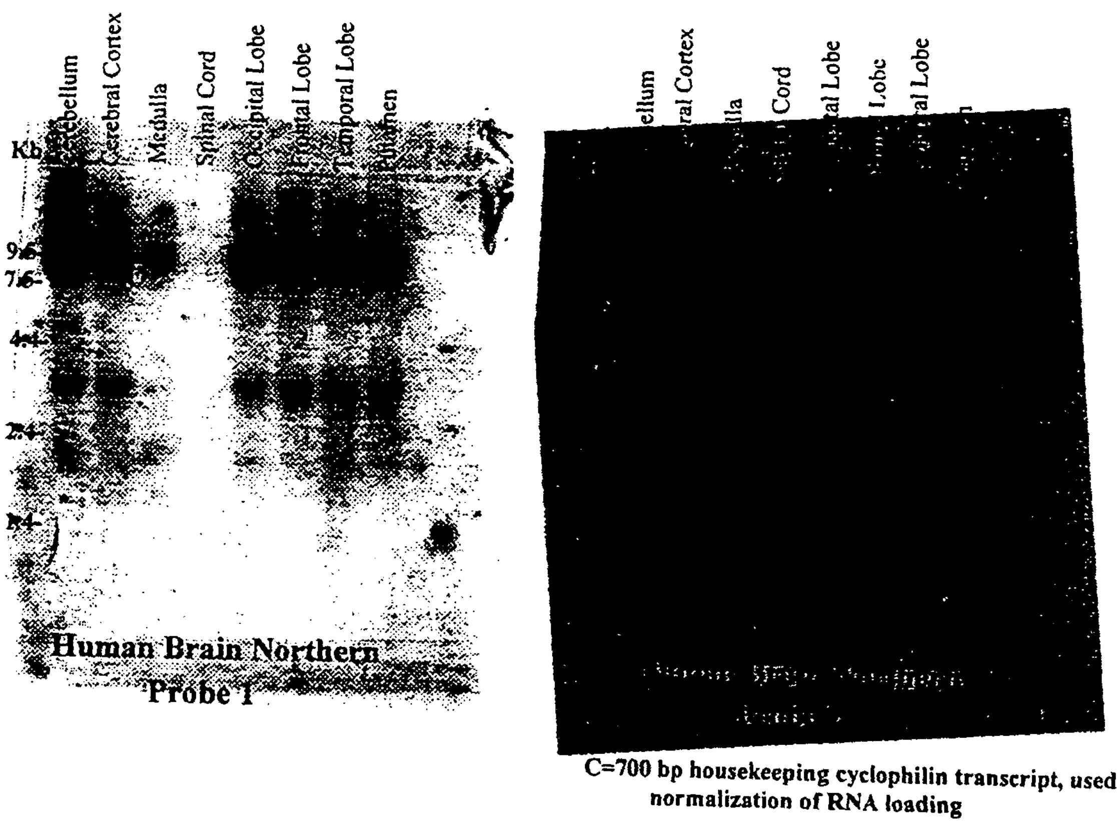


C=700 bp housekeeping cyclophilin transcript, used for normalization of RNA loading

**FIG.11**

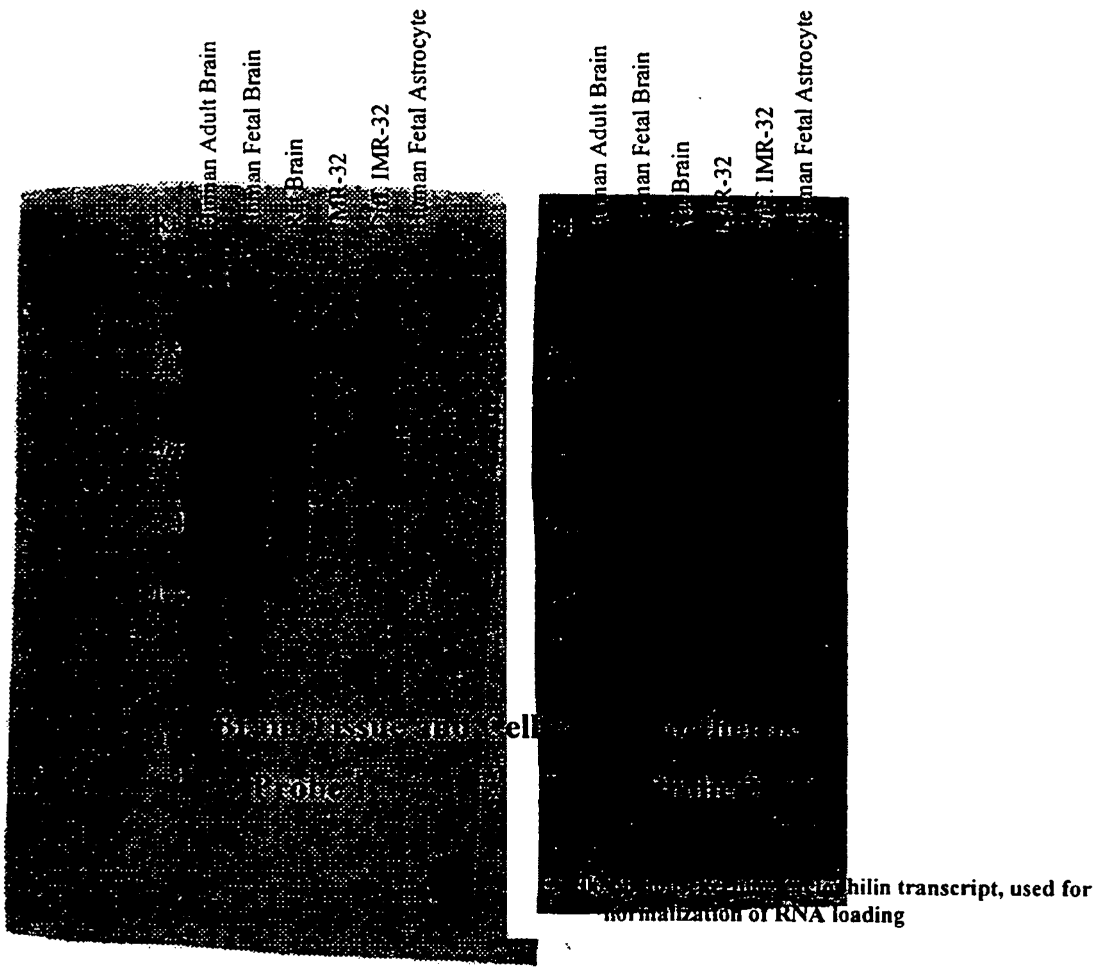
man Adult Brain
an Fetal Brain
Brain
R-32
IMR-32
man Fetal Astrocyte
an Adult Brain
n Fetal Brain
Brain
R-32
IMR-32
an Fetal Astrocyte
hilin transcript, used for
loading

**FIG.12**

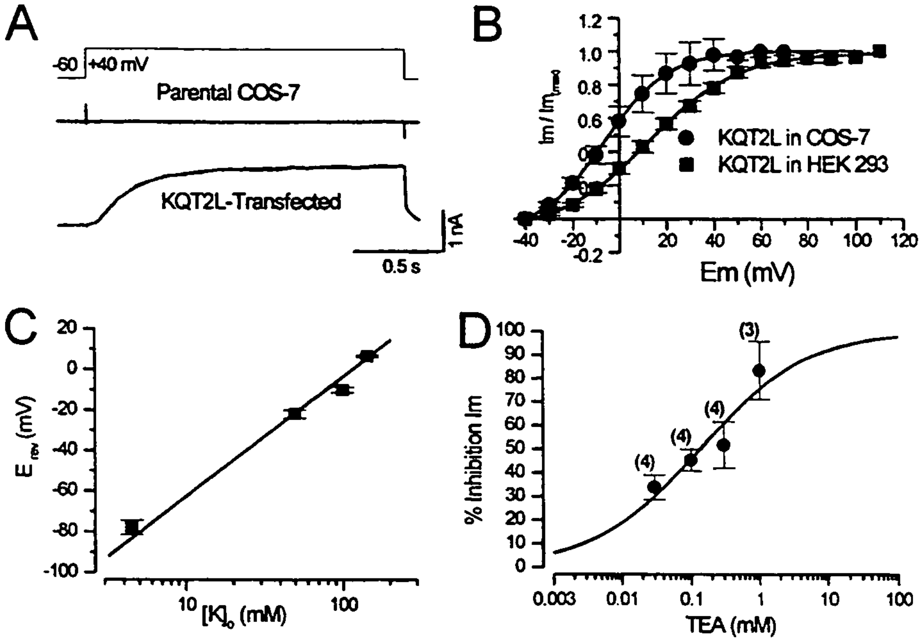
A
-60
+40 mV
Parental COS-7
KQT2L-Transfected
1 nA
0.5 s
B
Im / Im(max)
KQT2L in COS-7
KQT2L in HEK 293
Em (mV)
C
E rev (mV)
[K]o (mM)
D
% Inhibition Im
(4)
(4)
(4)
(3)
TEA (mM)

**FIG.13**

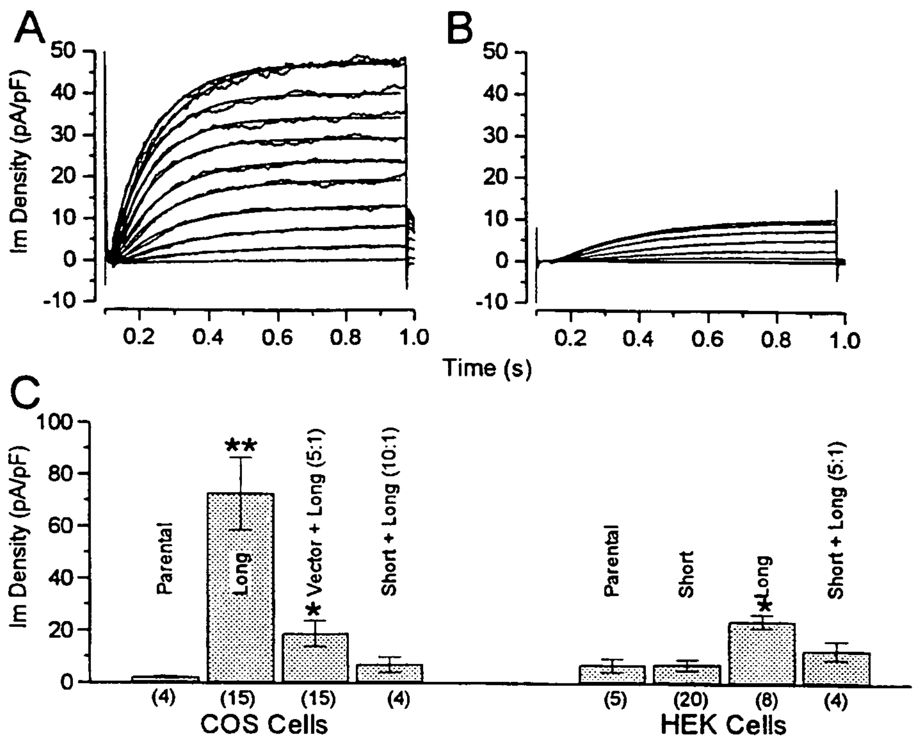
A
B
Im Density (pA/pF)
50
40
30
20
10
0
-10
0.2
0.4
0.6
0.8
1.0
Time (s)
C
100
80
60
Parental
Long
Vector + Long (5:1)
Short + Long (10:1)
Parental
Short
Long
Short + Long (5:1)
(4)
(15)
(15)
(4)
(5)
(20)
(8)
(4)
COS Cells
HEK Cells

FIG.14

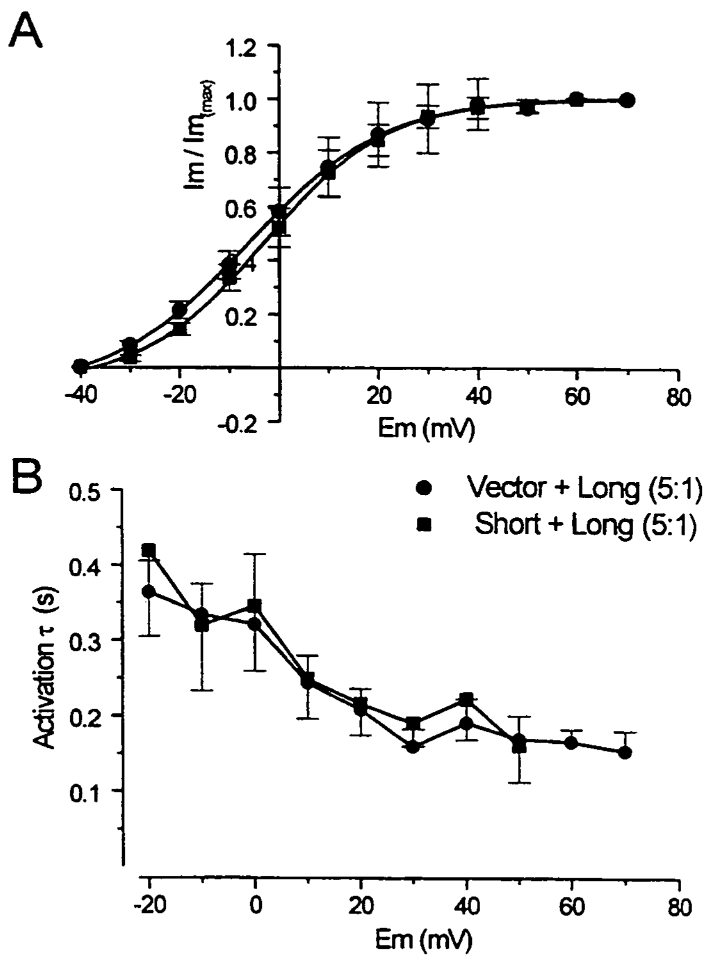
A
1.2
1.0
0.8
0.6
0.4
0.2
-0.2
Im / Im(max)
-40
-20
20
40
60
80
Em (mV)
B
0.5
0.4
0.3
0.2
0.1
Activation τ (s)
Vector + Long (5:1)
Short + Long (5:1)
-20
0
20
40
60
80
Em (mV)

FIG.15

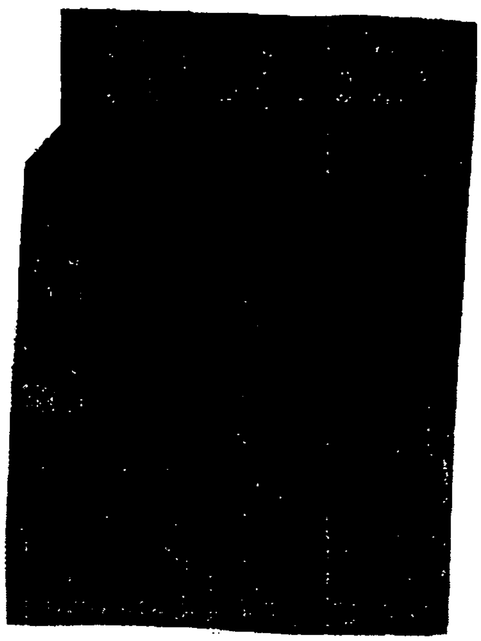

FIG.16

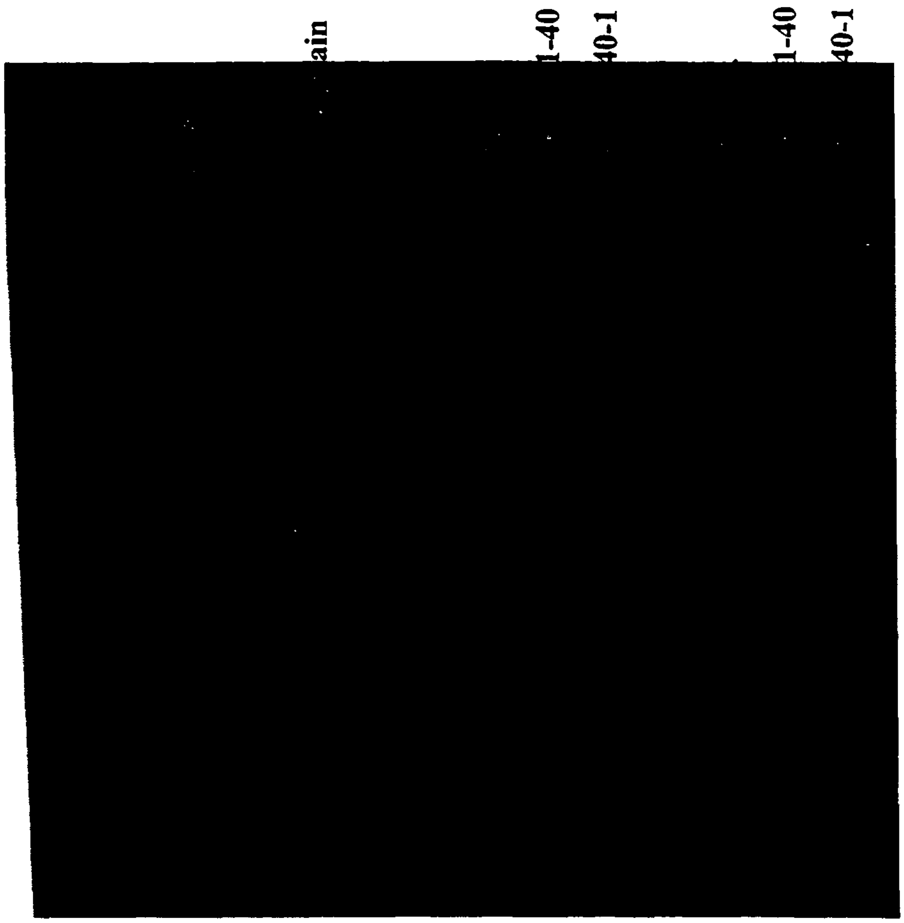

**FIG.17**

MGLKARRAAGAAGGGGGEGGGGGGGAANPAGGDSAVAGDEERKVGLAPGDVEQV
TLALGTGADKDGTLLLEGGGREEGQRRTPQGIGLLAKTPLSRPVKRNNAKYRRIQTLI
YDALERPRGWALLYHALVFLIVLGCLILAVLTTFKEYETVSGDWLLVPETFAIFIFGAE
FALRIWAAGCCCRYKGWRGRLKFARKPLCMLDIFVLIASVPVVAVGNQGNVLATSL
RSLRFLQILRMLRMDRRGGTWKLLGSAICAHSKELITAWYIGFLTLILSSFLVYLVEK
DVPEMDAQGEEMKEEFETYADALWWGLITLATIGYGDKTPKTWEGRLIAATFSLIGV
SFFALPAGILGSGLALKVQEQHRQKHFEKRRKPAAELIQAAWRYYATNNRLDLVAT
WRFYESVVSFPFFRKEQLEAAASQKLGLLDRVRLSNPRGSNTKGKLFTPLNVDAIEES
PSKEPKPVGLNNKERFRTAFRMKAYAFWQSSEDAGTGDPMTEDRGYGNDFLIEDMIP
TLKAAIRAVRILQFRLYKKKFKETLRPYDVKDVIEQYSAGHLDMLSRIKYLQTRIDMI
FTPGPPSTPKHKKSQKGSAFTYPSQQSPRNEPYVARAATSETEDQSMMGKFVKVERQ
VHDMGKKLDFLVDMHMQHMERLQVHVTEYYPTKGASSPAEGEKKEDNRYSDLKTI
ICNYSESGPPDPPYSFHQVPIDRVGPYGFFAHDPVKLTRGGPSSTKAQANLPSSGSTYA
ERPTVLPILTLLDSCVSYHSQTELQGPYSDHISPRQRRSITRDSDTPLSLMSVNHEELER
SPSGFSISQDRDDYVFGPSGGSSWMREKRYLAEGETDTDTDPFTPSGSMPMSSTGDGI
SDSIWTPSNKPT

**SEQ ID NO:7 · Rat KvQT3 (GENBANK Accession Number: AF087454)**

**FIG. 18**

GDVEQVTLALGAGADKDGTLLLEGGGRDEGQRRTPQGIGLLAKTPLSRPVKRNNAK
YRRIQTLIYDALERPRGWALLYHALVFLIVLGCLILAVLTTFKEYETVSGDWLLLLETF
AIFIFGAEFALRIWAAGCCCRYKGWRGRLKFARKPLCMLDIFVLIASVPVVAVGNQG
NVLATSLRSLRFLQILRMLRMDRRGGTWKLLGSAICAHSKELITAWYIGFLTLILSSFL
VYLVEKDVPEVDAQGEEMKEEFETYADALWWGLITLATIGYGDKTPKTWEGRLIAA
TFSLIGVSFFALPAGILGSGLALKVQEQHRQKHFEKRRKPAAELIQAAWRYYATNPNR
IDLVATWRFYESVVSFPFFRKEQLEAASSQKLGLLDRVRLSNPRGSNTKGKLFTPLNV
DAIEESPSKEPKPVGLNNKERFRTAFRMKAYAFWQSSEDAGTGDPMAEDRGYGNDF
PIEDMIPTLKAAIRAVRILQFRLYKKKFKETLRPYDVKDVIEQYSAGHLDMLSRIKYLQ
TRIDMIFTPGPPSTPKHKKSQKGSAFTFPSQQSPRNEPYVARPSTSEIEDQSMMGKFVK
VERQVQDMGKKLDFLVDMHMQHMERLQVQVTEYYPTKGTSSPAEAEKKEDNRYS
DLKTIICNYSETGPPEPPYSFHQVTIDKVSPYGFFAHDPVNLPRGGPSSGKVQATPPSS
ATTYVERPTVLPILTLLDSRVSCHSQADLQGPYSDRISPRQRRSITRDSDTPLSLMSVN
HEELERSPSGFSISQDRDDYVFGPNGGSSWMREKRYLAEGETDTDTDPFTPSGSMPLS
STGDGISDSVWTPSNKPI

**SEQ ID NO:8 · Human KvQT3 (partial)**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5397702 A
- US 5637470 A
- US 5607843 A
- WO 9603415 A1
- US 5602169 A
- US 5283173 A
- US 5468614 A
- US 3654090 A
- US 3850752 A
- US 4016043 A
- US 5639595 A
- US 5652355 A
- US 5652356 A
- US 5624820 A
- US 5589466 A
- US 5629158 A
- GB 9726339 A

### Non-patent literature cited in the description


- **TORAL, J. et al.** Use of Cultured Human Neuroblastoma Cells in Rapid Discovery of the Voltage-gated Potassium-channel Blockers. *J. Pharm. Pharmacol.,* 1994, vol. 46, 731
- **SHAN PING YU et al.** Mediation of Neuronal Apoptosis by Enhancement of Outward Potassium Current. *Science,* 1997, vol. 278, 114
- **WELLER, M. et al.** Developmental and Genetic Regulation of Programmed Neuronal Death. *J. Neural Transm. Suppl.,* 1997, vol. 50, 115
- **STAHL, S.M.** Apoptosis: Neuronal Death by Design. *J. Clin. Psychiatry,* 1997, vol. 5, 183
- **WANG, Q., et al.** *Nature Genet.,* 1996, vol. 12, 17
- **WEI, A. et al.** *Neuropharmacology,* 1996, vol. 35 (7), 805
- **CURRAN, M.E. et al.** *Cell,* 1995, vol. 80, 795
- **BARHANIN, J. et al.** *Nature,* 1996, vol. 384, 78-80
- **SANGUINETTI, M. C. et al.** *Nature,* 1996, vol. 384, 80
- **ATTALI, B.** *Nature,* 1996, vol. 384, 25
- **YOKOYAMA, M.** Identification and Cloning of Neuroblastoma-Specifcc and Nerve Tissue-Specific Genes through Compiled Express Profiles. *DNA Research,* 1996, vol. 3, 311
- **YOKOYAMA, M.** Identification and Cloning of Neuroblastoma-Specific and Nerve Tissue-Specific Genes through Compiled Express Profiles. *DNA Research,* 1996, vol. 3, 311
- **KYTE, J. ; DOOLITTLE, R.F.** *J. Mol. Biol.,* 1982, vol. 157, 105
- **WANG, Q. et al.** *Nature Genet.,* 1996, vol. 12, 17
- *Nature,* 1996, vol. 384, 78-80
- *Nature,* 1996, vol. 384, 80
- **BARHANIN, J. et al.** *Nature,* 1996, vol. 384, 25
- *DNA Research,* 1996, vol. 3, 311
- **SHER et al.** *J. Neurochemistry,* 1988, vol. 50, 1708
- **COLOM et al.** *J Neurochem.,* 1998, vol. 70, 1925
- **YU, S. et al.** *Neurobiol Dis.,* 1998, vol. 5 (2), 81
- **CHANDY ; GUTMAN.** *Handbook of Receptors and Channels,* 1995
- **SINGH et al.** *Nature Genetics,* 1998
- **CHARLIER et al.** *Nature Genetics,* 1998
- **SINGH N. A. et al.** *Nature Genetics,* 1998, vol. 18, 25
- **BIERVERT C. et al.** *Science,* 1998, vol. 279, 403
- **CHARLIER C. et al.** *Nature Genetics,* 1998, vol. 18, 53
- **NAKAMURA, M. et al.** *Receptors and Channels,* 1998, vol. 5, 255
- **MARRION N. V.** Control of M-Current. *Ann. Rev. Physiol.,* 1997, vol. 59, 483
- **BROWN D. A. ; ADAMS, P. R.** *Nature,* 1980, vol. 283, 673
- **WANG H.S. et al.** *Society for Neuroscience,* 1998, vol. 24, 792.1
- **YANG, W.P. et al.** *J. Biol.Chem.,* 1998, vol. 31, 19419
- **WEI et al.** *Neuropharmacology,* 1996, vol. 35, 805-829
- **BIERVERT et al.** *Science,* 1998, vol. 279, 403-406
- **SAMBROOK, J. et al.** Molecular Cloning Second Edition. Cold Spring Harbor Press, 1990

- **MILLER, L.K. et al.** *Curr. Op. Genet. Dev.,* 1993, vol. 3, 97
- **O'REILLY, D.R. et al.** *Baculovirus Expression Vectors: A Laboratory Manual,* 127
- **LA JOLLA, CA ; PLEASURE, S.J. et al.** *J. Neuroscience,* 1992, vol. 12, 1802
- *J. Neuroscience,* 1993, vol. 35, 585
- **HIRAKA, G. et al.** *J. Virol.,* 1991, vol. 65, 2732
- **WERTKIN, R. et al.** *PNAS,* 1993, vol. 90, 9513
- **YOUNKIN, D.P. et al.** *PNAS,* 1993, vol. 90, 2174
- **PORATH, J.** *Protein Exp. Purif.,* 1992, vol. 3, 263
- **RINAS, U. et al.** *Biotechnology,* 1990, vol. 8, 543
- **HOROWITZ, B. et al.** *J. Biol. Chem.,* 1989, vol. 265, 4189
- **HAMPTON, R. et al.** Serological Methods - a Laboratory Manual. APS Press, 1990
- **MADDOX, D.E. et al.** *J. Exp. Med.,* vol. 158, 1211
- **KREIG ; MELTON.** *Nucleic Acids Res.,* 1984, vol. 12, 7057
- **GOLDIN, A.** *Methods Enzyml.,* 1992, vol. 207, 266
- **STUHMER, W.** *Methods in Enzymol.,* 1992, vol. 207, 319
- **KOHLER et al.** *Science,* 1996, vol. 273, 1709
- **GRISSMER, S. et al.** Calcium-activated Potassium Channels in Resting and Activated Human T Lymphocytes. *J. Gen. Physiol,* 1993, vol. 102, 601
- **IKEDA et al.** *Pfueg. Arch. Eur. J. Physiol.,* 1992, vol. 422, 201
- **GRISSMER et al.** *J. Gen. Physiol.,* 1993, vol. 102, 601
- **OMARI, K. et al.** *J. Physiol.,* 1997, vol. 499, 369
- **PANYI, G. et al.** *J. Gen. Physiol.,* 1996, vol. 107 (3), 409
- **KAMB, A.** *Methods Enzymol.,* 1992, vol. 207, 423
- **SUN, T. et al.** *Biochemistry,* 1994, vol. 33 (33), 9992
- **SPENCER, R.H. et al.** *J. Biol. Chem.,* 1997, vol. 272, 2389
- *EMBO,* 1992, vol. 11 (6), 2033
- **GRISSMER et al.** *Mol. Pharm.,* 1994, vol. 45, 1227
- **HILL, R.J.** *Mol. Pharm.,* 1995, vol. 48, 98
- **DEUTSCH, C. et al.** *J. Biol. Chem.,* 1991, vol. 266, 3668
- **DEUTSCH et al.** *J. Biol. Chem.,* 1991, vol. 266, 3668
- **HOFFMAN, R. et al.** *Anal. Biochem.,* 1992, 20370
- **KIENUIS, C.B.M. et al.** *J. Recept. Res.,* 1992, vol. 12, 389
- **BRAUNER et al.** *Biochimica et Biophysica Acta,* 1984, vol. 771, 208
- **FIELDS, S. et al.** *Trends Genet.,* 1994, vol. 10, 286
- **ALLEN, J.B. et al.** *TIBS,* 1995, vol. 20, 511
- **FIELDS, S. ; SONG, O.** *Nature,* 1989, vol. 340, 245
- **MENDELSOHN, A.R. ; BRENT, R.** *Curr. Op. Biotech.,* 1994, vol. 5, 482
- **PHIZICKY. E.M. ; FIELDS, S.** *Microbiological Rev.,* 1995, vol. 59 (1), 94
- **YANG, M. et al.** *Nucleic Acids Res.,* 1995, vol. 23 (7), 1152
- **FIELDS, S. ; STEMGLANZ, R.** *TIG,* 1994, vol. 10 (8), 286
- **VIDAL M ; BRAUN P ; CHEN E ; BOEKE JD ; HARLOW E.** Genetic characterization of a mammalian protein-protein interaction domain by using a yeast reverse two-hybrid system. *Proc Natl Acad Sci U S A,* 1996, vol. 17;93 (19), 10321-10326
- **VIDAL M ; BRACHMANN RK ; FATTAEY A ; HARLOW E ; BOEKE JD.** Reverse two-hybrid and one-hybrid systems to detect dissociation of protein-protein and DNA-protein interactions. *Proc Natl Acad Sci U S A,* 1996, vol. 17;93 (19), 10315-10320
- **WHITE MA.** The yeast two-hybrid system: forward and reverse. *Proc Natl Acad Sci U S A,* 1996, vol. 17;93 (19), 10001-10003
- **LEANNA CA ; HANNINK M.** The reverse two-hybrid system: a genetic scheme for selection against specific protein/protein interactions. *Nucleic Acids Res,* 1996, vol. 1;24 (17), 3341-3347
- **KOHLER ; MILSTEIN.** *Nature,* 1975, vol. 256, 495
- **MACPHERSON.** Soft Agar Techniques, in Tissue Culture Methods and Applications. Academic Press, 1973
- **MADDOX, D.E. et al.** *J. Exp. Med.,* 1983, vol. 158, 1211
- **SITES, D.P. et al.** Basic and Clinical Immunology. Lange Medical Publications, 1982
- PCR Bibliography. **PERKIN ELMER.** Roche Molecular Systems. CLONTECH products
- Remington's Pharmaceutical Sciences. Maack Publishing Co
- **SOARES, B. et al.** *PNAS,* 1994, vol. 91, 9228
- **SCHAEFER.** *Anal. Biochem.,* 1995, vol. 227, 255
- **TIMPE et al.** *Nature,* 1988, vol. 331, 143
- **HAMMILL, O. et al.** *Pfugers Arch.,* 1981, vol. 391, 85
- **SOREQ, H. ; SEIDMAN., S.** *Methods Enzymol.,* 1992, vol. 207, 225
- **GOLDSTEIN, A. et al.** *PNAS,* 1986, vol. 83, 7503

- **SCHECTER, A.I. et al.** *Nature,* 1984, vol. 312, 513
- **DEUTSCH, C. et al.** *J. of Biological Chemistry,* 1991, vol. 266 (6), 3668-3674
- **DANIEL, S. et al.** *J. Pharmacol. Methods,* 1991, vol. 25, 185
- **CORMACK, B.P. et al.** *Gene,* 1996, vol. 173, 33
- **HAMMILL, O. P. et al.** *Pfluger Arch.,* 1981, vol. 391, 85-100
- **SAMBROOK et al.** Molecular Cloning. Cold Spring Harbor, 1989